(19) Europäisches Patentamt / European Patent Office / Office européen des brevets

(11) EP 4 628 104 A1

(12) **EUROPEAN PATENT APPLICATION**
published in accordance with Art. 153(4) EPC

(43) Date of publication:
**08.10.2025 Bulletin 2025/41**

(21) Application number: **23896811.9**

(22) Date of filing: **29.11.2023**

(51) International Patent Classification (IPC):
***A61K 47/68*** (2017.01) ***C07K 16/28*** (2006.01)
***A61K 47/65*** (2017.01) ***A61K 39/395*** (2006.01)
***A61K 31/4745*** (2006.01) ***A61P 35/00*** (2006.01)

(52) Cooperative Patent Classification (CPC):
**A61K 31/4745; A61K 39/395; A61K 47/65; A61K 47/68; A61P 35/00; C07K 16/28**

(86) International application number:
**PCT/CN2023/134928**

(87) International publication number:
**WO 2024/114667 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2022 CN 202211521035**

(71) Applicants:
- **Shanghai Chia Tai Tianqing Pharmaceutical Technology Development Co., Ltd.**
  **Shanghai 201107 (CN)**
- **Chia Tai Tianqing Pharmaceutical Group Co., Ltd.**
  **Lianyungang, Jiangsu 222062 (CN)**

(72) Inventors:
- **CHEN, Tianxi**
  **Lianyungang, Jiangsu 222062 (CN)**
- **YING, Shusong**
  **Lianyungang, Jiangsu 222062 (CN)**
- **WEI, Shanshan**
  **Lianyungang, Jiangsu 222062 (CN)**
- **XU, Tongjie**
  **Lianyungang, Jiangsu 222062 (CN)**
- **ZHANG, Zhengping**
  **Lianyungang, Jiangsu 222062 (CN)**

(74) Representative: **WSL Patentanwälte Partnerschaft mbB**
**Kaiser-Friedrich-Ring 98**
**65185 Wiesbaden (DE)**

Remarks:
The complete document including Reference Table(s) and the Sequence Listing(s) can be downloaded from the EPO website

# (54) ANTI-CLDN18.2 ANTIBODY-DRUG CONJUGATE, AND PHARMACEUTICAL COMPOSITION THEREOF AND USE THEREOF

(57) The present invention belongs to the field of biotechnology. Provided are an anti-CLDN18.2 antibody-drug conjugate, and a pharmaceutical composition thereof and the use thereof. The antibody-drug conjugate comprises an antibody or an antigen-binding fragment thereof, a linker and a cytotoxic drug, which are connected. The provided antibody-drug conjugate achieves excellent anti-tumor activity or/and good safety. The provided antibody-drug conjugate can be used in the treatment of tumors.



Processed by Luminess, 75001 PARIS (FR)

## Description

### CROSS-REFERENCE TO RELATED APPLICATION

The present application claims priority and benefit to the Chinese Patent Application No. CN 202211521035.0 filed on Nov. 30, 2022, which is incorporated herein by reference in its entirety.

### TECHNICAL FIELD

The present disclosure pertains to the field of biotechnology, and relates to an antibody-drug conjugate comprising an antibody or an antigen-binding fragment thereof, a linker, and a payload (e.g., a cytotoxic drug), which are connected to each other. The present disclosure further relates to use of the antibody-drug conjugate for preparing a medicament for treating a tumor.

### BACKGROUND

Tight junction protein 18 (CLDN18 or Claudin 18) belongs to the family of cell tight junction proteins. It participates in the formation of intercellular tight junction, controls the flow of intercellular molecules, and plays an important role in maintaining the polarity and permeability of cells and in intercellular signal transmission. CLDN18 is a tetraspanin, with the N-terminus and the C-terminus both located intracellularlly and formed two loop regions extracellularly. Due to alternative splicing of RNA, two different isoforms are present in tissues, namely tight junction protein 18.1 (CLDN18.1, e.g., human CLDN18.1 under the Uniprot accession No. P56856-1) and tight junction protein 18.2 (CLDN18.2, e.g., human CLDN18.2 under the Uniprot accession No. P56856-2).

CLDN18.1 (Claudin 18.1) and CLDN18.2 (Claudin 18.2) have high similarity in protein sequences but different expression distributions. CLDN18.1 is selectively expressed in normal lung tissues, while CLDN18.2 is expressed in normal tissues at a very limited level and is specifically expressed only in differentiated epithelial cells of the gastric mucosa. However, research shows that CLDN18.2 exhibits high expression in 70% of primary gastric cancers and metastases thereof, and furthermore, CLDN18.2 is found to be frequently ectopically activated in a variety of malignant tumors, including pancreatic cancer, esophageal cancer, lung cancer, breast cancer, ovarian cancer, and otorhinolaryngological tumors (Niimi et al., (2001) Mol Cell Biol 21 (21): 7380-7390; Sahin, U. et al., (2008) Clin Cancer Res. 14, 7624-34; Tanaka et al., (2011) J Histochem Cytochem 59(10): 942-952; Micke et al., (2014) Int J Cancer 135(9): 2206-2214; Shimobaba et al., (2016) Biochim Biophys Acta 1863(6Pt A): 1170-1178; Sing h et al., (2017) J Hema tol Oncol 10 (1): 105; Tokumitsu et al., (2017) Cytopathology 28(2): 116-121).

Antibody-drug conjugates (ADCs) are a class of drugs that combine the high specificity of therapeutic antibodies and the high killing activity of cytotoxic drugs. The therapeutic antibody moiety is linked to the cytotoxic drug moiety via an intermediate linker moiety. While antibody-drug conjugates targeting CLDN18.2 have been reported in the literature for anti-tumor applications, existing research remains limited, underscoring the need for additional available options.

### SUMMARY

*Antibody Drug Conjugate (ADC)*

The present disclosure provides an antibody-drug conjugate, wherein an antibody or an antigen-binding fragment thereof is conjugated to a cytotoxic drug, and the antibody or the antigen-binding fragment thereof specifically binds to CLDN18.2.

In one aspect, the present disclosure provides an antibody-drug conjugate of general formula $Ab\text{-}(L\text{-}U)_n$ or a pharmaceutically acceptable salt or a solvate thereof, wherein Ab is an antibody or an antigen-binding fragment thereof, L is a linker, and U is a cytotoxic drug. In some embodiments, n is an integer selected from1 to 10.

In some embodiments, the antibody or the antigen-binding fragment thereof Ab is linked to the linker L via a specific functional group, and the antibody or the antigen-binding fragment thereof can specifically bind to an antigen. In some embodiments, Ab is linked to the linker L via a covalent bond.

In some embodiments, the antibody or the antigen-binding fragment thereof binds to human CLDN18.2.

In some embodiments, the antibody or the antigen-binding fragment thereof is a murine antibody, a chimeric antibody, a humanized antibody, or a human antibody. In some embodiments, the antibody or the antigen-binding fragment thereof is a monoclonal antibody, a monospecific antibody, a multispecific antibody, a Fab fragment, a F(ab')2 fragment, an Fd fragment, an Fv fragment, a dAb, a single-chain Fv (scFv) molecule, or a nanobody.

In some embodiments, the anti-CLDN18.2 antibody or the antigen-binding fragment thereof comprises heavy chain CDR (HCDR) 1, HCDR2, HCDR3, light chain CDR (LCDR) 1, LCDR2, and LCDR3, wherein

(1) the HCDR1 comprises an HCDR1 amino acid sequence in SEQ ID NO: 11, the HCDR2 comprises an HCDR2 amino acid sequence in SEQ ID NO: 11, the HCDR3 comprises an HCDR3 amino acid sequence in SEQ ID NO: 11, the LCDR1 comprises an LCDR1 amino acid sequence in SEQ ID NO: 16, the LCDR2 comprises an LCDR2 amino acid sequence in SEQ ID NO: 16, and the LCDR3 comprises an LCDR3 amino acid sequence in SEQ ID NO: 16;
(2) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(3) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO: 13, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(4) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in SEQ ID NO: 14, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(5) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(6) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;
(7) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO: 13, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;
(8) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in SEQ ID NO: 14, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;
(9) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;
(10) the HCDR1 comprises an HCDR1 amino acid sequence in SEQ ID NO: 29, the HCDR2 comprises an HCDR2 amino acid sequence in SEQ ID NO: 29, the HCDR3 comprises an HCDR3 amino acid sequence in SEQ ID NO: 29, the LCDR1 comprises an LCDR1 amino acid sequence in SEQ ID NO: 34, the LCDR2 comprises an LCDR2 amino acid sequence in SEQ ID NO: 34, and the LCDR3 comprises an LCDR3 amino acid sequence in SEQ ID NO: 34;
(11) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;
(12) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;
(13) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;
(14) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;
(15) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30,

the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(16) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(17) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(18) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(19) the HCDR1 comprises an HCDR1 amino acid sequence in SEQ ID NO: 43, the HCDR2 comprises an HCDR2 amino acid sequence in SEQ ID NO: 43, the HCDR3 comprises an HCDR3 amino acid sequence in SEQ ID NO: 43, the LCDR1 comprises an LCDR1 amino acid sequence in SEQ ID NO: 44, the LCDR2 comprises an LCDR2 amino acid sequence in SEQ ID NO: 44, and the LCDR3 comprises an LCDR3 amino acid sequence in SEQ ID NO: 44;
(20) the HCDR1 comprises an HCDR1 amino acid sequence in SEQ ID NO: 51, the HCDR2 comprises an HCDR2 amino acid sequence in SEQ ID NO: 51, the HCDR3 comprises an HCDR3 amino acid sequence in SEQ ID NO: 51, the LCDR1 comprises an LCDR1 amino acid sequence in SEQ ID NO: 52, the LCDR2 comprises an LCDR2 amino acid sequence in SEQ ID NO: 52, and the LCDR3 comprises an LCDR3 amino acid sequence in SEQ ID NO: 52; or
(21) the HCDR1 comprises an HCDR1 amino acid sequence in SEQ ID NO: 59, the HCDR2 comprises an HCDR2 amino acid sequence in SEQ ID NO: 59, the HCDR3 comprises an HCDR3 amino acid sequence in SEQ ID NO: 59, the LCDR1 comprises an LCDR1 amino acid sequence in SEQ ID NO: 60, the LCDR2 comprises an LCDR2 amino acid sequence in SEQ ID NO: 60, and the LCDR3 comprises an LCDR3 amino acid sequence in SEQ ID NO: 60.

In some embodiments, the anti-CLDN18.2 antibody or the antigen-binding fragment thereof comprises HCDR1, HCDR2, HCDR3, LCDR1, LCDR2, and LCDR3, wherein

(1) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 3, 4, 7, 8, 9, and 10, respectively;
(2) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 3, 5, 7, 8, 9, and 10, respectively;
(3) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 3, 6, 7, 8, 9, and 10, respectively;
(4) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 20, 24, 25, 26, and 27, respectively;
(5) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 21, 24, 25, 26, and 27, respectively;
(6) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 22, 24, 25, 26, and 27, respectively;
(7) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 23, 24, 25, 26, and 27, respectively;
(8) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 20, 24, 25, 26, and 28, respectively;
(9) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 21, 24, 25, 26, and 28, respectively;
(10) the HCDR1, the HCDR2, the HCDR3, the LCDR1, the LCDR2, and the LCDR3 comprise amino acid sequences

having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 22, 24, 25, 26, and 28, respectively;
(11) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 19, 23, 24, 25, 26, and 28, respectively;
(12) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 37, 38, 39, 40, 41, and 42, respectively;
(13) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 45, 46, 47, 48, 49, and 50, respectively; or
(14) the HCDR1, the HCDR2, the HCDR3, the LCDR1, the LCDR2, and the LCDR3 comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 53, 54, 55, 56, 57, and 58, respectively.

In some embodiments, the anti-CLDN18.2 antibody or the antigen-binding fragment thereof comprises HCDR1, HCDR2, HCDR3, LCDR1, LCDR2, and LCDR3, wherein

(1) the HCDR1, the HCDR2, the HCDR3, the LCDR1, the LCDR2, and the LCDR3 comprise amino acid sequences set forth in SEQ ID NOs: 3, 69, 7, 8, 9, and 10, respectively;
(2) the HCDR1, the HCDR2, the HCDR3, the LCDR1, the LCDR2, and the LCDR3 comprise amino acid sequences set forth in SEQ ID NOs: 19, 70, 24, 25, 26, and 71, respectively;
(3) the HCDR1, the HCDR2, the HCDR3, the LCDR1, the LCDR2, and the LCDR3 comprise amino acid sequences set forth in SEQ ID NOs: 37, 38, 39, 40, 41, and 42, respectively;
(4) the HCDR1, the HCDR2, the HCDR3, the LCDR1, the LCDR2, and the LCDR3 comprise amino acid sequences set forth in SEQ ID NOs: 45, 46, 47, 48, 49, and 50, respectively; or
(5) the HCDR1, the HCDR2, the HCDR3, the LCDR1, the LCDR2, and the LCDR3 comprise amino acid sequences set forth in SEQ ID NOs: 53, 54, 55, 56, 57, and 58, respectively.

In some embodiments, the anti-CLDN18.2 antibody or the antigen-binding fragment thereof comprises HCDR1, HCDR2, HCDR3, LCDR1, LCDR2, and LCDR3, wherein

(1) the HCDR1, the HCDR2, the HCDR3, the LCDR1, the LCDR2, and the LCDR3 comprise amino acid sequences set forth in SEQ ID NOs: 3, 4, 7, 8, 9, and 10, respectively;
(2) the HCDR1, the HCDR2, the HCDR3, the LCDR1, the LCDR2, and the LCDR3 comprise amino acid sequences set forth in SEQ ID NOs: 3, 5, 7, 8, 9, and 10, respectively;
(3) the HCDR1, the HCDR2, the HCDR3, the LCDR1, the LCDR2, and the LCDR3 comprise amino acid sequences set forth in SEQ ID NOs: 3, 6, 7, 8, 9, and 10, respectively;
(4) the HCDR1, the HCDR2, the HCDR3, the LCDR1, the LCDR2, and the LCDR3 comprise amino acid sequences set forth in SEQ ID NOs: 19, 20, 24, 25, 26, and 27, respectively;
(5) the HCDR1, the HCDR2, the HCDR3, the LCDR1, the LCDR2, and the LCDR3 comprise amino acid sequences set forth in SEQ ID NOs: 19, 21, 24, 25, 26, and 27, respectively;
(6) the HCDR1, the HCDR2, the HCDR3, the LCDR1, the LCDR2, and the LCDR3 comprise amino acid sequences set forth in SEQ ID NOs: 19, 22, 24, 25, 26, and 27, respectively;
(7) the HCDR1, the HCDR2, the HCDR3, the LCDR1, the LCDR2, and the LCDR3 comprise amino acid sequences set forth in SEQ ID NOs: 19, 23, 24, 25, 26, and 27, respectively;
(8) the HCDR1, the HCDR2, the HCDR3, the LCDR1, the LCDR2, and the LCDR3 comprise amino acid sequences set forth in SEQ ID NOs: 19, 20, 24, 25, 26, and 28, respectively;
(9) the HCDR1, the HCDR2, the HCDR3, the LCDR1, the LCDR2, and the LCDR3 comprise amino acid sequences set forth in SEQ ID NOs: 19, 21, 24, 25, 26, and 28, respectively;
(10) the HCDR1, the HCDR2, the HCDR3, the LCDR1, the LCDR2, and the LCDR3 comprise amino acid sequences set forth in SEQ ID NOs: 19, 22, 24, 25, 26, and 28, respectively;
(11) the HCDR1, the HCDR2, the HCDR3, the LCDR1, the LCDR2, and the LCDR3 comprise amino acid sequences set forth in SEQ ID NOs: 19, 23, 24, 25, 26, and 28, respectively;
(12) the HCDR1, the HCDR2, the HCDR3, the LCDR1, the LCDR2, and the LCDR3 comprise amino acid sequences set forth in SEQ ID NOs: 37, 38, 39, 40, 41, and 42, respectively;
(13) the HCDR1, the HCDR2, the HCDR3, the LCDR1, the LCDR2, and the LCDR3 comprise amino acid sequences

set forth in SEQ ID NOs: 45, 46, 47, 48, 49, and 50, respectively; or
(14) the HCDR1, the HCDR2, the HCDR3, the LCDR1, the LCDR2, and the LCDR3 comprise amino acid sequences set forth in SEQ ID NOs: 53, 54, 55, 56, 57, and 58, respectively.

In some embodiments, the anti-CLDN18.2 antibody or the antigen-binding fragment thereof comprises HCDR1, HCDR2, HCDR3, LCDR1, LCDR2, and LCDR3, wherein

(1) amino acid sequences of the HCDR1, the HCDR2, the HCDR3, the LCDR1, the LCDR2, and the LCDR3 are set forth in SEQ ID NOs: 3, 4, 7, 8, 9, and 10, respectively;
(2) amino acid sequences of the HCDR1, the HCDR2, the HCDR3, the LCDR1, the LCDR2, and the LCDR3 are set forth in SEQ ID NOs: 3, 5, 7, 8, 9, and 10, respectively;
(3) amino acid sequences of the HCDR1, the HCDR2, the HCDR3, the LCDR1, the LCDR2, and the LCDR3 are set forth in SEQ ID NOs: 3, 6, 7, 8, 9, and 10, respectively;
(4) amino acid sequences of the HCDR1, the HCDR2, the HCDR3, the LCDR1, the LCDR2, and the LCDR3 are set forth in SEQ ID NOs: 19, 20, 24, 25, 26, and 27, respectively;
(5) amino acid sequences of the HCDR1, the HCDR2, the HCDR3, the LCDR1, the LCDR2, and the LCDR3 are set forth in SEQ ID NOs: 19, 21, 24, 25, 26, and 27, respectively;
(6) amino acid sequences of the HCDR1, the HCDR2, the HCDR3, the LCDR1, the LCDR2, and the LCDR3 are set forth in SEQ ID NOs: 19, 22, 24, 25, 26, and 27, respectively;
(7) amino acid sequences of the HCDR1, the HCDR2, the HCDR3, the LCDR1, the LCDR2, and the LCDR3 are set forth in SEQ ID NOs: 19, 23, 24, 25, 26, and 27, respectively;
(8) amino acid sequences of the HCDR1, the HCDR2, the HCDR3, the LCDR1, the LCDR2, and the LCDR3 are set forth in SEQ ID NOs: 19, 20, 24, 25, 26, and 28, respectively;
(9) amino acid sequences of the HCDR1, the HCDR2, the HCDR3, the LCDR1, the LCDR2, and the LCDR3 are set forth in SEQ ID NOs: 19, 21, 24, 25, 26, and 28, respectively;
(10) amino acid sequences of the HCDR1, the HCDR2, the HCDR3, the LCDR1, the LCDR2, and the LCDR3 are set forth in SEQ ID NOs: 19, 22, 24, 25, 26, and 28, respectively;
(11) amino acid sequences of the HCDR1, the HCDR2, the HCDR3, the LCDR1, the LCDR2, and the LCDR3 are set forth in SEQ ID NOs: 19, 23, 24, 25, 26, and 28, respectively;
(12) amino acid sequences of the HCDR1, the HCDR2, the HCDR3, the LCDR1, the LCDR2, and the LCDR3 are set forth in SEQ ID NOs: 37, 38, 39, 40, 41, and 42, respectively;
(13) amino acid sequences of the HCDR1, the HCDR2, the HCDR3, the LCDR1, the LCDR2, and the LCDR3 are set forth in SEQ ID NOs: 45, 46, 47, 48, 49, and 50, respectively; or
(14) amino acid sequences of the HCDR1, the HCDR2, the HCDR3, the LCDR1, the LCDR2, and the LCDR3 are set forth in SEQ ID NOs: 53, 54, 55, 56, 57, and 58, respectively.

In some embodiments, the anti-CLDN18.2 antibody or the antigen-binding fragment thereof comprises a heavy chain variable region and a light chain variable region, wherein

(1) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 11 and 16, respectively;
(2) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 12 and 17, respectively;
(3) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 13 and 17, respectively;
(4) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 14 and 17, respectively;
(5) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 15 and 17, respectively;
(6) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 12 and 18, respectively;
(7) the heavy chain variable region and the light chain variable region comprise amino acid sequences having at least

80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 13 and 18, respectively;

(8) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 14 and 18, respectively;

(9) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 15 and 18, respectively;

(10) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 29 and 34, respectively;

(11) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 30 and 35, respectively;

(12) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 31 and 35, respectively;

(13) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 32 and 35, respectively;

(14) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 33 and 35, respectively;

(15) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 30 and 36, respectively;

(16) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 31 and 36, respectively;

(17) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 32 and 36, respectively;

(18) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 33 and 36, respectively;

(19) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 43 and 44, respectively;

(20) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 51 and 52, respectively; or

(21) the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to amino acid sequences set forth in SEQ ID NOs: 59 and 60, respectively.

In some embodiments, compared with an amino acid sequence set forth in any one of SEQ ID NOs: 11 to 18, 29 to 36, 43 to 44, 51 to 52, and 59 to 60, amino acids that differ in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity predominantly (or entirely) present in a FR region (framework region).

In some embodiments, the anti-CLDN18.2 antibody or the antigen-binding fragment thereof comprises a heavy chain variable region and a light chain variable region, wherein

(1) the heavy chain variable region and the light chain variable region comprise amino acid sequences set forth in SEQ ID NOs: 11 and 16, respectively;

(2) the heavy chain variable region and the light chain variable region comprise amino acid sequences set forth in SEQ ID NOs: 12 and 17, respectively;

(3) the heavy chain variable region and the light chain variable region comprise amino acid sequences set forth in SEQ ID NOs: 13 and 17, respectively;

(4) the heavy chain variable region and the light chain variable region comprise amino acid sequences set forth in SEQ ID NOs: 14 and 17, respectively;
(5) the heavy chain variable region and the light chain variable region comprise amino acid sequences set forth in SEQ ID NOs: 15 and 17, respectively;
(6) the heavy chain variable region and the light chain variable region comprise amino acid sequences set forth in SEQ ID NOs: 12 and 18, respectively;
(7) the heavy chain variable region and the light chain variable region comprise amino acid sequences set forth in SEQ ID NOs: 13 and 18, respectively;
(8) the heavy chain variable region and the light chain variable region comprise amino acid sequences set forth in SEQ ID NOs: 14 and 18, respectively;
(9) the heavy chain variable region and the light chain variable region comprise amino acid sequences set forth in SEQ ID NOs: 15 and 18, respectively;
(10) the heavy chain variable region and the light chain variable region comprise amino acid sequences set forth in SEQ ID NOs: 29 and 34, respectively;
(11) the heavy chain variable region and the light chain variable region comprise amino acid sequences set forth in SEQ ID NOs: 30 and 35, respectively;
(12) the heavy chain variable region and the light chain variable region comprise amino acid sequences set forth in SEQ ID NOs: 31 and 35, respectively;
(13) the heavy chain variable region and the light chain variable region comprise amino acid sequences set forth in SEQ ID NOs: 32 and 35, respectively;
(14) the heavy chain variable region and the light chain variable region comprise amino acid sequences set forth in SEQ ID NOs: 33 and 35, respectively;
(15) the heavy chain variable region and the light chain variable region comprise amino acid sequences set forth in SEQ ID NOs: 30 and 36, respectively;
(16) the heavy chain variable region and the light chain variable region comprise amino acid sequences set forth in SEQ ID NOs: 31 and 36, respectively;
(17) the heavy chain variable region and the light chain variable region comprise amino acid sequences set forth in SEQ ID NOs: 32 and 36, respectively;
(18) the heavy chain variable region and the light chain variable region comprise amino acid sequences set forth in SEQ ID NOs: 33 and 36, respectively;
(19) the heavy chain variable region and the light chain variable region comprise amino acid sequences set forth in SEQ ID NOs: 43 and 44, respectively;
(20) the heavy chain variable region and the light chain variable region comprise amino acid sequences set forth in SEQ ID NOs: 51 and 52, respectively; or
(21) the heavy chain variable region and the light chain variable region comprise amino acid sequences set forth in SEQ ID NOs: 59 and 60, respectively.

Herein, various variants of the anti-CLDN18.2 antibody or the antigen-binding fragment thereof described above retain the ability to specifically bind to the antigen CLDN18.2.

Table S1 provides some exemplary CDR sequences and variable region sequences of anti-CLDN18.2 antibodies or antigen-binding fragments thereof. Some anti-CLDN18.2 antibodies or antigen-binding fragments thereof share identical CDRs, while some anti-CLDN18.2 antibodies or antigen-binding fragments thereof share identical VHs or VLs.

Table S1. Amino acid sequence IDs (SEQ ID NOs.) of variable regions and CDRs

<table>
<tr><th>Antibody</th><th>HCDR1</th><th>HCDR2</th><th>HCDR3</th><th>VH</th><th>LCDR1</th><th>LCDR2</th><th>LCDR3</th><th>VL</th></tr>
<tr><td>Mouse and chimeric 28G3</td><td rowspan="9">3</td><td>4</td><td rowspan="9">7</td><td>11</td><td rowspan="9">8</td><td rowspan="9">9</td><td rowspan="9">10</td><td>16</td></tr>
<tr><td>hz28G3-1.1</td><td>5</td><td>12</td><td rowspan="4">17</td></tr>
<tr><td>hz28G3-1.2</td><td>6</td><td>13</td></tr>
<tr><td>hz28G3-1.3</td><td>5</td><td>14</td></tr>
<tr><td>hz28G3-1.4</td><td>6</td><td>15</td></tr>
<tr><td>hz28G3-2.1</td><td>5</td><td>12</td><td rowspan="4">18</td></tr>
<tr><td>hz28G3-2.2</td><td>6</td><td>13</td></tr>
<tr><td>hz28G3-2.3</td><td>5</td><td>14</td></tr>
<tr><td>hz28G3-2.4</td><td>6</td><td>15</td></tr>
</table>

(continued)

<table>
<tr><th>Antibody</th><th>HCDR1</th><th>HCDR2</th><th>HCDR3</th><th>VH</th><th>LCDR1</th><th>LCDR2</th><th>LCDR3</th><th>VL</th></tr>
<tr><td>Mouse and chimeric 40F6</td><td rowspan="9">19</td><td>20</td><td rowspan="9">24</td><td>29</td><td rowspan="9">25</td><td rowspan="9">26</td><td rowspan="5">27</td><td>34</td></tr>
<tr><td>hz40F6-1.1</td><td>21</td><td>30</td><td rowspan="4">35</td></tr>
<tr><td>hz40F6-1.2</td><td>20</td><td>31</td></tr>
<tr><td>hz40F6-1.3</td><td>22</td><td>32</td></tr>
<tr><td>hz40F6-1.4</td><td>23</td><td>33</td></tr>
<tr><td>hz40F6-2.1</td><td>21</td><td>30</td><td rowspan="4">28</td><td rowspan="4">36</td></tr>
<tr><td>hz40F6-2.2</td><td>20</td><td>31</td></tr>
<tr><td>hz40F6-2.3</td><td>22</td><td>32</td></tr>
<tr><td>hz40F6-2.4</td><td>23</td><td>33</td></tr>
<tr><td>Mouse and chimeric 34G6</td><td>37</td><td>38</td><td>39</td><td>43</td><td>40</td><td>41</td><td>42</td><td>44</td></tr>
<tr><td>Mouse and chimeric 10D8</td><td>45</td><td>46</td><td>47</td><td>51</td><td>48</td><td>49</td><td>50</td><td>52</td></tr>
<tr><td>Mouse and chimeric 22F12</td><td>53</td><td>54</td><td>55</td><td>59</td><td>56</td><td>57</td><td>58</td><td>60</td></tr>
</table>

For the amino acid sequence of the variable region of an antibody, those skilled in the art can determine which residues constitute a particular CDR by conventional methods. It is well known to those skilled in the art that the CDRs of an antibody may be defined by a variety of methods, for example, by the numbering system/method of Kabat, Chothia, IMGT, AbM, or Contact; or by a combination of two or more of the numbering system/method of Kabat, Chothia, IMGT, AbM, or Contact; it may also be defined by a combined numbering system comprising Kabat and Chothia, wherein the combined numbering system combines the ranges defined by Kabat and Chothia and takes a greater range on this basis (e.g., if Kabat defines HCDR1 as H31-H35, and Chothia defines HCDR1 as H26-H32, the combined system defines HCDR1 as H26-H35). The exact numbering and position of the CDRs varies among different numbering systems. It should be understood by those skilled in the art that, unless otherwise specified, the term "CDR" or "complementarity determining region" of a given antibody or a region thereof (e.g., a variable region) will be understood to encompass complementarity determining regions defined by any of the known schemes. Although the CDRs claimed in the present disclosure are based on the sequences shown in Table S1 (as one definition scheme), the corresponding amino acid sequences according to other CDR definition rules shall also fall within the protection scope of the present disclosure.

In one embodiment, the antibody or the antigen-binding fragment thereof of the present disclosure is selected from a 28G3 antibody or an antigen-binding fragment thereof, a 40F6 antibody or an antigen-binding fragment thereof, a 34G6 antibody or an antigen-binding fragment thereof, a 10D8 antibody or an antigen-binding fragment thereof, and a 22F12 antibody or an antigen-binding fragment thereof, wherein

the 28G3 antibody or the antigen-binding fragment thereof comprises HCDR1, HCDR2, HCDR3, LCDR1, LCDR2, and LCDR3, which comprise amino acid sequences set forth in SEQ ID NOs: 3, 69, 7, 8, 9, and 10, respectively; preferably, the 28G3 antibody or the antigen-binding fragment thereof comprises HCDR1, HCDR2, HCDR3, LCDR1, LCDR2, and LCDR3, wherein (1) amino acid sequences of the HCDR1, the HCDR2, the HCDR3, the LCDR1, the LCDR2, and the LCDR3 are set forth in SEQ ID NOs: 3, 4, 7, 8, 9, and 10, respectively; (2) amino acid sequences of the HCDR1, the HCDR2, the HCDR3, the LCDR1, the LCDR2, and the LCDR3 are set forth in SEQ ID NOs: 3, 5, 7, 8, 9, and 10, respectively; or (3) amino acid sequences of the HCDR1, the HCDR2, the HCDR3, the LCDR1, the LCDR2, and the LCDR3 are set forth in SEQ ID NOs: 3, 6, 7, 8, 9, and 10, respectively;

the 40F6 antibody or the antigen-binding fragment thereof comprises HCDR1, HCDR2, HCDR3, LCDR1, LCDR2, and LCDR3, which comprise amino acid sequences set forth in SEQ ID NOs: 19, 70, 24, 25, 26, and 71, respectively; preferably, the 40F6 antibody or the antigen-binding fragment thereof comprises HCDR1, HCDR2, HCDR3, LCDR1, LCDR2, and LCDR3, wherein (1) amino acid sequences of the HCDR1, the HCDR2, the HCDR3, the LCDR1, the LCDR2, and the LCDR3 are set forth in SEQ ID NOs: 19, 20, 24, 25, 26, and 27, respectively; (2) amino acid sequences of the HCDR1, the HCDR2, the HCDR3, the LCDR1, the LCDR2, and the LCDR3 are set forth in SEQ ID NOs: 19, 21, 24, 25, 26, and 27, respectively; (3) amino acid sequences of the HCDR1, the HCDR2, the HCDR3, the LCDR1, the LCDR2, and the LCDR3 are set forth in SEQ ID NOs: 19, 22, 24, 25, 26, and 27, respectively; (4) amino acid sequences of the HCDR1, the HCDR2, the HCDR3, the LCDR1, the LCDR2, and the LCDR3 are set forth in SEQ ID NOs: 19, 23, 24, 25, 26, and 27, respectively; (5) amino acid sequences of the HCDR1, the HCDR2, the HCDR3, the LCDR1, the LCDR2, and the LCDR3 are set forth in SEQ ID NOs: 19, 20, 24, 25, 26, and 28, respectively; (6) amino

acid sequences of the HCDR1, the HCDR2, the HCDR3, the LCDR1, the LCDR2, and the LCDR3 are set forth in SEQ ID NOs: 19, 21, 24, 25, 26, and 28, respectively; (7) amino acid sequences of the HCDR1, the HCDR2, the HCDR3, the LCDR1, the LCDR2, and the LCDR3 are set forth in SEQ ID NOs: 19, 22, 24, 25, 26, and 28, respectively; or (8) amino acid sequences of the HCDR1, the HCDR2, the HCDR3, the LCDR1, the LCDR2, and the LCDR3 are set forth in SEQ ID NOs: 19, 23, 24, 25, 26, and 28, respectively;

the 34G6 antibody or the antigen-binding fragment thereof comprises HCDR1, HCDR2, HCDR3, LCDR1, LCDR2, and LCDR3, which comprise amino acid sequences set forth in 37, 38, 39, 40, 41, and 42, respectively; the 10D8 antibody or the antigen-binding fragment thereof comprises HCDR1, HCDR2, HCDR3, LCDR1, LCDR2, and LCDR3, which comprise amino acid sequences set forth in 45, 46, 47, 48, 49, and 50, respectively; or

the 22F12 antibody or the antigen-binding fragment thereof comprises HCDR1, HCDR2, HCDR3, LCDR1, LCDR2, and LCDR3, which comprise amino acid sequences set forth in 53, 54, 55, 56, 57, and 58, respectively;

In some preferred embodiments, the 28G3 antibody or the antigen-binding fragment thereof comprises a heavy chain variable region and a light chain variable region, wherein the heavy chain variable region and the light chain variable region comprise amino acid sequences set forth in SEQ ID NOs: 11 and 16, SEQ ID NOs: 12 and 17, SEQ ID NOs: 13 and 17, SEQ ID NOs: 14 and 17, SEQ ID NOs: 15 and 17, SEQ ID NOs: 12 and 18, SEQ ID NOs: 13 and 18, SEQ ID NOs: 14 and 18, or SEQ ID NOs: 15 and 18, respectively;

the 40F6 antibody or the antigen-binding fragment thereof comprises a heavy chain variable region and a light chain variable region, wherein the heavy chain variable region and the light chain variable region comprise amino acid sequences set forth in SEQ ID NOs: 29 and 34, SEQ ID NOs: 30 and 35, SEQ ID NOs: 31 and 35, SEQ ID NOs: 32 and 35, SEQ ID NOs: 33 and 35, SEQ ID NOs: 30 and 36, SEQ ID NOs: 31 and 36, SEQ ID NOs: 32 and 36, or SEQ ID NOs: 33 and 36, respectively;

the 34G6 antibody or the antigen-binding fragment thereof comprises a heavy chain variable region and a light chain variable region, wherein the heavy chain variable region and the light chain variable region comprise amino acid sequences set forth in SEQ ID NOs: 43 and 44, respectively;

the 10D8 antibody or the antigen-binding fragment thereof comprises a heavy chain variable region and a light chain variable region, wherein the heavy chain variable region and the light chain variable region comprise amino acid sequences set forth in SEQ ID NOs: 51 and 52, respectively; or

the 22F12 antibody or the antigen-binding fragment thereof comprises a heavy chain variable region and a light chain variable region, wherein the heavy chain variable region and the light chain variable region comprise amino acid sequences set forth in SEQ ID NOs: 59 and 60, respectively.

VH and/or VL sequences (or CDR sequences) of other antibodies that bind to CLDN18.2 may be "mixed and paired" with VH and/or VL sequences (or CDR sequences) of the anti-CLDN18.2 antibody or the antigen-binding fragment thereof of the present disclosure. Preferably, when VH and VL chains (or CDRs thereof) are mixed and paired, the VH sequence in a particular VH/VL pair can be substituted with a structurally similar VH sequence. Likewise, it is preferred to substitute the VL sequence in a particular VH/VL pair with a structurally similar VL sequence.

Thus, in one embodiment, the antibody or the antigen-binding fragment thereof of the present disclosure comprises:

(a) a heavy chain variable region comprising the amino acid sequence listed in Table S1; and

(b) a light chain variable region comprising the amino acid sequence listed in Table S1, or VL of another anti-CLDN18.2 antibody, wherein the antibody or the antigen-binding fragment thereof specifically binds to CLDN18.2.

In another embodiment, the antibody or the antigen-binding fragment thereof of the present disclosure comprises:

(a) the HCDR1, the HCDR2, and the HCDR3 listed in Table S1; and

(b) the LCDR1, the LCDR2, and the LCDR3 listed in Table S1, or light chain variable region CDRs of another anti-CLDN18.2 antibody, wherein the antibody or the antigen-binding fragment thereof specifically binds to CLDN18.2.

In another embodiment, the antibody or the antigen-binding fragment thereof of the present disclosure comprises the HCDR2 listed in Table S1 and CDR(s) of other anti-CLDN18.2 antibodies, e.g., HCDR1 and/or HCDR3 of other anti-CLDN18.2 antibodies and/or LCDR1, LCDR2, and/or LCDR3 of other anti-CLDN18.2 antibodies.

Furthermore, it is well known in the art that the CDR3 domain is independent of the CDR1 and/or CDR2 domains and can independently determine the antibody's binding specificity for an alloantigen, and that multiple antibodies with the same binding specificity can be predicted based on the CDR3 sequence.

In another embodiment, the antibody or the antigen-binding fragment thereof of the present disclosure comprises the HCDR2 listed in Table S1, the HCDR3 listed in Table S1, and/or the LCDR3 listed in Table S1, and CDR(s) of other anti-CLDN18.2 antibodies, wherein the antibody or the antigen-binding fragment thereof specifically binds to CLDN18.2. In another embodiment, the antibody or the antigen-binding fragment thereof of the present disclosure further comprises the LCDR2 listed in Table S1 and CDR(s) of other anti-CLDN18.2 antibodies, wherein the antibody or the antigen-binding fragment thereof specifically binds to CLDN18.2. In another embodiment, the antibody or the antigen-binding fragment thereof of the present disclosure further comprises the HCDR1 listed in Table S1 or the LCDR1 listed in Table S1, and CDR(s) of other anti-CLDN18.2 antibodies, wherein the antibody or the antigen-binding fragment thereof specifically binds to CLDN18.2. These antibodies preferably (a) compete for binding to CLDN18.2 with, (b) retain the same functional properties as, (c) bind to the same epitope as, and/or (d) have similar binding affinities as the anti-CLDN18.2 antibody of the present disclosure.

In some embodiments, the anti-CLDN18.2 antibody or the antigen-binding fragment thereof further comprises a heavy chain constant region and a light chain constant region, wherein the heavy chain constant region comprises a human IgG1, IgG2, or IgG4 constant region or a variant thereof, preferably a human IgG1 constant region or a variant thereof, and the light chain constant region comprises a human $\kappa$ constant region, a human $\lambda$ constant region, or a variant thereof, preferably a human $\kappa$ constant region or a variant thereof. Exemplary variants include IgG1, IgG2, or IgG4 heavy chain constant region variants with site-directed engineering and amino acid substitutions of the heavy chain constant region, such as AAA mutations, DLE mutations (Shields *et al.,* 2002; Lazar *et al.,* 2006), YTE mutations, and LS mutations (Ghetie *et al.,* 1997; Zalevsky *et al.,* 2010) known in the art. The C-terminal lysine of the heavy chain constant region may be present or absent.

In some embodiments, the heavy chain constant region comprises an amino acid sequence set forth in SEQ ID NO: 1 or an amino acid sequence having 1, 2, 3, 4, or 5 amino acid substitutions, deletions, and additions compared with the amino acid sequence set forth in SEQ ID NO: 1, and the light chain constant region comprises an amino acid sequence set forth in SEQ ID NO: 2 or an amino acid sequence having 1, 2, 3, 4, or 5 amino acid substitutions, deletions, and additions compared with the amino acid sequence set forth in SEQ ID NO: 2.

Herein, the amino acid substitution may be a conservative amino acid substitution, i.e., a substitution with another amino acid of the same class (having similar chemical properties or functions). By way of example, amino acids can be classified as follows according to their side chain properties: (1) non-polar amino acids: Ala (A), Val (V), Leu (L), Ile (I), Pro (P), Phe (F), Trp (W), and Met (M); (2) uncharged polar amino acids: Gly (G), Ser (S), Thr (T), Cys (C), Tyr (Y), Asn (N), and Gln (Q); (3) acidic amino acids: Asp (D) and Glu (E); (4) basic amino acids: Lys (K), Arg (R), and His (H). Alternatively, amino acids can be classified as follows based on common side chain properties: (1) hydrophobic amino acids: Met, Ala, Val, Leu, and Ile; (2) neutral hydrophilic amino acids: Cys, Ser, Thr, Asn, and Gln; (3) acidic amino acids: Asp and Glu; (4) basic amino acids: His, Lys, and Arg; (5) amino acids that influence chain orientation: Gly and Pro; (6) aromatic amino acids: Trp, Tyr, and Phe.

In some embodiments, the anti-CLDN18.2 antibody is a full-length antibody, e.g., of an IgG1 isotype. In another embodiment, the anti-CLDN18.2 antibody or the antigen-binding fragment thereof is a Fab fragment, a F(ab')2 fragment, an Fd fragment, an Fv fragment, a dAb, a single-chain Fv (scFv) molecule, or a nanobody.

In some embodiments, the anti-CLDN18.2 antibody comprises a heavy chain and a light chain, wherein the heavy chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 61, and the light chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 62.

In some embodiments, the anti-CLDN18.2 antibody comprises a heavy chain and a light chain, wherein the heavy chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 63, and the light chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 64.

In some embodiments, the anti-CLDN18.2 antibody comprises a heavy chain and a light chain, wherein the heavy chain comprises an amino acid sequence set forth in SEQ ID NO: 61, and the light chain comprises an amino acid sequence set forth in SEQ ID NO: 62.

In some embodiments, the anti-CLDN18.2 antibody comprises a heavy chain and a light chain, wherein the heavy chain comprises an amino acid sequence set forth in SEQ ID NO: 63, and the light chain comprises an amino acid sequence set forth in SEQ ID NO: 64.

It should be understood that some anti-CLDN18.2 antibodies with a C-terminal lysine deletion of the heavy chain are also provided.

In some specific embodiments, in the antibody-drug conjugate of general formula $Ab\text{-}(L\text{-}U)_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising

HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 12, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 17. Further, in these specific embodiments, the anti-CLDN18.2 antibody or the antigen-binding fragment thereof comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some other specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 14, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 17. Further, in these specific embodiments, the anti-CLDN18.2 antibody or the antigen-binding fragment thereof comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region comprises an amino acid sequence set forth in SEQ ID NO: 12, and the light chain variable region comprises an amino acid sequence set forth in SEQ ID NO: 17. In some other specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region comprises an amino acid sequence set forth in SEQ ID NO: 14, and the light chain variable region comprises an amino acid sequence set forth in SEQ ID NO: 17.

In some specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region has an amino acid sequence set forth in SEQ ID NO: 12, and the light chain variable region has an amino acid sequence set forth in SEQ ID NO: 17. In some other specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody or an antigen-binding fragment thereof comprising a heavy chain variable region and a light chain variable region, wherein the heavy chain variable region has an amino acid sequence set forth in SEQ ID NO: 14, and the light chain variable region has an amino acid sequence set forth in SEQ ID NO: 17.

In some specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody comprising a heavy chain and a light chain, wherein the heavy chain comprises an amino acid sequence set forth in SEQ ID NO: 61, and the light chain comprises an amino acid sequence set forth in SEQ ID NO: 62. In some other specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody comprising a heavy chain and a light chain, wherein the heavy chain comprises an amino acid sequence set forth in SEQ ID NO: 63, and the light chain comprises an amino acid sequence set forth in SEQ ID NO: 64.

In some specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody comprising a heavy chain and a light chain, wherein the

heavy chain has an amino acid sequence set forth in SEQ ID NO: 61, and the light chain has an amino acid sequence set forth in SEQ ID NO: 62. In some other specific embodiments, in the antibody-drug conjugate of general formula Ab-(L-U)$_n$ or the pharmaceutically acceptable salt or the solvate thereof provided by the present disclosure, L is a linker, U is a cytotoxic drug, n is an integer selected from 1 to 10, and Ab is an anti-CLDN18.2 antibody comprising a heavy chain and a light chain, wherein the heavy chain has an amino acid sequence set forth in SEQ ID NO: 63, and the light chain has an amino acid sequence set forth in SEQ ID NO: 64. In some other specific embodiments, the C-terminal Lys of the heavy chain (e.g., SEQ ID NO: 63 or SEQ ID NO: 61) is absent.

In some embodiments, the anti-CLDN18.2 antibody comprises or consists of two heavy (H) chains and two light (L) chains that are linked via disulfide bonds, wherein each of the heavy chains comprises the heavy chain variable region (VH) and the heavy chain constant region described above, wherein the heavy chain variable region (VH) comprises framework regions (FRs) and the heavy chain complementarity determining regions (HCDRs) described above; each of the light chains comprises the light chain variable region (VL) and the light chain constant region described above, wherein the light chain variable region (VL) comprises FRs and the light chain complementarity determining regions (LCDRs) described above; the C-terminus of the heavy chain variable region is linked to the N-terminus of the heavy chain constant region, and the C-terminus of the light chain variable region is linked to the N-terminus of the light chain constant region.

In some embodiments, the anti-CLDN18.2 antibody or the antigen-binding fragment thereof may be modified, such as including one or more amino acid alterations, additions, or deletions. Herein, the modified antibody or antigen-binding fragment thereof still retains the activity of specifically binding to its corresponding antigen.

In the antibody-drug conjugate of general formula Ab-(L-U)$_n$ or the pharmaceutically acceptable salt or the solvate thereof provided by the present disclosure, Ab is an antibody or an antigen-binding fragment thereof, wherein the antibody or the antigen-binding fragment thereof binds to CLDN18.2, thereby inducing antibody-dependent cell-mediated cytotoxicity (ADCC) and/or complement-dependent cytotoxicity (CDC) against cells expressing CLDN18.2. In some embodiments, in the antibody-drug conjugate of general formula Ab-(L-U)$_n$ or the pharmaceutically acceptable salt or the solvate thereof, the antibody or the antigen-binding fragment thereof binds to human CLDN18.2, thereby inducing ADCC and CDC against cells expressing human CLDN18.2. In some embodiments, in the antibody-drug conjugate of general formula Ab-(L-U)$_n$ or the pharmaceutically acceptable salt or the solvate thereof, the antibody or the antigen-binding fragment thereof does not bind to CLDN18.1. In some embodiments, in the antibody-drug conjugate of general formula Ab-(L-U)$_n$ or the pharmaceutically acceptable salt or the solvate thereof, the antibody or the antigen-binding fragment thereof does not bind to human CLDN18.1. In some embodiments, the antibody-drug conjugate of general formula Ab-(L-U)$_n$ or the pharmaceutically acceptable salt or the solvate thereof does not induce ADCC and/or CDC against cells expressing CLDN18.1. In some embodiments, the antibody-drug conjugate of general formula Ab-(L-U)$_n$ or the pharmaceutically acceptable salt or the solvate thereof does not induce ADCC and/or CDC against cells expressing human CLDN18.1.

In the antibody-drug conjugate of general formula Ab-(L-U)$_n$ or the pharmaceutically acceptable salt or the solvate thereof provided by the present disclosure, the cytotoxic drug U is conjugated to the antibody or the antigen-binding fragment thereof Ab via the linker L. In some specific embodiments, in the antibody-drug conjugate or the pharmaceutically acceptable salt or the solvate thereof provided by the present disclosure, each cytotoxic drug U is conjugated to the antibody or the antigen-binding fragment thereof Ab via one linker L. The linker L of the present disclosure may be linked to the antibody or the antigen-binding fragment thereof by any method known in the art. In some preferred embodiments, the linker L is linked to the antibody or the antigen-binding fragment thereof Ab via sulfydryl and/or amino. In some more preferred embodiments, the linker L is linked to the antibody or the antigen-binding fragment thereof Ab via sulfydryl.

In some embodiments, the linker L is a cleavable linker or a non-cleavable linker. In some embodiments, the linker is a cleavable linker, which may be, for example, a low pH-dependent degradation type (including a hydrazone bond, a carbonate bond, and the like), a proteolytic type (including a peptide-based bond), a high glutathione concentration-dependent degradation type (including a disulfide bond), or the like. The cleavable linker may be cleaved within the target cell, thereby releasing the cytotoxic drug. In some other embodiments, the linker L is a non-cleavable linker, which may be, for example, maleimidocaproyl or the like.

In one aspect, in the antibody-drug conjugate of general formula Ab-(L-U)$_n$ or the pharmaceutically acceptable salt or the solvate thereof provided by the present disclosure, the antibody or the antigen-binding fragment thereof Ab is conjugated to one or more cytotoxic drugs U. The cytotoxic drugs may be, for example, alkaloids, antimetabolites, anti-tumor antibiotics, alkylating agents, platinum-based drugs, and the like. In some embodiments, the cytotoxic agent U is a tubulin inhibitor cytotoxic drug or a cytotoxic drug acting on DNA. In some embodiments, the tubulin inhibitor cytotoxic drug includes, but is not limited to, maytansines (e.g., DM1, and DM4), auristatins (e.g., MMAE or MMAF), and dolastatin. In some embodiments, the cytotoxic drug acting on DNA includes, but is not limited to, calicheamicins, doxorubicin, duocarmycins, pyrrolobenzodiazepine (PBD), and topoisomerase I inhibitors.

In some specific embodiments, in the antibody-drug conjugate of general formula Ab-(L-U)$_n$ or the pharmaceutically acceptable salt or the solvate thereof provided by the present disclosure, the cytotoxic drug U is a topoisomerase I inhibitor.

In some specific embodiments, in the antibody-drug conjugate of general formula Ab-(L-U)$_n$ or the pharmaceutically acceptable salt or the solvate thereof provided by the present disclosure, the cytotoxic drug U is a camptothecin topoisomerase I inhibitor.

In some specific embodiments, in the antibody-drug conjugate of general formula Ab-(L-U)$_n$ or the pharmaceutically acceptable salt or the solvate thereof provided by the present disclosure, the cytotoxic drug U is SN-38, an SN-38 derivative, exatecan, or an exatecan derivative. In some specific embodiments, the cytotoxic drug U has a deuterated modification. In some specific embodiments, the cytotoxic drug U is an exatecan derivative and has a deuterated modification.

In some embodiments, the cytotoxic drug U is linked to the linker L via a functional group, so that cytotoxic drug molecules can be liberated in tumor cells to exert an anti-tumor effect.

In one aspect, in the antibody-drug conjugate of general formula Ab-(L-U)$_n$ or the pharmaceutically acceptable salt or the solvate thereof provided by the present disclosure, Ab is an antibody or an antigen-binding fragment thereof, L is a linker, U is a camptothecin topoisomerase I inhibitor, and n is an integer selected from 1 to 10. In some embodiments, L and/or U have a deuterated modification.

In some embodiments, in the antibody-drug conjugate of general formula Ab-(L-U)$_n$ or the pharmaceutically acceptable salt or the solvate thereof provided by the present disclosure, Ab is an antibody or an antigen-binding fragment thereof, L is a linker, U is a camptothecin topoisomerase I inhibitor, and n is an integer selected from 1 to 10, wherein L and/or U have a deuterated modification, and the cytotoxic drug U is SN-38, an SN-38 derivative, exatecan, or an exatecan derivative.

In some specific embodiments, in the antibody-drug conjugate of general formula Ab-(L-U)$_n$ or the pharmaceutically acceptable salt or the solvate thereof provided by the present disclosure, Ab is an antibody or an antigen-binding fragment thereof, L is a linker, U is a camptothecin topoisomerase I inhibitor, and n is an integer selected from 1 to 10, wherein U has a deuterated modification, and the cytotoxic drug U is an exatecan derivative.

In some embodiments, in the antibody-drug conjugate of general formula Ab-(L-U)$_n$ or the pharmaceutically acceptable salt or the solvate thereof provided by the present disclosure, -U is a structure represented by formula Ia below:

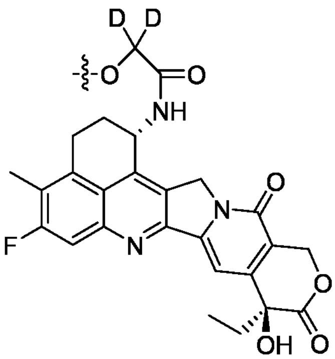
Ia.

In some embodiments, in the antibody-drug conjugate of general formula Ab-(L-U)$_n$ or the pharmaceutically acceptable salt or the solvate thereof provided by the present disclosure, -L- is a structure represented by formula IIa below:

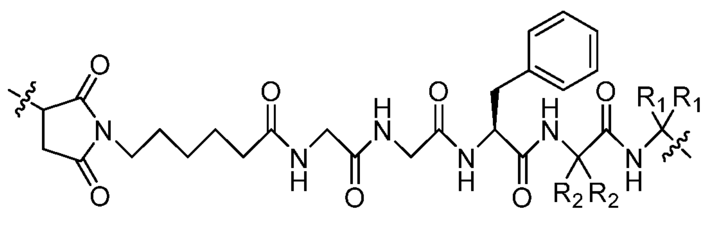
IIa,

wherein $R_1$ and $R_2$ are each independently selected from hydrogen (H) and deuterium (D); and the 3-position of -(succinimidyl-3-yl-N)- in the structure shown is linked to the antibody or the antigen-binding fragment thereof Ab, and the methylene at the other end is linked to the cytotoxic drug U.

In some embodiments, -L- is a structure represented by formula IIa-1, IIa-2, IIa-3, or IIa-4 below:

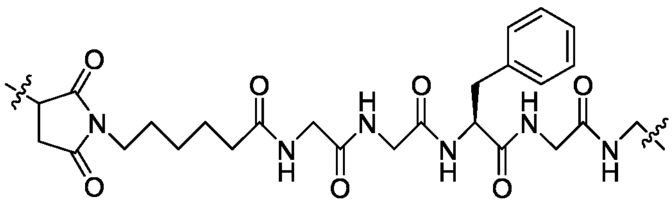

IIa-1,

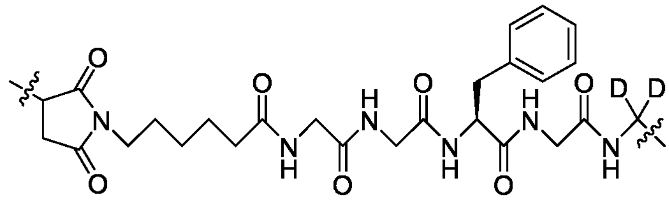

IIa-2,

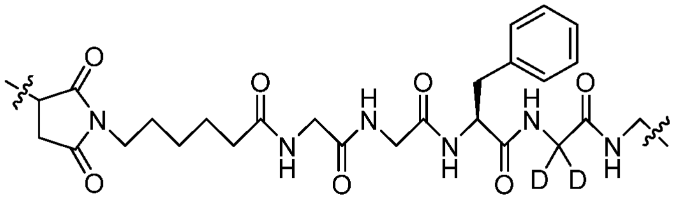

IIa-3,

or

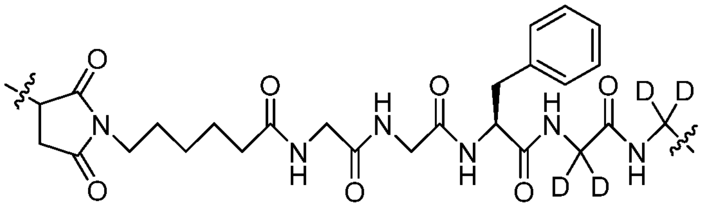

IIa-4,

wherein the 3-position of -(succinimidyl-3-yl-N)- in the structure shown is linked to the antibody or the antigen-binding fragment thereof Ab, and the methylene at the other end is linked to the cytotoxic drug U.

In some embodiments, in the antibody-drug conjugate of general formula Ab-$(L-U)_n$ or the pharmaceutically acceptable salt or the solvate thereof provided by the present disclosure, -L-U is a structure represented by formula IIIa below:

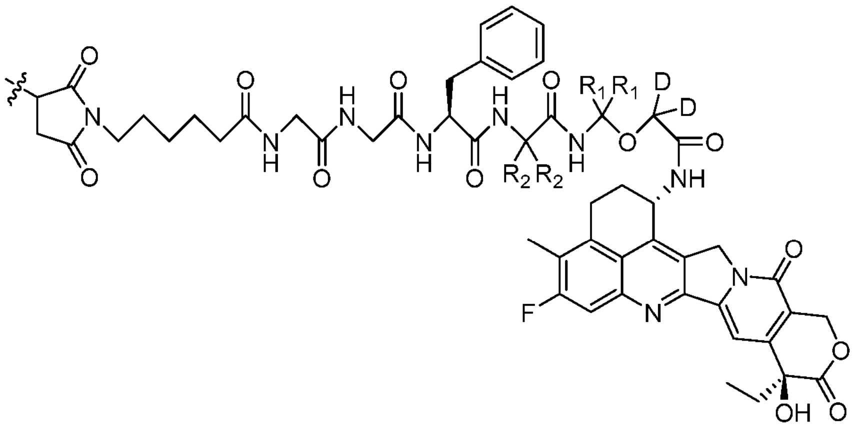

IIIa,

wherein $R_1$ and $R_2$ are each independently selected from hydrogen (H) and deuterium (D).

In some embodiments, -L-U is a structure represented by formula IIIa-1, IIIa-2, IIIa-3, or IIIa-4 below:

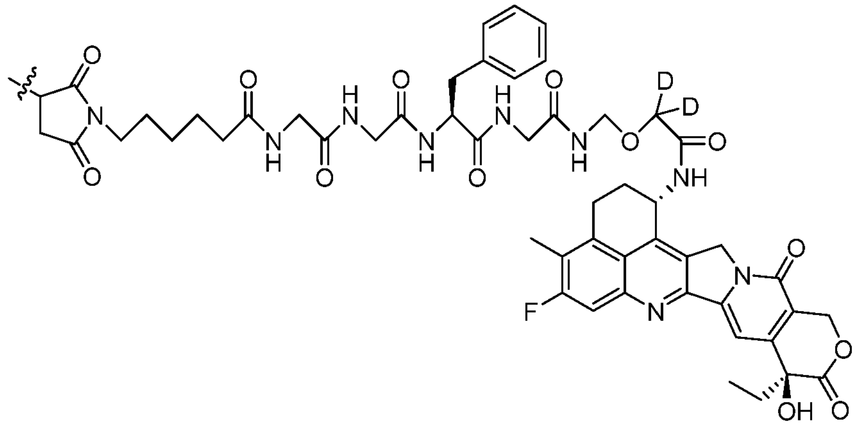

IIIa-1,

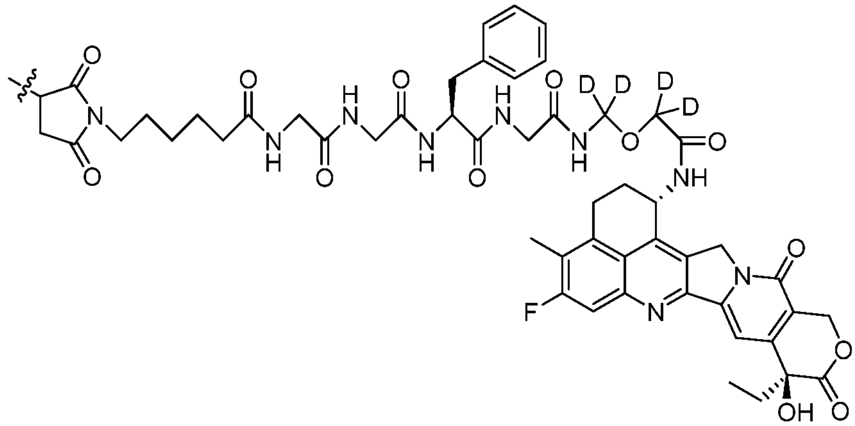

IIIa-2,

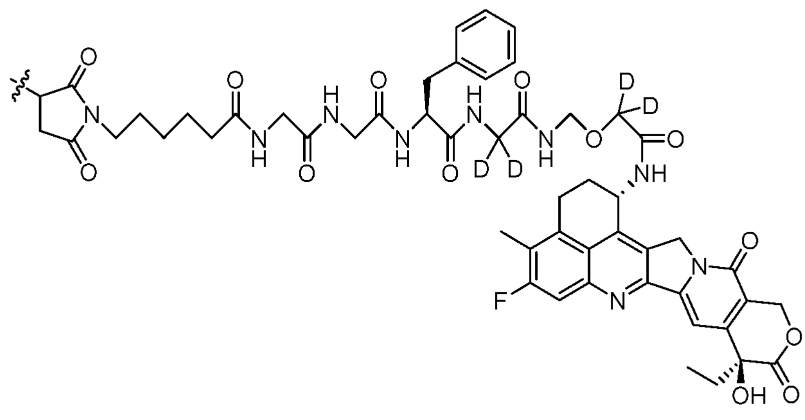

IIIa-3,

or

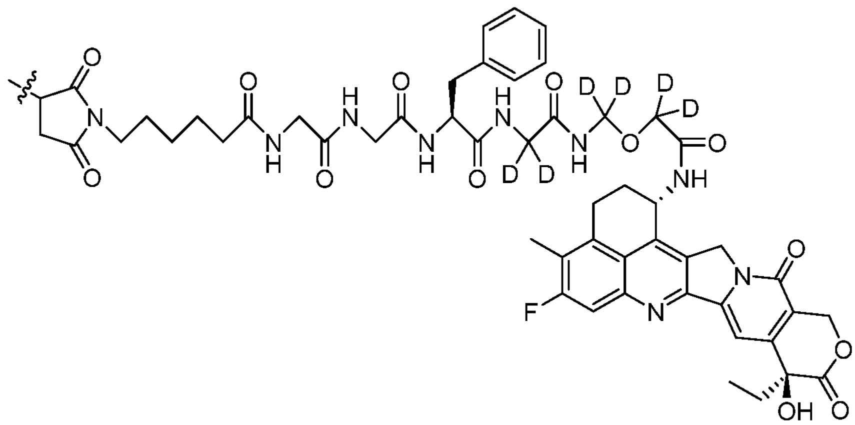

IIIa-4.

In some embodiments, in the antibody-drug conjugate of general formula Ab-(L-U)$_n$ or the pharmaceutically acceptable salt or the solvate thereof provided by the present disclosure, the antibody-drug conjugate has a structure represented by formula IV below:

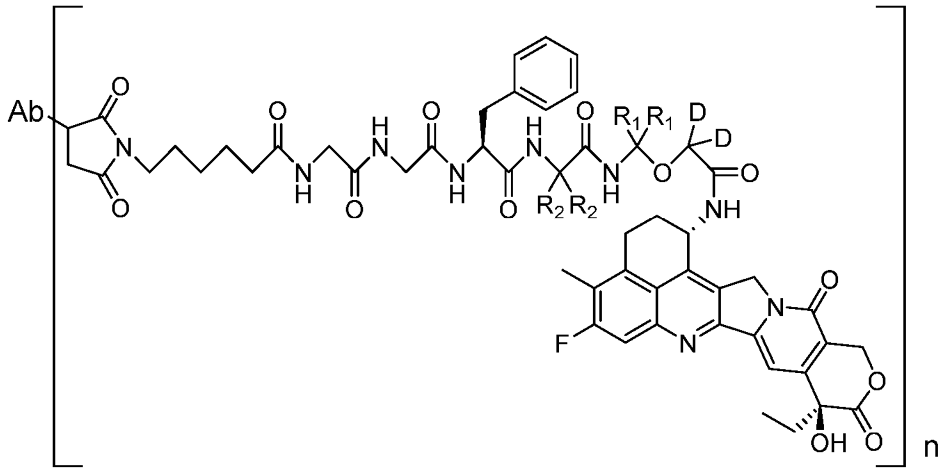

IV,

wherein Ab is an antibody or an antigen-binding fragment thereof, n is an integer selected from 1 to 10, and $R_1$ and $R_2$ are each independently selected from hydrogen (H) and deuterium (D).

In some specific embodiments, the antibody-drug conjugate has a structure represented by formula IV-1, IV-2, IV-3, or IV-4 below:

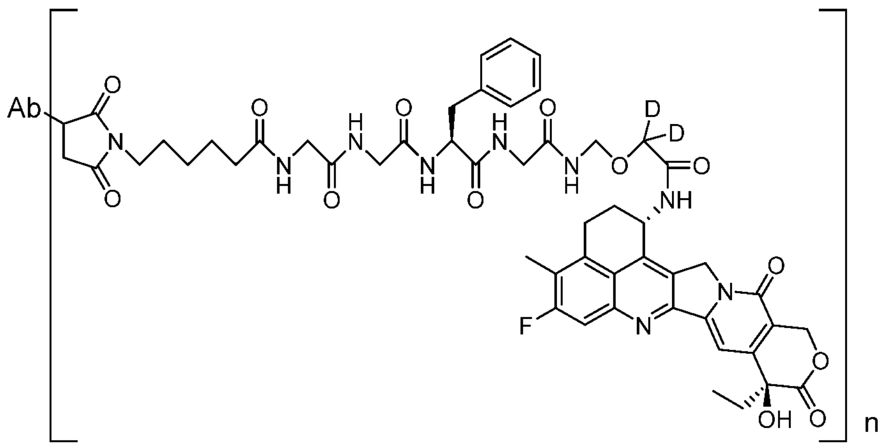

IV-1,

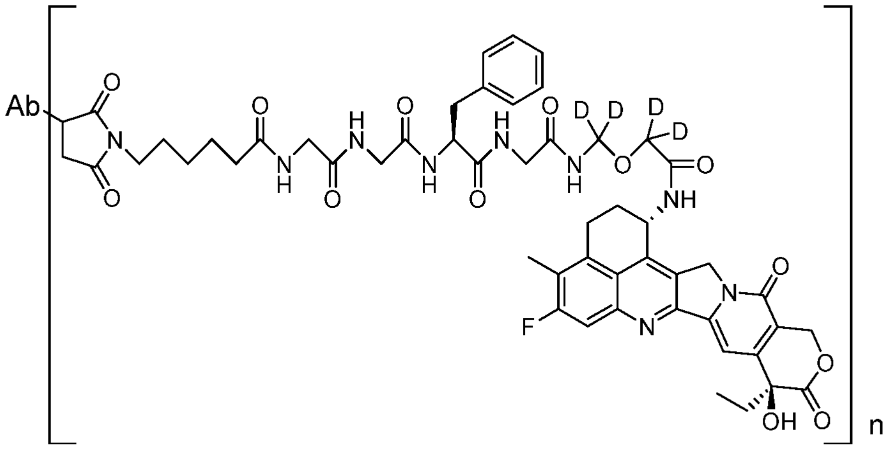

IV-2,

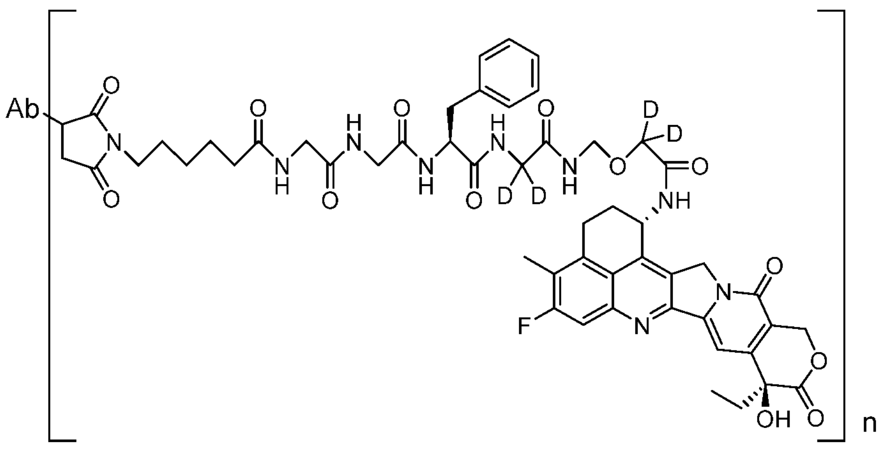

IV-3,

or

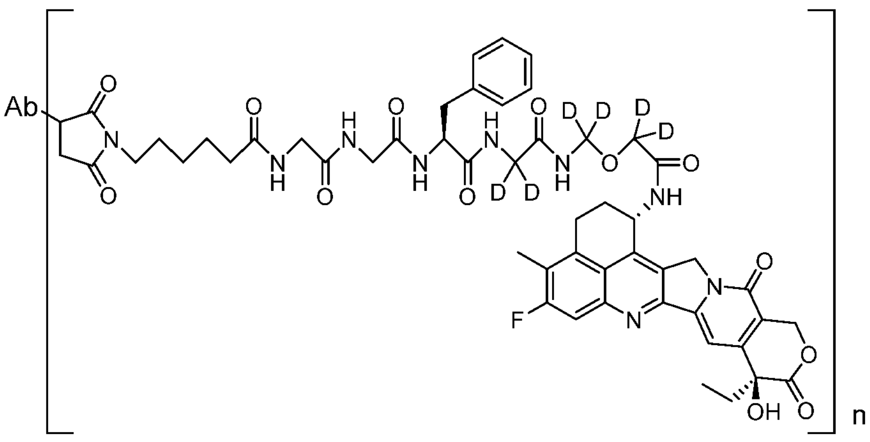

IV-4,

wherein Ab is an antibody or an antigen-binding fragment thereof, and n is an integer selected from 1 to 10.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof.

In some embodiments, in the antibody-drug conjugate or the pharmaceutically acceptable salt or the solvate thereof, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8. In some embodiments, n is an integer selected from 2 to 8, 3 to 8, 4 to 8, 5 to 8, 6 to 8, and 7 to 8.

In some embodiments, n is an integer selected from 2 to 8. In some embodiments, n is an integer selected from 4

to 8. In some embodiments, n is an integer selected from 6 to 8. In some embodiments, n is 6, 7, or 8. In some embodiments, n is 2, 4, 6, or 8. In some embodiments, n is 2, 4, or 8. In some embodiments, n is 4, 6, or 8. In some embodiments, n is 4 or 8. In some embodiments, n is 6 or 8.

In some embodiments, in the antibody-drug conjugate of general formula Ab-$(L-U)_n$ or the pharmaceutically acceptable salt or the solvate thereof provided by the present disclosure, a structure of the antibody-drug conjugate is represented by formula IV-1, wherein Ab is an anti-CLDN18.2 antibody or an antigen-binding fragment thereof comprising HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, a structure of the antibody-drug conjugate is represented by formula IV-1, wherein Ab is an anti-CLDN18.2 antibody or an antigen-binding fragment thereof comprising a heavy chain variable region and a light chain variable region, wherein the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 12 and 17, respectively. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8. Further, in some of these embodiments, the anti-CLDN18.2 antibody or the antigen-binding fragment thereof comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof comprising a heavy chain variable region and a light chain variable region, wherein the heavy chain variable region and the light chain variable region comprise amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 14 and 17, respectively. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8. Further, in some of these embodiments, the anti-CLDN18.2 antibody or the antigen-binding fragment thereof comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof comprising a heavy chain variable region and a light chain variable region, wherein the heavy chain variable region comprises an amino acid sequence set forth in SEQ ID NO: 12, and the light chain variable region comprises an amino acid sequence set forth in SEQ ID NO: 17. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof comprising a heavy chain variable region and a light chain variable region, wherein the heavy chain variable region comprises an amino acid sequence set forth in SEQ ID NO: 14, and the light chain variable region comprises an amino acid sequence set forth in SEQ ID NO: 17. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof comprising a heavy chain variable region and a light chain variable region, wherein the heavy chain variable region has an amino acid sequence set forth in SEQ ID NO: 12, and the light chain variable region has an amino acid sequence set forth in SEQ ID NO: 17. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an

integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody or an antigen-binding fragment thereof comprising a heavy chain variable region and a light chain variable region, wherein the heavy chain variable region has an amino acid sequence set forth in SEQ ID NO: 14, and the light chain variable region has an amino acid sequence set forth in SEQ ID NO: 17. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody comprising a heavy chain and a light chain, wherein the heavy chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 61, and the light chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 62. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8. Further, in some of these embodiments, the anti-CLDN18.2 antibody comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody comprising a heavy chain and a light chain, wherein the heavy chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 63, and the light chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 64. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8. Further, in some of these embodiments, the anti-CLDN18.2 antibody comprises HCDR1 set forth in SEQ ID NO: 3, HCDR2 set forth in SEQ ID NO: 5, HCDR3 set forth in SEQ ID NO: 7, LCDR1 set forth in SEQ ID NO: 8, LCDR2 set forth in SEQ ID NO: 9, and LCDR3 set forth in SEQ ID NO: 10.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody comprising a heavy chain and a light chain, wherein the heavy chain comprises an amino acid sequence set forth in SEQ ID NO: 61, and the light chain comprises an amino acid sequence set forth in SEQ ID NO: 62. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody comprising a heavy chain and a light chain, wherein the heavy chain comprises an amino acid sequence set forth in SEQ ID NO: 63, and the light chain comprises an amino acid sequence set forth in SEQ ID NO: 64. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure represented by formula IV-1, wherein Ab is an anti-CLDN18.2 antibody comprising a heavy chain and a light chain, wherein the heavy chain has an amino acid sequence set forth in SEQ ID NO: 61, and the light chain has an amino acid sequence set forth in SEQ ID NO: 62. In some of such embodiments, n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, in the antibody-drug conjugate of general formula Ab-$(L-U)_n$ or the pharmaceutically acceptable salt or the solvate thereof provided by the present disclosure, the antibody-drug conjugate has a structure

represented by formula IV-1, wherein Ab is an anti-CLDN18.2 antibody comprising a heavy chain and a light chain, wherein the heavy chain has an amino acid sequence set forth in SEQ ID NO: 63, and the light chain has an amino acid sequence set forth in SEQ ID NO: 64. In some of such embodiments, n is an integer selected from 1 to 10, 1 to 9, 1 to 8, 2 to 8, 2 to 7, 2 to 6, 2 to 5, 2 to 4, 2 to 3,3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8; preferably, n is an integer selected from 4 to 8 and 6 to 8; and more preferably, n is 4, 6, or 8.

In some embodiments, the present disclosure provides an antibody-drug conjugate or a pharmaceutically acceptable salt or a solvate thereof, wherein the antibody-drug conjugate is formed by linking a structure represented by formula IIIa below to an antibody or an antigen-binding fragment thereof,

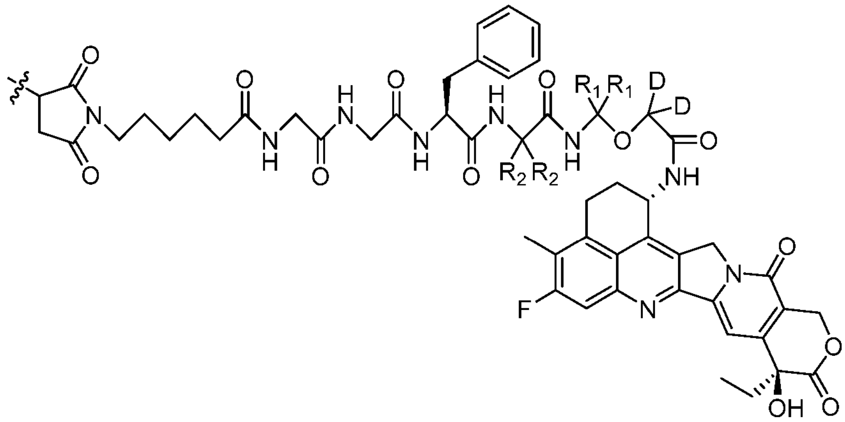

IIIa

wherein $R_1$ and $R_2$ are each independently selected from hydrogen (H) and deuterium (D), and the 3-position of -(succinimidyl-3-yl-N)- is linked to an antibody or an antigen-binding fragment thereof. In some specific embodiments, the antibody-drug conjugate is formed by linking a structure represented by formula IIIa to an antibody or an antigen-binding fragment thereof via a thioether bond. In some specific embodiments, the antibody or the antigen-binding fragment thereof is the anti-CLDN18.2 antibody or the antigen-binding fragment thereof of the present disclosure.

In some embodiments, the present disclosure provides an antibody-drug conjugate or a pharmaceutically acceptable salt or a solvate thereof, wherein the antibody-drug conjugate is formed by linking a structure represented by formula IIIa-1 below to an antibody or an antigen-binding fragment thereof,

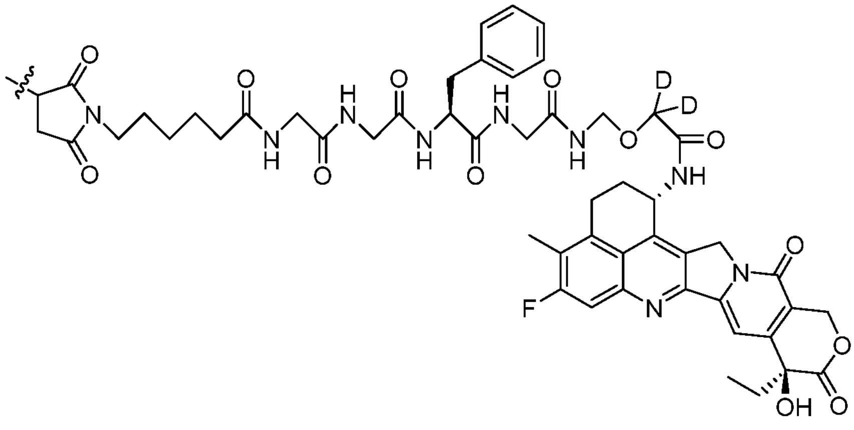

IIIa-1

wherein the 3-position of -(succinimidyl-3-yl-N)- in the structure represented by formula IIIa-1 is linked to the antibody or the antigen-binding fragment thereof. In some specific embodiments, the antibody-drug conjugate is formed by linking the structure represented by formula IIIa-1 to an antibody or an antigen-binding fragment thereof via a thioether bond. In some specific embodiments, the antibody or the antigen-binding fragment thereof is the anti-CLDN18.2 antibody or the antigen-binding fragment thereof of the present disclosure.

In some specific embodiments, the present disclosure provides an antibody-drug conjugate or a pharmaceutically acceptable salt or a solvate thereof, wherein the antibody-drug conjugate is formed by linking a structure represented by formula IIIa-1 below to an antibody or an antigen-binding fragment thereof,

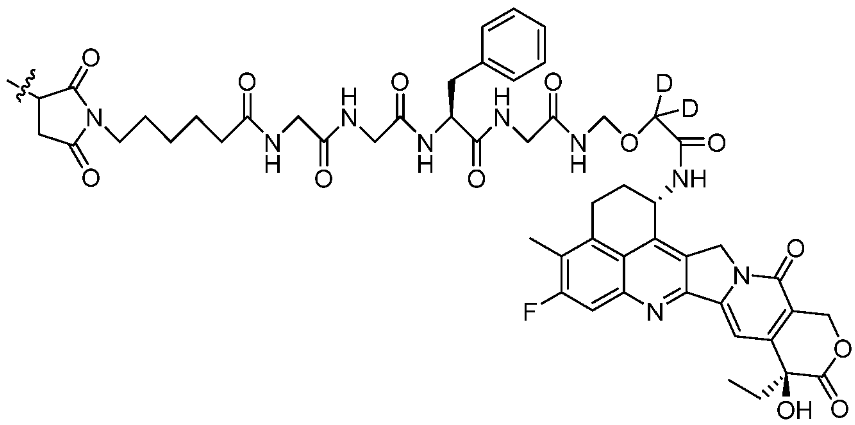

IIIa-1

wherein the 3-position of -(succinimidyl-3-yl-N)- in the structure represented by formula IIIa-1 is linked to an antibody or an antigen-binding fragment thereof; the antibody or the antigen-binding fragment thereof is an anti-CLDN18.2 antibody or an antigen-binding fragment thereof comprising HCDR1, HCDR2, HCDR3, LCDR1, LCDR2, and LCDR3, wherein the HCDR1, the HCDR2, the HCDR3, the LCDR1, the LCDR2, and the LCDR3 comprise amino acid sequences set forth in SEQ ID NOs: 3, 5, 7, 8, 9, and 10, respectively. Further, in some specific embodiments, the anti-CLDN18.2 antibody or the antigen-binding fragment thereof comprises a heavy chain variable region and a light chain variable region, wherein: (1) the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 12, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 17; or (2) the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 14, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 17. In some specific embodiments, the antibody-drug conjugate is formed by linking the structure represented by formula IIIa-1 to an antibody or an antigen-binding fragment thereof via a thioether bond.

In one aspect, the antibody-drug conjugate of general formula Ab-(L-U)$_n$ or the pharmaceutically acceptable salt or the solvate thereof provided by the present disclosure exhibits one of or a combination of several of the following properties:

(a) binding to CLDN18.2;
(b) blocking binding of CLDN18.2 to a ligand;
(c) showing endocytosis in cells expressing CLDN18.2;
(d) having killing activity against tumor cells expressing CLDN18.2;
(e) having a bystander effect;
(f) having ADCC activity;
(g) having CDC activity;
(h) not binding to CLDN18.1;
(i) not binding to cells not expressing CLDN18.2; and
(j) having no ADCC and/or CDC activity against cells expressing CLDN18.1.

In some embodiments, the antibody-drug conjugate or the pharmaceutically acceptable salt or the solvate thereof has properties (a) to (j). In some embodiments, the antibody-drug conjugate or the pharmaceutically acceptable salt or the solvate thereof has properties (a) and (c) to (j).

In some embodiments, the antibody-drug conjugate or the pharmaceutically acceptable salt or the solvate thereof binds to human CLDN18.2. In some embodiments, the antibody-drug conjugate or the pharmaceutically acceptable salt or the solvate thereof does not bind to human CLDN18.1.

In some embodiments, the antibody-drug conjugate or the pharmaceutically acceptable salt or the solvate thereof provided by the present disclosure shows strong endocytosis in cells with different CLDN18.2 expression amounts and can continuously accumulate the endocytosed ADC amount with increasing endocytosis time.

*Pharmaceutical Composition*

In one aspect, the present disclosure provides a pharmaceutical composition comprising the antibody-drug conjugate or the pharmaceutically acceptable salt or the solvate thereof of the present disclosure. In some embodiments, the present disclosure provides a pharmaceutical composition comprising the antibody-drug conjugate or the pharmaceutically acceptable salt or the solvate thereof according to the present disclosure and a pharmaceutically acceptable carrier. The pharmaceutically acceptable carrier includes, for example, excipients, diluents, encapsulating materials, fillers, buffering agents, or other agents.

The number of cytotoxic drugs conjugated to the antibody or the antigen-binding fragment thereof in the antibody-drug conjugate of the present disclosure may vary such that the antibody-drug conjugates or the pharmaceutically acceptable salts or the solvates thereof in the pharmaceutical composition provided by the present disclosure may be heterogeneous or homogeneous. By "heterogeneous" is meant that the pharmaceutical composition comprises antibody-drug conjugates with different numbers of cytotoxic drugs, for example, an antibody-drug conjugate in which 1 molecule of an antibody or an antigen-binding fragment thereof is conjugated to 0 (i.e., without cytotoxic drug), 1, 2, 3, 4, 5, 6, 7, 8, or more molecules of a cytotoxic drug. The antibody-drug conjugate in which 1 molecule of the antibody or the antigen-binding fragment thereof is conjugated to 0, 1, 2, 3, 4, 5, 6, 7, 8, or 9 molecules of the cytotoxic drug is referred to as D0, D1, D2, D3, D4, D5, D6, D7, D8, or D9, respectively.

By controlling the ratio of the antibody-drug conjugates with different numbers (n) of the cytotoxic drugs in the pharmaceutical composition described above, the pharmaceutical composition comprising ADCs having different drug antibody ratios (DARs) can be produced. It should be understood that the DAR is the mean number of cytotoxic drugs conjugated per molecule of an antibody or an antigen-binding fragment thereof in the pharmaceutical composition. For example, "DAR of about 4" refers to the following pharmaceutical composition: a heterogeneous mixture of ADCs in which each antibody or antigen-binding fragment thereof is conjugated with the same or different numbers of cytotoxic drugs (e.g., 0, 1, 2, 3, 4, 5, 6, 7, or 8 cytotoxic drugs per antibody or antigen-binding fragment thereof), but the mean number of cytotoxic drugs conjugated per molecule of an antibody or an antigen-binding fragment thereof in the composition is about 4. Similarly, "DAR of about 8" means that the mean number of cytotoxic drugs conjugated per molecule of an antibody or an antigen-binding fragment thereof in the pharmaceutical composition is about 8.

In some embodiments, the DAR of the pharmaceutical composition is 0 to 10, 0 to 9, 0 to 8, 1 to 10, 1 to 9, 1 to 8, 2 to 10, 2 to 9, 2 to 8, 3 to 8, 4 to 8, 5 to 8, 6 to 8, 7 to 8, 7.2 to 8, 7.4 to 8, 7.5 to 8, 7.6 to 8, 7.7 to 8, 7.8 to 8, 7.8 to 7.9, or 7.9 to 8. In some embodiments, the DAR of the pharmaceutical composition is about 1, about 2, about 3, about 4, about 5, about 6, about 7, about 7.1, about 7.2, about 7.3, about 7.4, about 7.5, about 7.6, about 7.7, about 7.8, about 7.9, or about 8.0.

In some embodiments, the DAR of the pharmaceutical composition is 7 to 8, 7.2 to 8, 7.4 to 8, 7.6 to 8, or 7.7 to 8. In some embodiments, the DAR of the pharmaceutical composition is about 7.1, about 7.2, about 7.4, about 7.6, about 7.7, or about 8.

In some embodiments, the antibody-drug conjugates or the pharmaceutically acceptable salts or the solvates thereof in the pharmaceutical composition are heterogeneous, and the distribution of antibody-drug conjugates or the pharmaceutically acceptable salts or the solvates thereof to which different numbers of cytotoxic drugs are linked is as follows: D0 to D7 account for 0% to 30%, D8 accounts for 60% to 100%, and D9 or above accounts for 0% to 15%; in some alternative embodiments, D0 to D3 account for 0% to 10%, D4 to D7 account for 0% to 30%, D8 accounts for 60% to 100%, and D9 or above accounts for 0% to 15%; in some alternative embodiments, D0 to D6 account for 0% to 30%, D7 to D8 account for 60% to 100%, and D9 or above accounts for 0% to 15%; in some alternative embodiments, D0 to D5 account for 0% to 30%, D6 to D8 account for 70% to 100%, and D9 or above accounts for 0% to 15%; in some alternative embodiments, D0 accounts for 0% to 10%, D1 accounts for 0% to 10%, D2 accounts for 0% to 10%, D3 accounts for 0% to 10%, D4 accounts for 0% to 20%, D5 accounts for 0% to 10%, D6 accounts for 0% to 20%, D7 accounts for 0% to 10%, D8 accounts for 60% to 100%, and D9 or above accounts for 0% to 10%; in some alternative embodiments, D0 accounts for 0% to 6%, D1 accounts for 0% to 6%, D2 accounts for 0% to 6%, D3 accounts for 0% to 6%, D4 accounts for 0% to 15%, D5 accounts for 0% to 6%, D6 accounts for 0% to 20%, D7 accounts for 0% to 6%, D8 accounts for 60% to 100%, and D9 or above accounts for 0% to 10%; in some alternative embodiments, D0 accounts for 0% to 6%, D1 to D3 account for 0% to 6%, D4 accounts for 0% to 15%, D5 to D7 account for 0% to 20%, D8 accounts for 60% to 100%, and D9 or above accounts for 0% to 10%; in some alternative embodiments, D0 accounts for 0% to 5%, D1 to D3 account for 0% to 5%, D4 accounts for 0% to 15%, D5 to D7 account for 0% to 20%, D8 accounts for 85% to 100%, and D9 or above accounts for 0% to 5%; the distribution ratios of the antibody-drug conjugates or the pharmaceutically acceptable salts or the solvates thereof are based on molar quantity.

*Use*

The present disclosure provides use of the antibody-drug conjugate or the pharmaceutically acceptable salt or the solvate thereof of the present disclosure, or the pharmaceutical composition of the present disclosure.

In one aspect, the present disclosure provides use of the antibody-drug conjugate or the pharmaceutically acceptable salt or the solvate thereof of the present disclosure for preparing a medicament for treating a tumor. In one aspect, the present disclosure provides use of the antibody-drug conjugate or the pharmaceutically acceptable salt or the solvate thereof of the present disclosure for treating a tumor. In one aspect, the present disclosure provides use of the pharmaceutical composition of the present disclosure for preparing a medicament for treating a tumor. In one aspect, the present disclosure provides use of the pharmaceutical composition of the present disclosure for treating a tumor.

In one aspect, the present disclosure provides use of the antibody-drug conjugate or the pharmaceutically acceptable salt or the solvate thereof of the present disclosure and a second or additional therapeutic agent for preparing a medicament for treating a tumor. In one aspect, the present disclosure provides use of the antibody-drug conjugate or the pharmaceutically acceptable salt or the solvate thereof of the present disclosure and a second or additional therapeutic agent for treating a tumor. In one aspect, the present disclosure provides use of the pharmaceutical composition of the present disclosure and a second or additional therapeutic agent for preparing a medicament for treating a tumor. In one aspect, the present disclosure provides use of the pharmaceutical composition of the present disclosure and a second or additional therapeutic agent for treating a tumor.

In one aspect, the present disclosure provides the antibody-drug conjugate or the pharmaceutically acceptable salt or the solvate thereof or the pharmaceutical composition described above for use in treating a tumor. In one aspect, the present disclosure provides the antibody-drug conjugate or the pharmaceutically acceptable salt or the solvate thereof, or the pharmaceutical composition described above, in combination with a second or additional therapeutic agent for treating a tumor.

In one aspect, the present disclosure provides a method for treating a tumor, comprising administering to a subject the antibody-drug conjugate or the pharmaceutically acceptable salt or the solvate thereof of the present disclosure. In one aspect, the present disclosure provides a method for treating a tumor, comprising administering to a subject in need a therapeutically effective amount of the antibody-drug conjugate or the pharmaceutically acceptable salt or the solvate thereof of the present disclosure. In one aspect, the present disclosure provides a method for treating a tumor, comprising administering to a subject the pharmaceutical composition of the present disclosure. In one aspect, the present disclosure provides a method for treating a tumor, comprising administering to a subject in need a therapeutically effective amount of the pharmaceutical composition of the present disclosure. In some embodiments, the method comprises contacting tumor cells with the antibody-drug conjugate or the pharmaceutically acceptable salt or the solvate thereof or the pharmaceutical composition, thereby killing or inhibiting growth of the tumor cells.

In one aspect, the present disclosure provides a method for treating a tumor, comprising administering to a subject the antibody-drug conjugate or the pharmaceutically acceptable salt or the solvate thereof of the present disclosure and a second or additional therapeutic agent. In one aspect, the present disclosure provides a method for treating a tumor, comprising administering to a subject in need a therapeutically effective amount of the antibody-drug conjugate or the pharmaceutically acceptable salt or the solvate thereof of the present disclosure and a second or additional therapeutic agent. In one aspect, the present disclosure provides a method for treating a tumor, comprising administering to a subject the pharmaceutical composition of the present disclosure and a second or additional therapeutic agent. In one aspect, the present disclosure provides a method for treating a tumor, comprising administering to a subject in need a therapeutically effective amount of the pharmaceutical composition of the present disclosure and a second or additional therapeutic agent. In some embodiments, the method comprises contacting tumor cells with the antibody-drug conjugate or the pharmaceutically acceptable salt or the solvate thereof or the pharmaceutical composition, and concurrently or sequentially contacting the tumor cells with a second or additional therapeutic agent, thereby killing or inhibiting growth of the tumor cells.

In some embodiments, administration of the antibody-drug conjugate or the pharmaceutically acceptable salt or the solvate thereof of the present disclosure to a subject can kill or inhibit growth of tumor cells. In some embodiments, administration of a therapeutically effective amount of the antibody-drug conjugate or the pharmaceutically acceptable salt or the solvate thereof of the present disclosure to a subject can kill or inhibit growth of tumor cells. In some embodiments, administration of the pharmaceutical composition of the present disclosure to a subject can kill or inhibit growth of tumor cells. In some embodiments, administration of a therapeutically effective amount of the pharmaceutical composition of the present disclosure to a subject can kill or inhibit growth of tumor cells.

In some embodiments, administration of the antibody-drug conjugate or the pharmaceutically acceptable salt or the solvate thereof of the present disclosure and a second or additional therapeutic agent to a subject can kill or inhibit growth of tumor cells. In some embodiments, administration of a therapeutically effective amount of the antibody-drug conjugate or the pharmaceutically acceptable salt or the solvate thereof of the present disclosure and a second or additional therapeutic agent to a subject can kill or inhibit growth of tumor cells. In some embodiments, administration of the pharmaceutical composition of the present disclosure and a second or additional therapeutic agent to a subject can kill or inhibit growth of tumor cells. In some embodiments, administration of a therapeutically effective amount of the pharmaceutical composition of the present disclosure and a second or additional therapeutic agent to a subject can kill or inhibit growth of tumor cells.

In the method or use described above, the pharmaceutical composition comprises the antibody-drug conjugate or the pharmaceutically acceptable salt or the solvate thereof of the present disclosure, and optionally, a pharmaceutically acceptable carrier.

In some embodiments, in the method or use described above, the tumor is a CLDN18.2-positive tumor. In some embodiments, administration of a therapeutically effective amount of the antibody-drug conjugate or the pharmaceutically acceptable salt or the solvate thereof of the present disclosure or the pharmaceutical composition of the present disclosure to a subject can kill or inhibit growth of tumor cells expressing CLDN18.2. Examples of the tumor include, but are not limited to, gastric cancer, esophageal cancer, gastrointestinal cancer (including colon cancer and rectal cancer), pancreatic cancer, liver cancer, lung cancer (including small cell lung cancer and non-small cell lung cancer), bronchial cancer, mesothelioma, kidney cancer, ovarian cancer (including Krukenberg tumor), breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and myeloma.

***Kit***

The present disclosure provides a kit comprising the anti-CLDN18.2 antibody-drug conjugate or the pharmaceutically acceptable salt or the solvate thereof of the present disclosure or the pharmaceutical composition of the present disclosure.

The present disclosure describes a kit comprising the anti-CLDN18.2 antibody-drug conjugate or the pharmaceutically acceptable salt or the solvate thereof or the pharmaceutical composition. The kit may be used to implement the use of the anti-CLDN18.2 antibody-drug conjugate or the pharmaceutically acceptable salt or the solvate thereof provided by the present disclosure or the pharmaceutical composition provided by the present disclosure, or other uses. In some embodiments, the kit may comprise the anti-CLDN18.2 antibody-drug conjugate or the pharmaceutically acceptable salt or the solvate thereof or the pharmaceutical composition. The kit may further comprise an instruction for use. The kit may also comprise other materials as required from a commercial and user standpoint, for example, other buffers, diluents, needles, syringes, and the like.

*Cytotoxic Drug and Linker-Payload*

In some aspects, the present disclosure provides a linker-payload having a structure represented by formula III below,

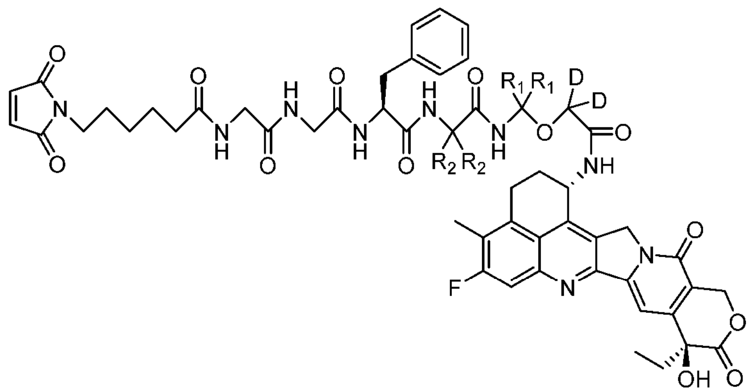 III,

wherein $R_1$ and $R_2$ are each independently selected from hydrogen (H) and deuterium (D).

In some specific embodiments, the present disclosure provides a linker-payload having a structure represented by formula III-1, III-2, III-3, or III-4 below,

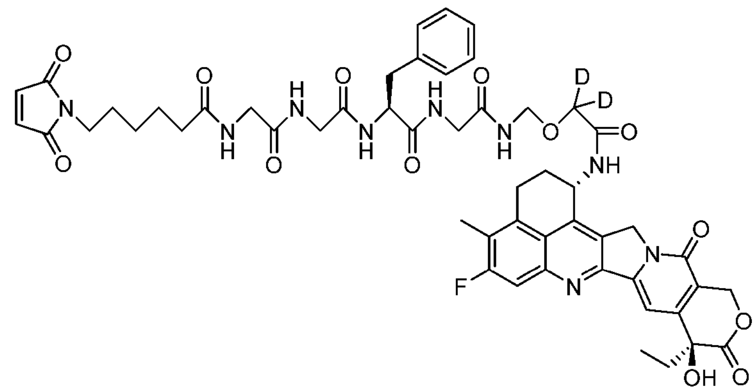

III-1,

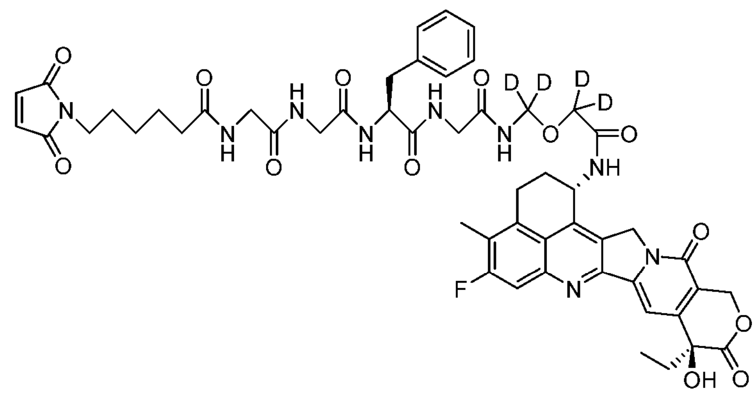

III-2,

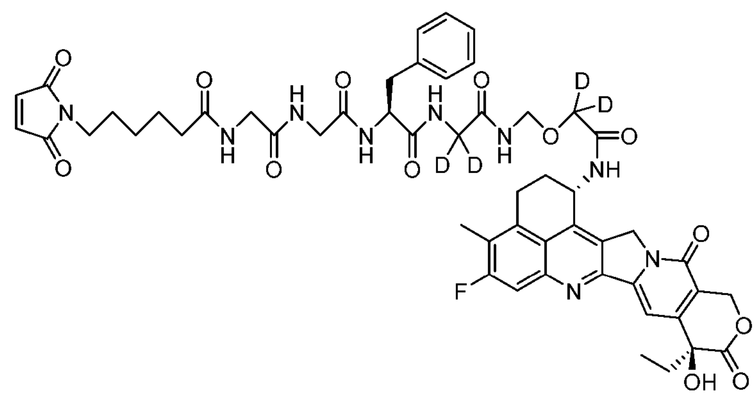

III-3,

or

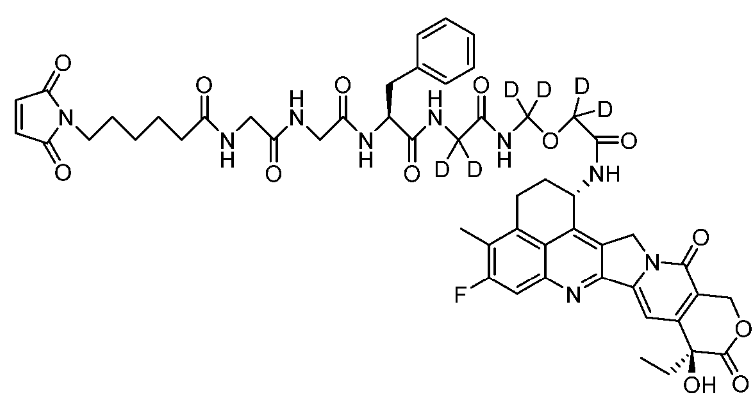

III-4.

The linker-payload is for use in obtaining an antibody-drug conjugate formed by linking an antibody or an antigen-binding fragment thereof to the linker-payload.

In some aspects, the present disclosure provides a compound having a structure represented by formula I below,

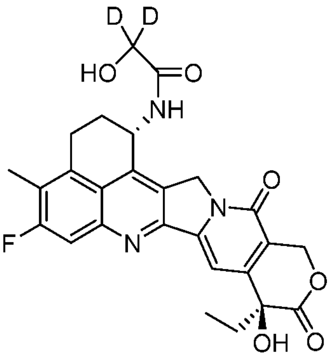
I.

The antibody-drug conjugate or the pharmaceutically acceptable salt or the solvate thereof provided by the present disclosure achieves excellent anti-tumor effect and/or safety. In some embodiments, the provided anti-CLDN18.2 antibody-drug conjugate exhibits good killing activity against various tumor cells expressing CLDN18.2. In some embodiments, the provided anti-CLDN18.2 antibody-drug conjugate exhibits no killing activity against cells expressing CLDN18.1. In some embodiments, the provided anti-CLDN18.2 antibody-drug conjugate effectively binds to cells expressing CLDN18.2 and efficiently exerts endocytosis in cells expressing CLDN18.2. In some embodiments, the provided anti-CLDN18.2 antibody-drug conjugate has good *in vivo* anti-tumor activity. In some embodiments, the provided anti-CLDN18.2 antibody-drug conjugate has excellent safety. In some embodiments, the provided anti-CLDN18.2 antibody-drug conjugate is less prone to aggregation.

The antibody-drug conjugate or the pharmaceutically acceptable salt or the solvate thereof provided by the present disclosure has excellent pharmacokinetic properties. The excellent pharmacokinetic properties may lead to relatively low toxicity, relatively high safety and/or tolerance, relatively high therapeutic efficacy, and relatively wide therapeutic window.

## ***Preparation Method***

The present disclosure provides a method for preparing the antibody-drug conjugate or the pharmaceutically acceptable salt or the solvate thereof of the present disclosure, comprising treating an anti-CLDN18.2 antibody or an antigen-binding fragment thereof under reducing conditions, and then reacting the anti-CLDN18.2 antibody or the antigen-binding fragment thereof with a linker-payload selected from a structure represented by formula III, wherein $R_1$ and $R_2$ are each independently selected from hydrogen or deuterium. In some embodiments, $R_1$ and $R_2$ are hydrogen.

In some embodiments, the method comprises treating an anti-CLDN18.2 antibody or an antigen-binding fragment thereof under reducing conditions, and then reacting the anti-CLDN18.2 antibody or the antigen-binding fragment thereof with a linker-payload having a structure represented by formula III-1. In some embodiments, the method comprises reducing an anti-CLDN18.2 antibody or an antigen-binding fragment thereof in the presence of TCEP, and then mixing the anti-CLDN18.2 antibody or the antigen-binding fragment thereof with a linker-payload having a structure represented by formula III-1 to perform a conjugation reaction, thereby obtaining the antibody-drug conjugate or the pharmaceutically acceptable salt or the solvate thereof.

## BRIEF DESCRIPTION OF THE DRAWINGS

FIGs. 1A-1C show the binding activity of hz28G3-1.1-DDDXd (with DAR of 7.4), hz28G3-1.3-DDDXd (with DAR of 7.6), hz28G3-1.1, and hz28G3-1.3 to CLDN18.2-overexpressing NIH-3T3, BxPC-3, and NCI-N87 cells;

FIGs. 2A-2D show the endocytosis of hz28G3-1.1-DDDXd (with DAR of 7.4), hz28G3-1.3-DDDXd (with DAR of 7.6), hz28G3-1.1, and hz28G3-1.3 in CLDN18.2-overexpressing NIH-3T3, BxPC-3, and NCI-N87 cells and a CLDN18.2-positive natural cell line NUGC-4;

FIGs. 3A-3C show the killing of CLDN18.2-overexpressing NIH-3T3, BxPC-3, and NCI-N87 cells by hz28G3-1.1-DDDXd (with DAR of 7.4) and hz28G3-1.3-DDDXd (with DAR of 7.6);

FIG. 4 shows the CDC activity of hz28G3-1.1-DDDXd (with DAR of 7.4) and hz28G3-1.3-DDDXd (with DAR of 7.6) on CLDN18.2-overexpressing NIH-3T3 cells;

FIG. 5 shows the non-specific killing of NIH-3T3-CLDN18.1 cells by hz28G3-1.1-DDDXd (with DAR of 7.4) and hz28G3-1.3-DDDXd (with DAR of 7.6);

FIG. 6 shows the bystander effect of hz28G3-1.1-DDDXd (with DAR of 7.4) and hz28G3-1.3-DDDXd (with DAR of 7.6), with CLDN18.2-overexpressing BxPC-3 as positive cells and U20S-Luciferase as negative cells.

**Explanation and definitions**

Unless otherwise stated, the following terms used in the present disclosure shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted, and oxo is not possible in an aromatic group.

When any variable (e.g., R) occurs once or more times in the constitution or structure of a compound, the definition of the variable in each case is independent. Therefore, for example, if a group is substituted with 2 R, the definition of each R is independent.

As used herein, a structure of "-(succinimidyl-3-yl-N)-" is as the formula below:

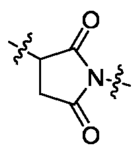.

Unless otherwise stated, the absolute configuration of a stereogenic center is represented by a wedged solid bond (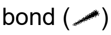) and a wedged dashed bond (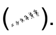).

Unless otherwise specified, when a certain group has a connectable site, the chemical bond connecting the site to other groups may be represented by a wavy line (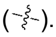). For example,

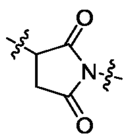

indicates that the nitrogen atom at the 1-position and the carbon atom at the 3-position in -(succinimidyl-3-yl-N)- are connected to other groups. The "derivative": a compound formed by substituting atoms or atom groups in the molecule of the parent compound with other atoms or atom groups is referred to as a derivative of the parent compound.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The compounds of the present disclosure can be asymmetrical, for example, has one or more stereoisomers. The compounds of the present disclosure may also exist as specific geometric isomers. Unless otherwise stated, all geometric isomers and stereoisomers are included, such as *cis* and *trans* isomers, levorotatory and dextrorotatory forms, enantiomers, diastereoisomers, (D)-isomers, and (L)-isomers. Racemic mixtures and other mixtures, such as enantiomer- or diastereoisomer-enriched mixtures, of the isomers described above are also encompassed within the scope of the present disclosure. The compounds containing asymmetric carbon atoms of the present disclosure can be isolated in an optically active pure form or in a racemic form. The optically active pure form may be separated from a racemic mixture or may be synthesized using a chiral raw material or a chiral reagent.

Unless otherwise stated, the term *"cis-trans* isomer" or "geometric isomer" results from the inability of a double bond or a single bond of a ring carbon atom to rotate freely. Unless otherwise stated, the term "enantiomer" refers to stereoisomers that are mirror images of each other. Unless otherwise stated, the term "diastereoisomer" refers to stereoisomers whose molecules have two or more chiral centers and are not mirror images of each other.

Unless otherwise stated, those skilled in the art shall understand that D in the structural formula herein represents deuterium.

As used herein, the term "bystander effect" refers to an effect in which a cytotoxic drug conjugated to an antibody or an antigen-binding fragment thereof via a cleavable or non-cleavable linker has the ability to diffuse and penetrate through the cell membrane after release from the antibody or the antigen-binding fragment thereof and thereby result in killing of adjacent cells. The ability to diffuse and penetrate through the cell membrane is related to the hydrophobicity of the cytotoxic drug or the combination of the cytotoxic drug and the linker. Such cytotoxic drugs may be, for example, an exatecan derivative or MMAE. The bystander effect may be desirable particularly in tumors with heterogeneous target expression and solid tumors where antibody penetration may be limited.

The term "treating" or "treatment" means administering the compound or pharmaceutical composition described herein to prevent, ameliorate, or eliminate a disease or one or more symptoms associated with the disease, including but not limited to: (i) preventing the occurrence of a disease or disease state in a mammal, particularly when such a mammal is predisposed to the disease state but has not yet been diagnosed with it; (ii) inhibiting a disease or disease state, i.e., arresting its progression; (iii) alleviating a disease or disease state, i.e., causing its regression; and (iv) reducing any direct or indirect pathological consequences of a disease or disease state.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying onset of one or more symptoms of the specific disease, condition, or disorder described herein. The amount of the compound or the pharmaceutical composition of the present disclosure that constitutes a "therapeutically effective amount" may vary depending on factors such as the compound or the pharmaceutical composition and its ability to elicit a desired response in an individual, the disease state and its severity, the mode of administration, and the age, sex, and weight of the mammal to be treated.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of a compound (e.g., the antibody-drug conjugate of the present disclosure) that exhibits safety and efficacy when used in mammals and possess the desired biological activity. For example, such salts may be a metal salt, an ammonium salt, a salt formed with an organic base, a salt formed with an inorganic acid, a salt formed with an organic acid, a salt formed with a basic or acidic amino acid, or the like.

The term "solvate" refers to a substance formed by association of a compound with a solvent molecule.

The term "antibody" is used in its broadest sense and encompasses a variety of antibody structures including, but not limited to, monoclonal antibodies, polyclonal antibodies, and multispecific antibodies (e.g., bispecific antibodies or trispecific antibodies), so long as they exhibit the desired antigen-binding activity.

The "antigen-binding fragment" of an antibody refers to one or more fragments of the antibody that retain the functionality of specifically binding to an antigen (e.g., CLDN18.2 protein). It has been demonstrated that the antigen binding functionality of an antibody can be implemented by fragments of a full-length antibody. Examples encompassed within the term "antigen-binding fragment" of an antibody include: (i) a Fab fragment: a monovalent fragment consisting of VL, VH, CL, and CH1 domains; (ii) a F(ab')2 fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); and (vi) a nanobody, an antibody comprising a single variable domain and two constant domains. Furthermore, although the two domains VL and VH of the Fv fragment are encoded by different genes, the VH and VL domains can be joined via a linker by recombinant methods into a single protein chain in which VL and VH are paired to form a monovalent molecule referred to as a single-chain Fv (scFv) (see, Bird et al., Science. 242:423-426 (1988); Huston et al., Proc. Natl. Acad. Sci. 85:5879-5883 (1988)). These single-chain antibodies are also encompassed within the term "antigen-binding fragment". These antibody fragments can be obtained using conventional techniques known to those skilled in the art, and the fragments can be subjected to functional screening using the same method as full-length antibodies.

The anti-CLDN18.2 antibody or the antigen-binding fragment thereof of the present disclosure may be of an IgG1, IgG2, IgG3, or IgG4 isotype. The term "isotype" refers to the class of antibodies encoded by the heavy chain constant region gene. In some embodiments, the anti-CLDN18.2 antibody or the antigen-binding fragment thereof of the present disclosure is of an IgG1 isotype. The anti-CLDN18.2 antibody or the antigen-binding fragment thereof of the present disclosure may be derived from any species, including but not limited to mouse, rat, rabbit, non-human primate (e.g., chimpanzee, cynomolgus monkey, spider monkey, or macaque), llama, and human. The anti-CLDN18.2 antibody or the antigen-binding fragment thereof of the present disclosure may be a murine antibody, a chimeric antibody, a humanized antibody, or a human antibody.

The term "mouse antibody" or "murine antibody" refers to an antibody in which the framework regions and CDRs

in the variable region are derived from mouse germline immunoglobulin sequences. Furthermore, if the antibody comprises a constant region, the constant region is also derived from mouse germline immunoglobulin sequences. The murine antibody of the present disclosure may comprise amino acid residues not encoded by mouse germline immunoglobulin sequences (e.g., mutations introduced by *in vitro* random mutation or point mutation or by *in vivo* somatic mutation), but "mouse antibody" or "murine antibody" does not include antibodies in which CDR sequences derived from other mammalian species are inserted into mouse framework sequences.

The "chimeric antibody" is an antibody obtained by fusing a variable region of a murine antibody with a constant region of a human antibody and can reduce the immune response induced by the murine antibody. The establishment of a chimeric antibody comprises establishing a hybridoma secreting a murine specific monoclonal antibody first, cloning a variable region gene from the hybridoma cell, then cloning a constant region gene of a human antibody as required, linking the murine variable region gene and the human constant region gene into a chimeric gene and then inserting the chimeric gene into an expression vector, and finally expressing a chimeric antibody in a eukaryotic system or a prokaryotic system.

The "humanized antibody" refers to an antibody comprising complementarity determining regions (CDRs) derived from a non-human antibody and framework and constant regions derived from a human antibody. For example, in the antibody-drug conjugate provided herein, the humanized antibody that binds to CLDN18.2 may comprise CDRs derived from one or more murine antibodies as well as human framework and constant regions. Thus, in some embodiments, in the antibody-drug conjugate provided herein, the humanized antibody binds to the same epitope on CLDN18.2 as the murine antibody from which the CDRs of the humanized antibody are derived. Exemplary humanized antibodies are provided herein. Additional humanized antibodies that bind to CLDN18.2 or variants thereof comprising the heavy and light chain CDRs provided herein may be generated using any human framework sequences and are also included in the present disclosure. In some embodiments, framework sequences suitable for use in the present disclosure include those similar in structure to the framework sequences provided herein. Additional modifications may be made in the framework regions to alter the properties of the antibodies provided herein. Such additional framework region modifications may include chemical modifications, point mutations for reducing immunogenicity or removing T cell epitopes, or back mutations to residues in original germline sequences. In some embodiments, such additional modifications include those corresponding to the mutations exemplified herein, including back mutations to germline sequences. For example, in some embodiments, one or more amino acids in the human VH and/or VL framework regions of the humanized antibodies provided herein are back-mutated to the corresponding amino acids in the parent murine antibodies. In the present disclosure, humanized antibodies are also denoted as "hz".

The term "CDR" (complementarity determining region) is also referred to as "hypervariable region". Natural four-chain antibodies typically comprise six CDRs, three in the heavy chain variable region and three in the light chain variable region.

The term "variable region" refers to a domain of about 100 to 110 or more amino acids defined by the N-terminal domain of the light or heavy chain of an antibody and primarily responsible for antigen recognition. The terms light chain variable region (VL) and heavy chain variable region (VH) refer to these light and heavy chain domains, respectively.

As used herein, the term "$EC_{50}$" refers to the effective concentration required to elicit 50% of the maximum response by an antibody or an antigen-binding fragment thereof, or ADC; the term "$IC_{50}$" refers to the inhibitory concentration required to elicit 50% of the maximum response by the antibody or the antigen-binding fragment thereof, or ADC. Both $EC_{50}$ and $IC_{50}$ may be measured by ELISA or FACS assay or any other method known in the art.

The "$K_D$" refers to the equilibrium dissociation constant, which is derived from the ratio of $K_d$ to $K_a$ (i.e., $K_d/K_a$) and expressed in molar concentration (M). The $K_D$ value of an antibody may be determined using methods well established in the art. A preferred method for determining the $K_D$ of an antibody is surface plasmon resonance, preferably using a biosensor system such as BIACORE® surface plasmon resonance system for analysis.

The term "identity" is also referred to as homology. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

As used herein, the terms "X" and "Xaa" are equivalent and refer to unspecified amino acids, and the scopes encompassed thereby are specified by definitions in relevant expressions. To distinguish multiple "X"s occurring in the same amino acid sequence, consecutive numbers are assigned to the "X"s that appear in sequence (i.e., written as $X_n$), and the scopes encompassed thereby are separately defined.

The term "subject" includes any human or non-human animal. The term "non-human animal" includes all vertebrates, e.g., mammals and non-mammals, such as non-human primates, sheep, goats, dogs, cats, horses, cows, chickens, amphibians, reptiles, and the like. Preferably, the subject according to the present disclosure is a human. The

terms "patient" and "subject" are used interchangeably unless otherwise indicated. "Subjects in need" include those with a disease or condition, those at risk of developing the disease or condition, and those who may have the disease or condition and whose purpose is to prevent, delay, or alleviate the disease or condition.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to $\pm$ 5%, for example, fluctuating within a particular numerical range given $\pm$ 2%, $\pm$ 1%, or $\pm$ 0.5%. Where a particular value is given in the scope of the present disclosure, unless otherwise stated, "about" should be considered to mean being within an acceptable error range for that particular value. In this context, unless otherwise stated, the values of the parameters or conditions in a step are all modified by "about" by default.

The terms "comprise", "comprises", "comprising" and equivalents thereof (e.g., contain, contains, containing, include, includes, and including) are to be construed as "including, but not limited to", meaning that additional unspecified elements, components, and steps may be included in addition to the enumerated elements, components, and steps.

Herein, unless otherwise specified clearly in the context, singular terms encompass plural referents, and *vice versa.* Similarly, unless otherwise specified clearly in the context, the word "or" is intended to include "and".

## DETAILED DESCRIPTION

The present disclosure further provides the following specific embodiments, which, however, are not intended to limit the scope of the present disclosure:

Embodiment 1. An antibody-drug conjugate of general formula Ab-$(L\text{-}U)_n$ or a pharmaceutically acceptable salt or a solvate thereof, wherein Ab is an antibody or an antigen-binding fragment thereof, L is a linker, U is a cytotoxic drug, and n is an integer selected from 1 to 10, wherein Ab is an anti-CLDN18.2 antibody or an antigen-binding fragment thereof comprising HCDR1, HCDR2, HCDR3, LCDR1, LCDR2, and LCDR3, wherein

(1) the HCDR1 comprises an HCDR1 amino acid sequence in SEQ ID NO: 11, the HCDR2 comprises an HCDR2 amino acid sequence in SEQ ID NO: 11, the HCDR3 comprises an HCDR3 amino acid sequence in SEQ ID NO: 11, the LCDR1 comprises an LCDR1 amino acid sequence in SEQ ID NO: 16, the LCDR2 comprises an LCDR2 amino acid sequence in SEQ ID NO: 16, and the LCDR3 comprises an LCDR3 amino acid sequence in SEQ ID NO: 16;
(2) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(3) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO: 13, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(4) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in SEQ ID NO: 14, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(5) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;
(6) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;
(7) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO:

13, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(8) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in SEQ ID NO: 14, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(9) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(10) the HCDR1 comprises an HCDR1 amino acid sequence in SEQ ID NO: 29, the HCDR2 comprises an HCDR2 amino acid sequence in SEQ ID NO: 29, the HCDR3 comprises an HCDR3 amino acid sequence in SEQ ID NO: 29, the LCDR1 comprises an LCDR1 amino acid sequence in SEQ ID NO: 34, the LCDR2 comprises an LCDR2 amino acid sequence in SEQ ID NO: 34, and the LCDR3 comprises an LCDR3 amino acid sequence in SEQ ID NO: 34;

(11) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(12) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(13) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(14) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(15) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;

(16) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;

(17) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;

(18) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;

(19) the HCDR1 comprises an HCDR1 amino acid sequence in SEQ ID NO: 43, the HCDR2 comprises an HCDR2 amino acid sequence in SEQ ID NO: 43, the HCDR3 comprises an HCDR3 amino acid sequence in SEQ ID NO: 43, the LCDR1 comprises an LCDR1 amino acid sequence in SEQ ID NO: 44, the LCDR2 comprises an LCDR2 amino acid sequence in SEQ ID NO: 44, and the LCDR3 comprises an LCDR3 amino acid sequence in SEQ ID NO: 44;
(20) the HCDR1 comprises an HCDR1 amino acid sequence in SEQ ID NO: 51, the HCDR2 comprises an HCDR2 amino acid sequence in SEQ ID NO: 51, the HCDR3 comprises an HCDR3 amino acid sequence in SEQ ID NO: 51, the LCDR1 comprises an LCDR1 amino acid sequence in SEQ ID NO: 52, the LCDR2 comprises an LCDR2 amino acid sequence in SEQ ID NO: 52, and the LCDR3 comprises an LCDR3 amino acid sequence in SEQ ID NO: 52; or
(21) the HCDR1 comprises an HCDR1 amino acid sequence in SEQ ID NO: 59, the HCDR2 comprises an HCDR2 amino acid sequence in SEQ ID NO: 59, the HCDR3 comprises an HCDR3 amino acid sequence in SEQ ID NO: 59, the LCDR1 comprises an LCDR1 amino acid sequence in SEQ ID NO: 60, the LCDR2 comprises an LCDR2 amino acid sequence in SEQ ID NO: 60, and the LCDR3 comprises an LCDR3 amino acid sequence in SEQ ID NO: 60.

Embodiment 2. The antibody-drug conjugate or the pharmaceutically acceptable salt or the solvate thereof according to embodiment 1, wherein

(1) the HCDR1, the HCDR2, the HCDR3, the LCDR1, the LCDR2, and the LCDR3 comprise amino acid sequences set forth in SEQ ID NOs: 3, 4, 7, 8, 9, and 10, respectively;
(2) the HCDR1, the HCDR2, the HCDR3, the LCDR1, the LCDR2, and the LCDR3 comprise amino acid sequences set forth in SEQ ID NOs: 3, 5, 7, 8, 9, and 10, respectively;
(3) the HCDR1, the HCDR2, the HCDR3, the LCDR1, the LCDR2, and the LCDR3 comprise amino acid sequences set forth in SEQ ID NOs: 3, 6, 7, 8, 9, and 10, respectively;
(4) the HCDR1, the HCDR2, the HCDR3, the LCDR1, the LCDR2, and the LCDR3 comprise amino acid sequences set forth in SEQ ID NOs: 19, 20, 24, 25, 26, and 27, respectively;
(5) the HCDR1, the HCDR2, the HCDR3, the LCDR1, the LCDR2, and the LCDR3 comprise amino acid sequences set forth in SEQ ID NOs: 19, 21, 24, 25, 26, and 27, respectively;
(6) the HCDR1, the HCDR2, the HCDR3, the LCDR1, the LCDR2, and the LCDR3 comprise amino acid sequences set forth in SEQ ID NOs: 19, 22, 24, 25, 26, and 27, respectively;
(7) the HCDR1, the HCDR2, the HCDR3, the LCDR1, the LCDR2, and the LCDR3 comprise amino acid sequences set forth in SEQ ID NOs: 19, 23, 24, 25, 26, and 27, respectively;
(8) the HCDR1, the HCDR2, the HCDR3, the LCDR1, the LCDR2, and the LCDR3 comprise amino acid sequences set forth in SEQ ID NOs: 19, 20, 24, 25, 26, and 28, respectively;
(9) the HCDR1, the HCDR2, the HCDR3, the LCDR1, the LCDR2, and the LCDR3 comprise amino acid sequences set forth in SEQ ID NOs: 19, 21, 24, 25, 26, and 28, respectively;
(10) the HCDR1, the HCDR2, the HCDR3, the LCDR1, the LCDR2, and the LCDR3 comprise amino acid sequences set forth in SEQ ID NOs: 19, 22, 24, 25, 26, and 28, respectively;
(11) the HCDR1, the HCDR2, the HCDR3, the LCDR1, the LCDR2, and the LCDR3 comprise amino acid sequences set forth in SEQ ID NOs: 19, 23, 24, 25, 26, and 28, respectively;
(12) the HCDR1, the HCDR2, the HCDR3, the LCDR1, the LCDR2, and the LCDR3 comprise amino acid sequences set forth in SEQ ID NOs: 37, 38, 39, 40, 41, and 42, respectively;
(13) the HCDR1, the HCDR2, the HCDR3, the LCDR1, the LCDR2, and the LCDR3 comprise amino acid sequences set forth in SEQ ID NOs: 45, 46, 47, 48, 49, and 50, respectively; or
(14) the HCDR1, the HCDR2, the HCDR3, the LCDR1, the LCDR2, and the LCDR3 comprise amino acid sequences set forth in SEQ ID NOs: 53, 54, 55, 56, 57, and 58, respectively.

Embodiment 3. The antibody-drug conjugate or the pharmaceutically acceptable salt or the solvate thereof according to embodiment 1 or 2, wherein the anti-CLDN18.2 antibody or the antigen-binding fragment thereof comprises a heavy chain variable region and a light chain variable region, wherein

(1) the heavy chain variable region and the light chain variable region comprise amino acid sequences having at least 80% identity to amino acid sequences set forth in SEQ ID NOs: 11 and 16, respectively;
(2) the heavy chain variable region and the light chain variable region comprise amino acid sequences having at least 80% identity to amino acid sequences set forth in SEQ ID NOs: 12 and 17, respectively;
(3) the heavy chain variable region and the light chain variable region comprise amino acid sequences having at least 80% identity to amino acid sequences set forth in SEQ ID NOs: 13 and 17, respectively;

(4) the heavy chain variable region and the light chain variable region comprise amino acid sequences having at least 80% identity to amino acid sequences set forth in SEQ ID NOs: 14 and 17, respectively;
(5) the heavy chain variable region and the light chain variable region comprise amino acid sequences having at least 80% identity to amino acid sequences set forth in SEQ ID NOs: 15 and 17, respectively;
(6) the heavy chain variable region and the light chain variable region comprise amino acid sequences having at least 80% identity to amino acid sequences set forth in SEQ ID NOs: 12 and 18, respectively;
(7) the heavy chain variable region and the light chain variable region comprise amino acid sequences having at least 80% identity to amino acid sequences set forth in SEQ ID NOs: 13 and 18, respectively;
(8) the heavy chain variable region and the light chain variable region comprise amino acid sequences having at least 80% identity to amino acid sequences set forth in SEQ ID NOs: 14 and 18, respectively;
(9) the heavy chain variable region and the light chain variable region comprise amino acid sequences having at least 80% identity to amino acid sequences set forth in SEQ ID NOs: 15 and 18, respectively;
(10) the heavy chain variable region and the light chain variable region comprise amino acid sequences having at least 80% identity to amino acid sequences set forth in SEQ ID NOs: 29 and 34, respectively;
(11) the heavy chain variable region and the light chain variable region comprise amino acid sequences having at least 80% identity to amino acid sequences set forth in SEQ ID NOs: 30 and 35, respectively;
(12) the heavy chain variable region and the light chain variable region comprise amino acid sequences having at least 80% identity to amino acid sequences set forth in SEQ ID NOs: 31 and 35, respectively;
(13) the heavy chain variable region and the light chain variable region comprise amino acid sequences having at least 80% identity to amino acid sequences set forth in SEQ ID NOs: 32 and 35, respectively;
(14) the heavy chain variable region and the light chain variable region comprise amino acid sequences having at least 80% identity to amino acid sequences set forth in SEQ ID NOs: 33 and 35, respectively;
(15) the heavy chain variable region and the light chain variable region comprise amino acid sequences having at least 80% identity to amino acid sequences set forth in SEQ ID NOs: 30 and 36, respectively;
(16) the heavy chain variable region and the light chain variable region comprise amino acid sequences having at least 80% identity to amino acid sequences set forth in SEQ ID NOs: 31 and 36, respectively;
(17) the heavy chain variable region and the light chain variable region comprise amino acid sequences having at least 80% identity to amino acid sequences set forth in SEQ ID NOs: 32 and 36, respectively;
(18) the heavy chain variable region and the light chain variable region comprise amino acid sequences having at least 80% identity to amino acid sequences set forth in SEQ ID NOs: 33 and 36, respectively;
(19) the heavy chain variable region and the light chain variable region comprise amino acid sequences having at least 80% identity to amino acid sequences set forth in SEQ ID NOs: 43 and 44, respectively;
(20) the heavy chain variable region and the light chain variable region comprise amino acid sequences having at least 80% identity to amino acid sequences set forth in SEQ ID NOs: 51 and 52, respectively; or
(21) the heavy chain variable region and the light chain variable region comprise amino acid sequences having at least 80% identity to amino acid sequences set forth in SEQ ID NOs: 59 and 60, respectively.

Embodiment 4. The antibody-drug conjugate or the pharmaceutically acceptable salt or the solvate thereof according to any one of embodiments 1 to 3, wherein the anti-CLDN18.2 antibody or the antigen-binding fragment thereof is of an IgG1, IgG2, or IgG4 isotype.
Embodiment 5. The antibody-drug conjugate or the pharmaceutically acceptable salt or the solvate thereof according to any one of embodiments 1-4, wherein the anti-CLDN18.2 antibody further comprises a heavy chain constant region and a light chain constant region, wherein the heavy chain constant region comprises an amino acid sequence set forth in SEQ ID NO: 1 or an amino acid sequence having 1, 2, 3, 4, or 5 amino acid substitutions, deletions, and additions compared to the amino acid sequence set forth in SEQ ID NO: 1, and the light chain constant region comprises an amino acid sequence set forth in SEQ ID NO: 2 or an amino acid sequence having 1, 2, 3, 4, or 5 amino acid substitutions, deletions, and additions compared to the amino acid sequence set forth in SEQ ID NO: 2.
Embodiment 6. The antibody-drug conjugate or the pharmaceutically acceptable salt or the solvate thereof according to any one of embodiments 1 to 5, wherein the anti-CLDN18.2 antibody comprises a heavy chain and a light chain, wherein

(1) the heavy chain comprises an amino acid sequence having at least 80% identity to an amino acid sequence set forth in SEQ ID NO: 61 and the light chain comprises an amino acid sequence having at least 80% identity to an amino acid sequence set forth in SEQ ID NO: 62; or
(2) the heavy chain comprises an amino acid sequence having at least 80% identity to an amino acid sequence set forth in SEQ ID NO: 63 and the light chain comprises an amino acid sequence having at least 80% identity to an amino acid sequence set forth in SEQ ID NO: 64.

Embodiment 7. The antibody-drug conjugate or the pharmaceutically acceptable salt or the solvate thereof according to any one of embodiments 1 to 6, wherein U is a camptothecin topoisomerase I inhibitor and L and/or U has a deuterated modification.

Embodiment 8. The antibody-drug conjugate or the pharmaceutically acceptable salt or the solvate thereof according to embodiment 7, wherein U is SN-38, an SN-38 derivative, exatecan, or an exatecan derivative, preferably an exatecan derivative.

Embodiment 9. The antibody-drug conjugate or the pharmaceutically acceptable salt or the solvate thereof according to embodiment 8, wherein -U is a structure represented by formula Ia below:

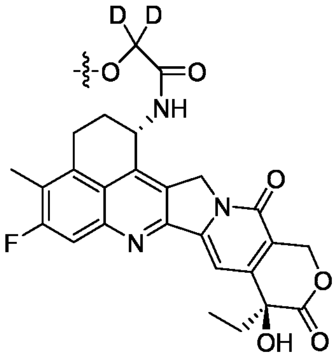

Ia.

Embodiment 10. The antibody-drug conjugate or the pharmaceutically acceptable salt or the solvate thereof according to any one of embodiments 7 to 9, wherein -L- is a structure represented by formula IIa below:

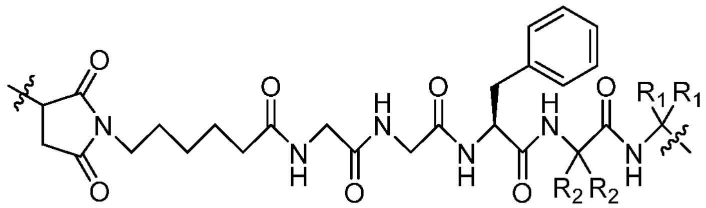

IIa,

wherein $R_1$ and $R_2$ are each independently selected from hydrogen and deuterium;
and the 3-position of -(succinimidyl-3-yl-N)- in the structure shown is linked to Ab, and the methylene at the other end is linked to U.

Embodiment 11. The antibody-drug conjugate or the pharmaceutically acceptable salt or the solvate thereof according to embodiment 7, wherein -L-U is a structure represented by formula IIIa below:

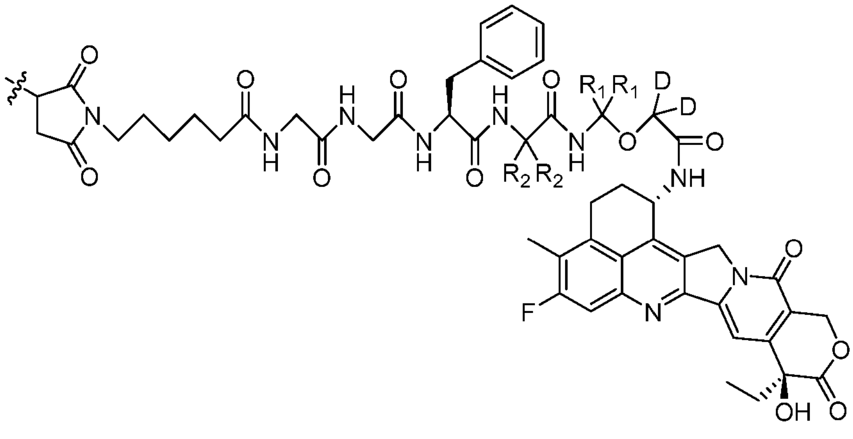

IIIa,

wherein $R_1$ and $R_2$ are each independently selected from hydrogen and deuterium.

Embodiment 12. The antibody-drug conjugate or the pharmaceutically acceptable salt or the solvate thereof according to embodiment 11, wherein -L-U is a structure represented by formula IIIa-1, IIIa-2, IIIa-3, or IIIa-4 below:

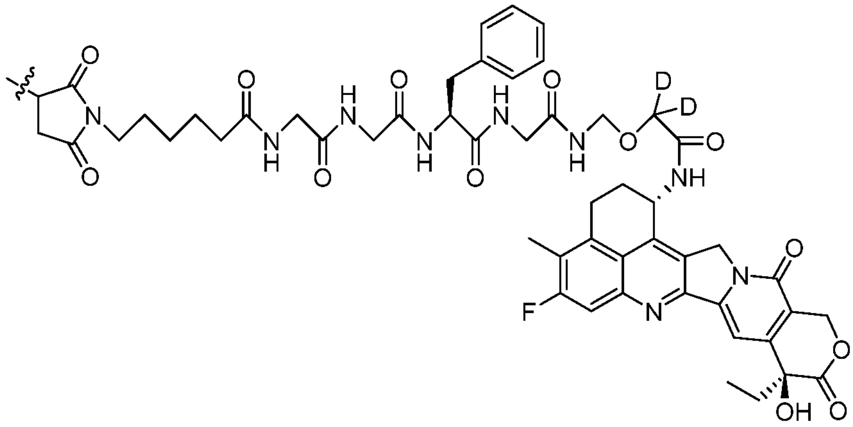

IIIa-1,

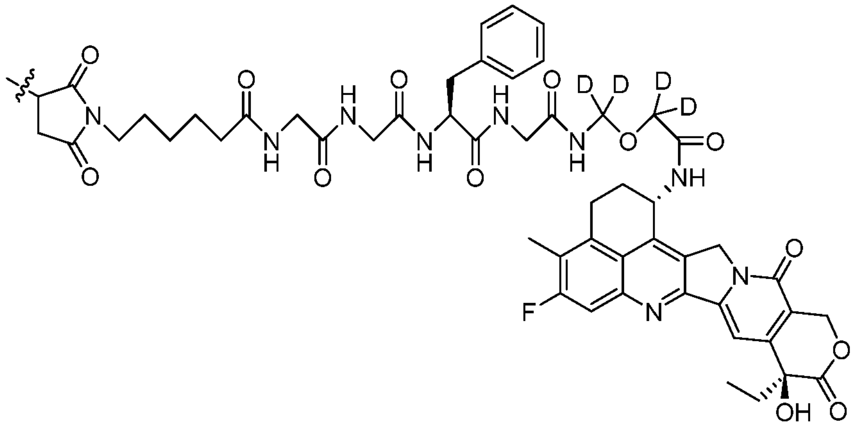

IIIa-2,

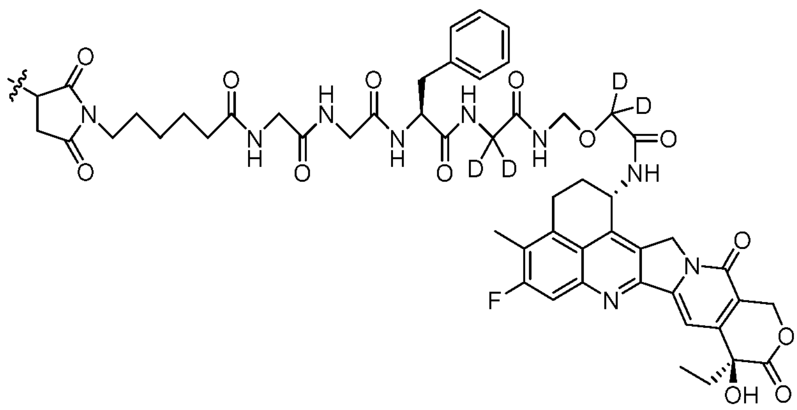

IIIa-3,

or

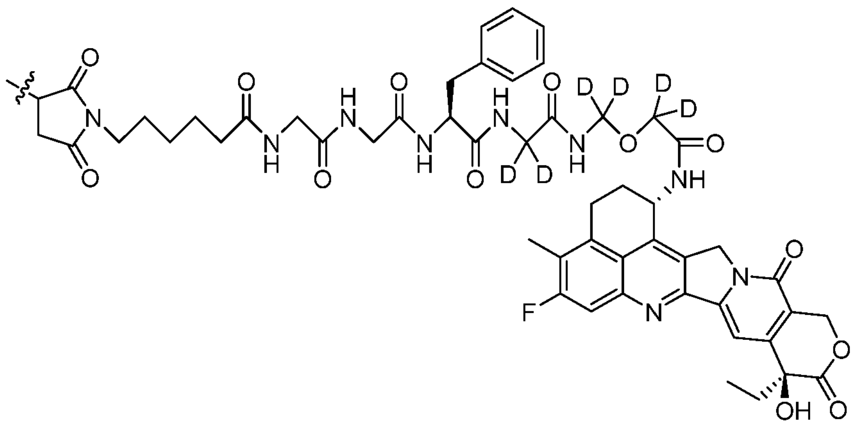

IIIa-4.

Embodiment 13. The antibody-drug conjugate or the pharmaceutically acceptable salt or the solvate thereof according to embodiment 7, wherein the antibody-drug conjugate has a structure represented by formula IV below:

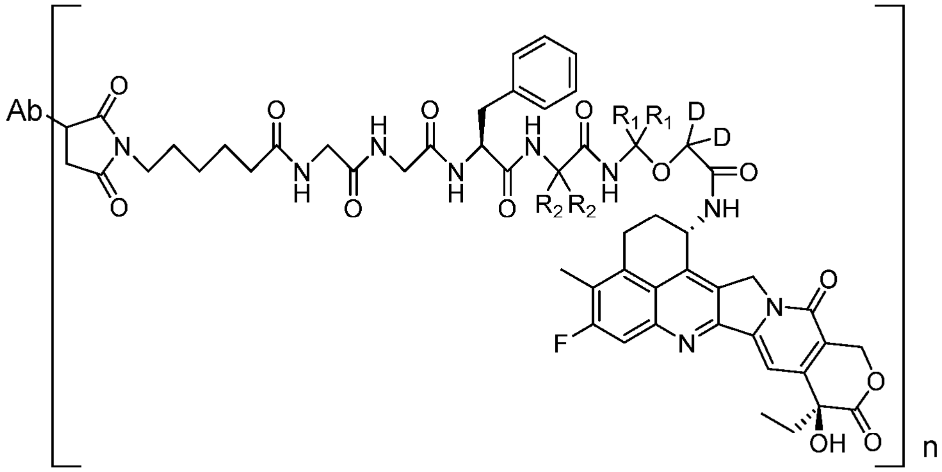

IV,

wherein $R_1$ and $R_2$ are each independently selected from hydrogen and deuterium.

Embodiment 14. The antibody-drug conjugate or the pharmaceutically acceptable salt or the solvate thereof according to embodiment 13, wherein the antibody-drug conjugate has a structure represented by formula IV-1, IV-2, IV-3, or IV-4 below:

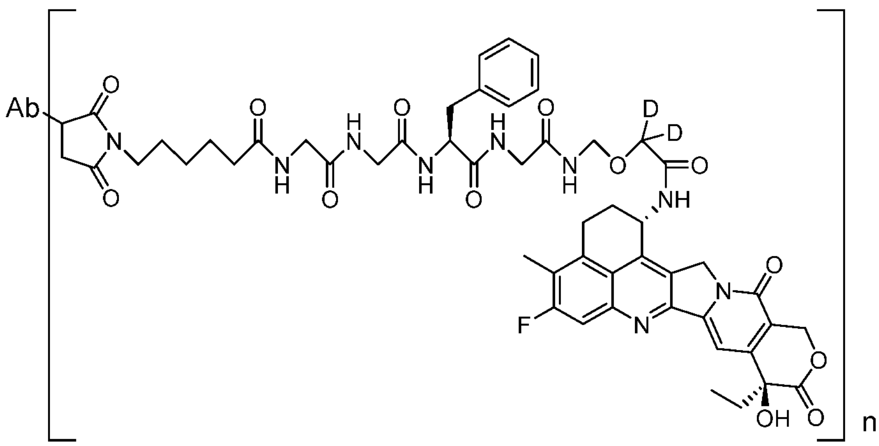

IV-1,

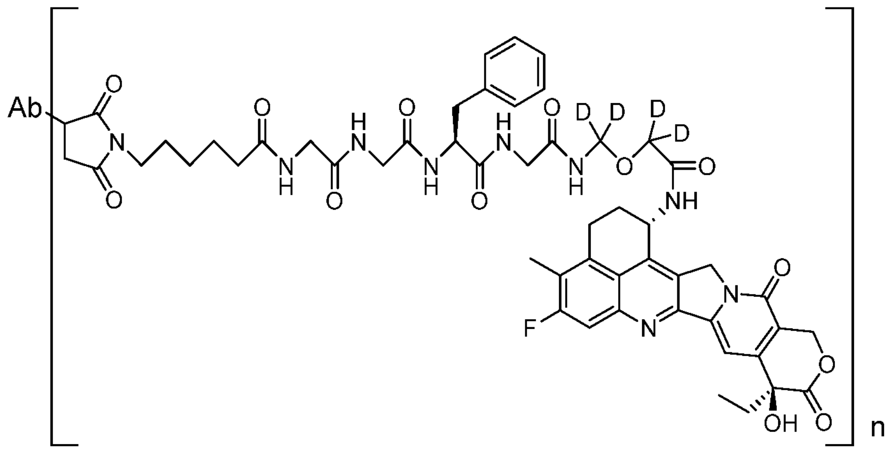

IV-2,

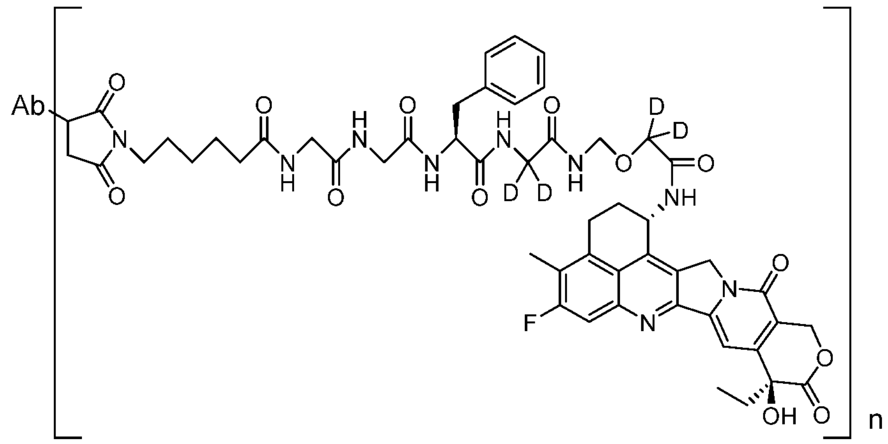

IV-3,

or

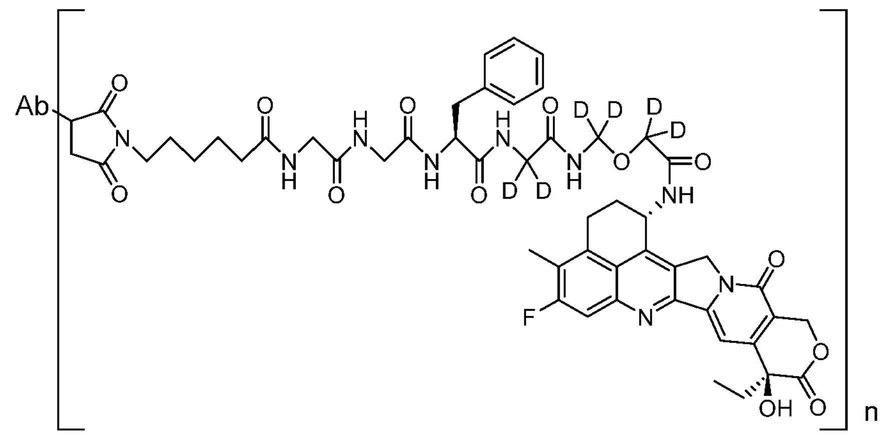

IV-4.

Embodiment 15. The antibody-drug conjugate or the pharmaceutically acceptable salt or the solvate thereof according to any one of embodiments 1 to 14, wherein n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, and 7 to 8, preferably an integer selected from 2 to 8, 3 to 8, 4 to 8, 5 to 8, 6 to 8, and 7 to 8, and more preferably an integer selected from 6 to 8.

Embodiment 16. The antibody-drug conjugate or the pharmaceutically acceptable salt or the solvate thereof according to any one of embodiments 1 to 15, wherein the antibody-drug conjugate or the pharmaceutically acceptable salt or the solvate thereof exhibits one of or a combination of several of the following properties:

(a) binding to CLDN18.2;

(b) blocking binding of CLDN18.2 to a ligand;
(c) showing endocytosis in cells expressing CLDN18.2;
(d) having killing activity against tumor cells expressing CLDN18.2;
(e) having a bystander effect;
(f) having ADCC activity;
(g) having CDC activity;
(h) not binding to CLDN18.1;
(i) not binding to cells not expressing CLDN18.2; and
(j) having no ADCC and/or CDC activity against cells expressing CLDN18.1.

Embodiment 17. A pharmaceutical composition comprising the antibody-drug conjugate or the pharmaceutically acceptable salt or the solvate thereof according to any one of embodiments 1 to 16, wherein preferably, the antibody-drug conjugate or the pharmaceutically acceptable salt or the solvate thereof in the pharmaceutical composition is heterogeneous or homogeneous; optionally, the pharmaceutical composition further comprises a pharmaceutically acceptable carrier.

Embodiment 18. The pharmaceutical composition according to embodiment 17, wherein the antibody-drug conjugate or the pharmaceutically acceptable salt or the solvate thereof in the pharmaceutical composition is heterogeneous, and the pharmaceutical composition has a DAR of 0 to 10, 0 to 9, 0 to 8, 1 to 10, 1 to 9, 1 to 8, 2 to 10, 2 to 9, 2 to 8, 3 to 8, 4 to 8, 5 to 8, 6 to 8, 7 to 8, 7.2 to 8, 7.4 to 8, 7.5 to 8, 7.6 to 8, 7.7 to 8, 7.8 to 8, 7.8 to 7.9, or 7.9 to 8; preferably 7 to 8, 7.2 to 8, 7.4 to 8, 7.6 to 8, or 7.7 to 8; more preferably about 7.1, about 7.2, about 7.4, about 7.6, about 7.7, or about 8.

Embodiment 19. Use of the antibody-drug conjugate or the pharmaceutically acceptable salt or the solvate thereof according to any one of embodiments 1 to 16, or the pharmaceutical composition according to embodiment 17 or 18 for preparing a medicament for treating a tumor.

Embodiment 20. Use of the antibody-drug conjugate or the pharmaceutically acceptable salt or the solvate thereof according to any one of embodiments 1 to 16, or the pharmaceutical composition according to embodiment 17 or 18, with a second therapeutic agent, for preparing a medicament for treating a tumor.

Embodiment 21. The use according to embodiment 19 or 20, wherein the tumor is a CLDN18.2-positive tumor; preferably, the tumor is gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and/or myeloma.

Embodiment 22. A method for treating a tumor, comprising administering to a patient in need a therapeutically effective amount of the antibody-drug conjugate or the pharmaceutically acceptable salt or the solvate thereof according to any one of embodiments 1 to 16 or the pharmaceutical composition according to embodiment 17 or 18.

Embodiment 23. The method according to embodiment 22, comprising contacting a tumor cell with the antibody-drug conjugate or the pharmaceutically acceptable salt or the solvate thereof according to any one of embodiments 1 to 16, or the pharmaceutical composition according to embodiment 17 or 18, thereby killing or inhibiting growth of the tumor cell.

Embodiment 24. The method according to embodiment 22 or 23, wherein the method further comprises administering a second therapeutic agent.

Embodiment 25. The method according to any one of embodiments 22-24, wherein the tumor is a CLDN18.2-positive tumor; preferably, the tumor is gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and/or myeloma.

Embodiment 26. A method for preparing the antibody-drug conjugate or the pharmaceutically acceptable salt or the solvate thereof according to any one of embodiments 11-16, comprising treating an anti-CLDN18.2 antibody or an antigen-binding fragment thereof under reducing conditions, and then reacting the anti-CLDN18.2 antibody or the antigen-binding fragment thereof with a linker-payload selected from a structure represented by formula III, wherein the structure represented by formula III is as follows:

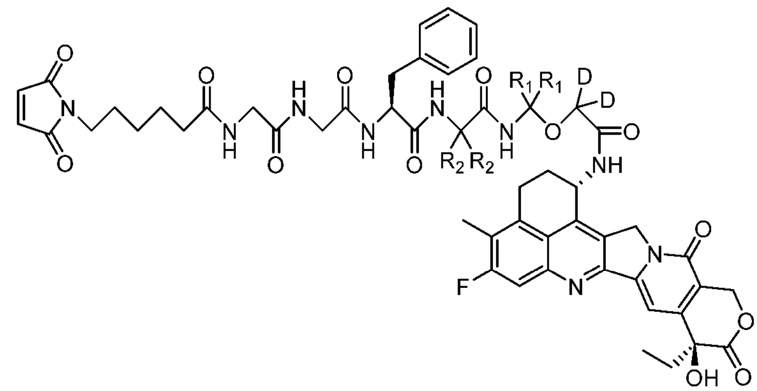

III,

wherein $R_1$ and $R_2$ are each independently selected from hydrogen and deuterium.

Embodiment 27. The method according to embodiment 26, wherein $R_1$ and $R_2$ are hydrogen.

Embodiment 28. An antibody-drug conjugate or a pharmaceutically acceptable salt or a solvate thereof, wherein the antibody-drug conjugate is formed by linking a structure represented by formula IIIa below to an antibody or an antigen-binding fragment thereof,

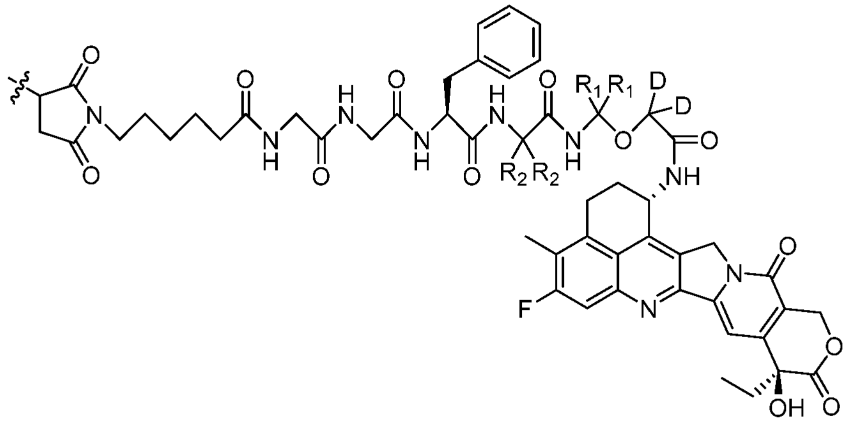

IIIa

wherein $R_1$ and $R_2$ are each independently selected from hydrogen and deuterium, and the 3-position of -(succinimidyl-3-yl-N)- in the structure represented by formula IIIa is linked to the antibody or the antigen-binding fragment thereof;

the antibody or the antigen-binding fragment thereof is an anti-CLDN18.2 antibody or an antigen-binding fragment thereof comprising HCDR1, HCDR2, HCDR3, LCDR1, LCDR2, and LCDR3, wherein

(1) the HCDR1 comprises an HCDR1 amino acid sequence in SEQ ID NO: 11, the HCDR2 comprises an HCDR2 amino acid sequence in SEQ ID NO: 11, the HCDR3 comprises an HCDR3 amino acid sequence in SEQ ID NO: 11, the LCDR1 comprises an LCDR1 amino acid sequence in SEQ ID NO: 16, the LCDR2 comprises an LCDR2 amino acid sequence in SEQ ID NO: 16, and the LCDR3 comprises an LCDR3 amino acid sequence in SEQ ID NO: 16;

(2) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;

(3) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO: 13, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;

(4) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in

SEQ ID NO: 14, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;

(5) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 17, the LCDR2 comprises an LCDR2 amino acid sequence in SEQ ID NO: 17, and the LCDR3 comprises an LCDR3 amino acid sequence in SEQ ID NO: 17;

(6) the HCDR1 comprises an HCDR1 amino acid sequence in SEQ ID NO: 12, the HCDR2 comprises an HCDR2 amino acid sequence in SEQ ID NO: 12, the HCDR3 comprises an HCDR3 amino acid sequence in SEQ ID NO: 12, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(7) the HCDR1 comprises an HCDR1 amino acid sequence in SEQ ID NO: 13, the HCDR2 comprises an HCDR2 amino acid sequence in SEQ ID NO: 13, the HCDR3 comprises an HCDR3 amino acid sequence in SEQ ID NO: 13, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(8) the HCDR1 comprises an HCDR1 amino acid sequence in SEQ ID NO: 14, the HCDR2 comprises an HCDR2 amino acid sequence in SEQ ID NO: 14, the HCDR3 comprises an HCDR3 amino acid sequence in SEQ ID NO: 14, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(9) the HCDR1 comprises an HCDR1 amino acid sequence in SEQ ID NO: 15, the HCDR2 comprises an HCDR2 amino acid sequence in SEQ ID NO: 15, the HCDR3 comprises an HCDR3 amino acid sequence in SEQ ID NO: 15, the LCDR1 comprises an LCDR1 amino acid sequence in SEQ ID NO: 18, the LCDR2 comprises an LCDR2 amino acid sequence in SEQ ID NO: 18, and the LCDR3 comprises an LCDR3 amino acid sequence in SEQ ID NO: 18;

(10) the HCDR1 comprises an HCDR1 amino acid sequence in SEQ ID NO: 29, the HCDR2 comprises an HCDR2 amino acid sequence in SEQ ID NO: 29, the HCDR3 comprises an HCDR3 amino acid sequence in SEQ ID NO: 29, the LCDR1 comprises an LCDR1 amino acid sequence in SEQ ID NO: 34, the LCDR2 comprises an LCDR2 amino acid sequence in SEQ ID NO: 34, and the LCDR3 comprises an LCDR3 amino acid sequence in SEQ ID NO: 34;

(11) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(12) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(13) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(14) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 35, the LCDR2 comprises an LCDR2 amino acid sequence in SEQ ID NO: 35, and the LCDR3 comprises an LCDR3 amino acid sequence in SEQ ID NO: 35;

(15) the HCDR1 comprises an HCDR1 amino acid sequence in SEQ ID NO: 30, the HCDR2 comprises an HCDR2 amino acid sequence in SEQ ID NO: 30, the HCDR3 comprises an HCDR3 amino acid sequence in SEQ ID NO: 30, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;

(16) the HCDR1 comprises an HCDR1 amino acid sequence in SEQ ID NO: 31, the HCDR2 comprises an HCDR2 amino acid sequence in SEQ ID NO: 31, the HCDR3 comprises an HCDR3 amino acid sequence in SEQ ID NO: 31, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(17) the HCDR1 comprises an HCDR1 amino acid sequence in SEQ ID NO: 32, the HCDR2 comprises an HCDR2 amino acid sequence in SEQ ID NO: 32, the HCDR3 comprises an HCDR3 amino acid sequence in SEQ ID NO: 32, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(18) the HCDR1 comprises an HCDR1 amino acid sequence in SEQ ID NO: 33, the HCDR2 comprises an HCDR2 amino acid sequence in SEQ ID NO: 33, the HCDR3 comprises an HCDR3 amino acid sequence in SEQ ID NO: 33, the LCDR1 comprises an LCDR1 amino acid sequence in SEQ ID NO: 36, the LCDR2 comprises an LCDR2 amino acid sequence in SEQ ID NO: 36, and the LCDR3 comprises an LCDR3 amino acid sequence in SEQ ID NO: 36;
(19) the HCDR1 comprises an HCDR1 amino acid sequence in SEQ ID NO: 43, the HCDR2 comprises an HCDR2 amino acid sequence in SEQ ID NO: 43, the HCDR3 comprises an HCDR3 amino acid sequence in SEQ ID NO: 43, the LCDR1 comprises an LCDR1 amino acid sequence in SEQ ID NO: 44, the LCDR2 comprises an LCDR2 amino acid sequence in SEQ ID NO: 44, and the LCDR3 comprises an LCDR3 amino acid sequence in SEQ ID NO: 44;
(20) the HCDR1 comprises an HCDR1 amino acid sequence in SEQ ID NO: 51, the HCDR2 comprises an HCDR2 amino acid sequence in SEQ ID NO: 51, the HCDR3 comprises an HCDR3 amino acid sequence in SEQ ID NO: 51, the LCDR1 comprises an LCDR1 amino acid sequence in SEQ ID NO: 52, the LCDR2 comprises an LCDR2 amino acid sequence in SEQ ID NO: 52, and the LCDR3 comprises an LCDR3 amino acid sequence in SEQ ID NO: 52; or
(21) the HCDR1 comprises an HCDR1 amino acid sequence in SEQ ID NO: 59, the HCDR2 comprises an HCDR2 amino acid sequence in SEQ ID NO: 59, the HCDR3 comprises an HCDR3 amino acid sequence in SEQ ID NO: 59, the LCDR1 comprises an LCDR1 amino acid sequence in SEQ ID NO: 60, the LCDR2 comprises an LCDR2 amino acid sequence in SEQ ID NO: 60, and the LCDR3 comprises an LCDR3 amino acid sequence in SEQ ID NO: 60.

Embodiment 29. The antibody-drug conjugate or the pharmaceutically acceptable salt or the solvate thereof according to embodiment 28, wherein

(1) the HCDR1, the HCDR2, the HCDR3, the LCDR1, the LCDR2, and the LCDR3 comprise amino acid sequences set forth in SEQ ID NOs: 3, 4, 7, 8, 9, and 10, respectively;
(2) the HCDR1, the HCDR2, the HCDR3, the LCDR1, the LCDR2, and the LCDR3 comprise amino acid sequences set forth in SEQ ID NOs: 3, 5, 7, 8, 9, and 10, respectively;
(3) the HCDR1, the HCDR2, the HCDR3, the LCDR1, the LCDR2, and the LCDR3 comprise amino acid sequences set forth in SEQ ID NOs: 3, 6, 7, 8, 9, and 10, respectively;
(4) the HCDR1, the HCDR2, the HCDR3, the LCDR1, the LCDR2, and the LCDR3 comprise amino acid sequences set forth in SEQ ID NOs: 19, 20, 24, 25, 26, and 27, respectively;
(5) the HCDR1, the HCDR2, the HCDR3, the LCDR1, the LCDR2, and the LCDR3 comprise amino acid sequences set forth in SEQ ID NOs: 19, 21, 24, 25, 26, and 27, respectively;
(6) the HCDR1, the HCDR2, the HCDR3, the LCDR1, the LCDR2, and the LCDR3 comprise amino acid sequences set forth in SEQ ID NOs: 19, 22, 24, 25, 26, and 27, respectively;
(7) the HCDR1, the HCDR2, the HCDR3, the LCDR1, the LCDR2, and the LCDR3 comprise amino acid sequences set forth in SEQ ID NOs: 19, 23, 24, 25, 26, and 27, respectively;
(8) the HCDR1, the HCDR2, the HCDR3, the LCDR1, the LCDR2, and the LCDR3 comprise amino acid sequences set forth in SEQ ID NOs: 19, 20, 24, 25, 26, and 28, respectively;
(9) the HCDR1, the HCDR2, the HCDR3, the LCDR1, the LCDR2, and the LCDR3 comprise amino acid sequences set forth in SEQ ID NOs: 19, 21, 24, 25, 26, and 28, respectively;
(10) the HCDR1, the HCDR2, the HCDR3, the LCDR1, the LCDR2, and the LCDR3 comprise amino acid sequences set forth in SEQ ID NOs: 19, 22, 24, 25, 26, and 28, respectively;
(11) the HCDR1, the HCDR2, the HCDR3, the LCDR1, the LCDR2, and the LCDR3 comprise amino acid sequences set forth in SEQ ID NOs: 19, 23, 24, 25, 26, and 28, respectively;
(12) the HCDR1, the HCDR2, the HCDR3, the LCDR1, the LCDR2, and the LCDR3 comprise amino acid sequences set forth in SEQ ID NOs: 37, 38, 39, 40, 41, and 42, respectively;

(13) the HCDR1, the HCDR2, the HCDR3, the LCDR1, the LCDR2, and the LCDR3 comprise amino acid sequences set forth in SEQ ID NOs: 45, 46, 47, 48, 49, and 50, respectively; or
(14) the HCDR1, the HCDR2, the HCDR3, the LCDR1, the LCDR2, and the LCDR3 comprise amino acid sequences set forth in SEQ ID NOs: 53, 54, 55, 56, 57, and 58, respectively.

Embodiment 30. The antibody-drug conjugate or the pharmaceutically acceptable salt or the solvate thereof according to embodiment 28 or 29, wherein the anti-CLDN18.2 antibody or the antigen-binding fragment thereof comprises a heavy chain variable region and a light chain variable region, wherein

(1) the heavy chain variable region and the light chain variable region comprise amino acid sequences having at least 80% identity to amino acid sequences set forth in SEQ ID NOs: 11 and 16, respectively;
(2) the heavy chain variable region and the light chain variable region comprise amino acid sequences having at least 80% identity to amino acid sequences set forth in SEQ ID NOs: 12 and 17, respectively;
(3) the heavy chain variable region and the light chain variable region comprise amino acid sequences having at least 80% identity to amino acid sequences set forth in SEQ ID NOs: 13 and 17, respectively;
(4) the heavy chain variable region and the light chain variable region comprise amino acid sequences having at least 80% identity to amino acid sequences set forth in SEQ ID NOs: 14 and 17, respectively;
(5) the heavy chain variable region and the light chain variable region comprise amino acid sequences having at least 80% identity to amino acid sequences set forth in SEQ ID NOs: 15 and 17, respectively;
(6) the heavy chain variable region and the light chain variable region comprise amino acid sequences having at least 80% identity to amino acid sequences set forth in SEQ ID NOs: 12 and 18, respectively;
(7) the heavy chain variable region and the light chain variable region comprise amino acid sequences having at least 80% identity to amino acid sequences set forth in SEQ ID NOs: 13 and 18, respectively;
(8) the heavy chain variable region and the light chain variable region comprise amino acid sequences having at least 80% identity to amino acid sequences set forth in SEQ ID NOs: 14 and 18, respectively;
(9) the heavy chain variable region and the light chain variable region comprise amino acid sequences having at least 80% identity to amino acid sequences set forth in SEQ ID NOs: 15 and 18, respectively;
(10) the heavy chain variable region and the light chain variable region comprise amino acid sequences having at least 80% identity to amino acid sequences set forth in SEQ ID NOs: 29 and 34, respectively;
(11) the heavy chain variable region and the light chain variable region comprise amino acid sequences having at least 80% identity to amino acid sequences set forth in SEQ ID NOs: 30 and 35, respectively;
(12) the heavy chain variable region and the light chain variable region comprise amino acid sequences having at least 80% identity to amino acid sequences set forth in SEQ ID NOs: 31 and 35, respectively;
(13) the heavy chain variable region and the light chain variable region comprise amino acid sequences having at least 80% identity to amino acid sequences set forth in SEQ ID NOs: 32 and 35, respectively;
(14) the heavy chain variable region and the light chain variable region comprise amino acid sequences having at least 80% identity to amino acid sequences set forth in SEQ ID NOs: 33 and 35, respectively;
(15) the heavy chain variable region and the light chain variable region comprise amino acid sequences having at least 80% identity to amino acid sequences set forth in SEQ ID NOs: 30 and 36, respectively;
(16) the heavy chain variable region and the light chain variable region comprise amino acid sequences having at least 80% identity to amino acid sequences set forth in SEQ ID NOs: 31 and 36, respectively;
(17) the heavy chain variable region and the light chain variable region comprise amino acid sequences having at least 80% identity to amino acid sequences set forth in SEQ ID NOs: 32 and 36, respectively;
(18) the heavy chain variable region and the light chain variable region comprise amino acid sequences having at least 80% identity to amino acid sequences set forth in SEQ ID NOs: 33 and 36, respectively;
(19) the heavy chain variable region and the light chain variable region comprise amino acid sequences having at least 80% identity to amino acid sequences set forth in SEQ ID NOs: 43 and 44, respectively;
(20) the heavy chain variable region and the light chain variable region comprise amino acid sequences having at least 80% identity to amino acid sequences set forth in SEQ ID NOs: 51 and 52, respectively; or
(21) the heavy chain variable region and the light chain variable region comprise amino acid sequences having at least 80% identity to amino acid sequences set forth in SEQ ID NOs: 59 and 60, respectively.

Embodiment 31. The antibody-drug conjugate or the pharmaceutically acceptable salt or the solvate thereof according to any one of embodiments 28-30, wherein the anti-CLDN18.2 antibody or the antigen-binding fragment thereof is of an IgG1, IgG2, or IgG4 isotype.
Embodiment 32. The antibody-drug conjugate or the pharmaceutically acceptable salt or the solvate thereof according to any one of embodiments 28-31, wherein the anti-CLDN18.2 antibody further comprises a heavy chain constant region and a light chain constant region, wherein the heavy chain constant region comprises an amino acid

sequence set forth in SEQ ID NO: 1 or an amino acid sequence having 1, 2, 3, 4, or 5 amino acid substitutions, deletions, and additions compared to the amino acid sequence set forth in SEQ ID NO: 1, and the light chain constant region comprises an amino acid sequence set forth in SEQ ID NO: 2 or an amino acid sequence having 1, 2, 3, 4, or 5 amino acid substitutions, deletions, and additions compared to the amino acid sequence set forth in SEQ ID NO: 2.
Embodiment 33. The antibody-drug conjugate or the pharmaceutically acceptable salt or the solvate thereof according to any one of embodiments 28-32, wherein the anti-CLDN18.2 antibody comprises a heavy chain and a light chain, wherein

(1) the heavy chain comprises an amino acid sequence having at least 80% identity to an amino acid sequence set forth in SEQ ID NO: 61 and the light chain comprises an amino acid sequence having at least 80% identity to an amino acid sequence set forth in SEQ ID NO: 62; or
(2) the heavy chain comprises an amino acid sequence having at least 80% identity to an amino acid sequence set forth in SEQ ID NO: 63 and the light chain comprises an amino acid sequence having at least 80% identity to an amino acid sequence set forth in SEQ ID NO: 64.

Embodiment 34. The antibody-drug conjugate or the pharmaceutically acceptable salt or the solvate thereof according to any one of embodiments 28-33, wherein both $R_1$ and $R_2$ are hydrogen.
Embodiment 35. The antibody-drug conjugate or the pharmaceutically acceptable salt or the solvate thereof according to any one of embodiments 28-34, wherein the antibody-drug conjugate is formed by linking a structure represented by formula IIIa to the antibody or the antigen-binding fragment thereof via a thioether bond.
Embodiment 36. The antibody-drug conjugate or the pharmaceutically acceptable salt or the solvate thereof according to any one of embodiments 28-35, wherein the antibody-drug conjugate or the pharmaceutically acceptable salt or the solvate thereof exhibits one of or a combination of several of the following properties:

(a) binding to CLDN18.2;
(b) blocking binding of CLDN18.2 to a ligand;
(c) showing endocytosis in cells expressing CLDN18.2;
(d) having killing activity against tumor cells expressing CLDN18.2;
(e) having a bystander effect;
(f) having ADCC activity;
(g) having CDC activity;
(h) not binding to CLDN18.1;
(i) not binding to cells not expressing CLDN18.2; and
(j) having no ADCC and/or CDC activity against cells expressing CLDN18.1.

Embodiment 37. A pharmaceutical composition comprising the antibody-drug conjugate or the pharmaceutically acceptable salt or the solvate thereof according to any one of embodiments 28-36, wherein preferably, the antibody-drug conjugate or the pharmaceutically acceptable salt or the solvate thereof in the pharmaceutical composition is heterogeneous or homogeneous; optionally, the pharmaceutical composition further comprises a pharmaceutically acceptable carrier.
Embodiment 38. The pharmaceutical composition according to embodiment 37, wherein the antibody-drug conjugate or the pharmaceutically acceptable salt or the solvate thereof in the pharmaceutical composition is heterogeneous, and the pharmaceutical composition has a DAR of 0 to 10, 0 to 9, 0 to 8, 1 to 10, 1 to 9, 1 to 8, 2 to 10, 2 to 9, 2 to 8, 3 to 8, 4 to 8, 5 to 8, 6 to 8, 7 to 8, 7.2 to 8, 7.4 to 8, 7.5 to 8, 7.6 to 8, 7.7 to 8, 7.8 to 8, 7.8 to 7.9, or 7.9 to 8; preferably 7 to 8, 7.2 to 8, 7.4 to 8, 7.6 to 8, or 7.7 to 8; more preferably about 7.1, about 7.2, about 7.4, about 7.6, about 7.7, or about 8.
Embodiment 39. Use of the antibody-drug conjugate or the pharmaceutically acceptable salt or the solvate thereof according to any one of embodiments 28-36, or the pharmaceutical composition according to embodiment 37 or 38 for preparing a medicament for treating a tumor.
Embodiment 40. Use of the antibody-drug conjugate or the pharmaceutically acceptable salt or the solvate thereof according to any one of embodiments 28-36, or the pharmaceutical composition according to embodiment 37 or 38, with a second therapeutic agent, for preparing a medicament for treating a tumor.
Embodiment 41. The use according to embodiment 39 or 40, wherein the tumor is a CLDN18.2-positive tumor; preferably, the tumor is gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and/or myeloma.

Embodiment 42. A method for treating a tumor, comprising administering to a patient in need a therapeutically effective amount of the antibody-drug conjugate or the pharmaceutically acceptable salt or the solvate thereof according to any one of embodiments 28-36 or the pharmaceutical composition according to embodiment 37 or 38.

Embodiment 43. The method according to embodiment 42, comprising contacting a tumor cell with the antibody-drug conjugate or the pharmaceutically acceptable salt or the solvate thereof according to any one of embodiments 28-36, or the pharmaceutical composition according to embodiment 37 or 38, thereby killing or inhibiting growth of the tumor cell.

Embodiment 44. The method according to embodiment 42 or 43, wherein the method further comprises administering a second therapeutic agent.

Embodiment 45. The method according to any one of embodiments 42-44, wherein the tumor is a CLDN18.2-positive tumor; preferably, the tumor is gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and/or myeloma.

**Example**

For clarity, the present disclosure is further described with the following examples, which are, however, not intended to limit the scope of the present disclosure. The reagents used in the present disclosure are commercially available and can be used without further purification.

The cells involved in the examples of the present disclosure and their sources are shown in the table below:

| Cell | Type | Source |
|---|---|---|
| KATOIII | Human gastric cancer cell | Cell Bank of the Chinese Academy of Sciences |
| NUGC-4 | Human gastric cancer cell | Cell Bank of the Chinese Academy of Sciences |
| NCI-N87 | Human gastric cancer cell | Kyinno Biotechnology Co., Ltd. |
| BxPC-3 | Human pancreatic cancer cell | Kyinno Biotechnology Co., Ltd. |
| NIH-3T3 | Mouse embryonic fibroblast | Cell Bank of the Chinese Academy of Sciences |
| U2OS | Human osteosarcoma cell | Cell Culture Center, Institute of Basic Medical Sciences, Chinese Academy of Medical Sciences |

## Example 1. Generation of Anti-CLDN18.2 Hybridoma Monoclonal Antibodies

### Construction of stable cell lines

cDNA encoding human CLDN18.2 (SEQ ID NO: 65) was obtained by gene synthesis and then subcloned into an expression vector pcDNA3.1. The expression vector was transfected into U2OS cells and NIH-3T3 cells according to the instructions of the Lipofectamine 2000 transfection reagent (Thermo, Cat. No. 11668019) to obtain stable cell lines U2OS-CLDN18.2 and NIH-3T3-CLDN18.2, respectively.

### Immunization of mice

Balb/c mice were immunized with the immunogen, the NIH-3T3-CLDN18.2 cells, by intraperitoneally injecting $5 \times 10^6$ to $10 \times 10^6$ NIH-3T3-CLDN18.2 cells into each mouse, and the immunization was performed once every 2 weeks for 5 times. Three days before fusion, each mouse was intraperitoneally injected with $1 \times 10^7$ NIH-3T3-CLDN18.2 cells for boost. After the completion of the immunization, serum was collected from each mouse for the titer assay of anti-CLDN18.2 specific antibodies. Mice with a higher antibody titer were selected for subsequent spleen cell fusion.

### Cell fusion

Based on the electrofusion technique, spleen cells ($1 \times 10^8$) from the immunized mice were fused with SP2/0 myeloma cells ($5 \times 10^7$) using the electrofusion system (BTX, ECM2001) to obtain hybridoma cells. After the fusion, the

cells were resuspended in HAT complete medium, added to a 96-well plate at 0.2 mL/well, and cultured in an incubator at 37 °C with 5% $CO_2$ for 7-10 days, and then hybridoma cell culture supernatants were taken for detection.

Screening of hybridomas

The binding activity of the antibodies against CLDN18.2 was detected by ELISA. U2OS-CLDN18.2 cells were added to a 96-well plate at 10,000 cells/well and cultured overnight in an incubator at 37 °C with 5% $CO_2$. The cell culture supernatant was discarded, the 96-well plate was washed 3 times with PBS buffer, and 2% polyoxymethylene was added, followed by immobilization at room temperature for 30 min. The 96-well plate was washed 3 times with PBS buffer, the hybridoma cell culture supernatant was added at 100 μL/well, and the mixture was incubated at room temperature for 1 h. The 96-well plate was washed 3 times with PBS buffer, HRP-Goat Anti-Mouse IgG Fcγ (Jackson immunoresearch, Cat. No. 115-035-071) was added at 100 μL/well, and the mixture was incubated at room temperature for 1 h. The 96-well plate was washed 3 times with PBS buffer, TMB (Thermo, Cat. No. 00-4201-56) was added at 100 μL/well, and the mixture was incubated for 10 min, followed by addition of a stop solution (1 M $H_2SO_4$) at 50 μL/well to stop the reaction. OD values at a wavelength of 450 nm were read using a microplate reader (Thermo, Varioskan Flash).

Hybridoma cells positive for ELISA detection were selected. The binding activity of the antibodies in the hybridoma cell culture supernatants against the cell lines KATOIII and NUGC-4 naturally expressing CLDN18.2 was detected by the FACS method.

Acquisition of cDNA

Hybridoma cells positive for ELISA and FACS detections were selected. Total RNA was isolated as a template from the cells using a total RNA extraction kit (Takara, Cat. No. 9767), and first strand cDNA was synthesized using the superscript III reverse transcriptase according to the instructions of the kit (Thermo, Cat. No. 18080051). Using the first strand cDNA as the template, PCR amplification was performed with the mouse IgG primer and the Kappa primer to obtain the sequences of the heavy chain variable region and the light chain variable region of the antibody. The PCR mixture was electrophoretically separated in a 1% agarose/Tris-borate gel containing 0.5 μg/mL ethidium bromide. A DNA fragment of the expected size was cut off from the gel and purified. The purified PCR product was cloned into the pMD-19T vector (Takara, Cat. No. 6013), which was then transfected into DH5α competent *E. coli* cells (Takara, Cat. No. 9057) for culturing. Single colonies were picked from the LB solid culture plate and subjected to DNA sequencing. The heavy chain variable region sequence and light chain variable region sequence of the antibodies (murine antibodies 28G3, 40F6, 22F12, 34G6, and 10D8) were obtained.

Construction and expression of humanized antibodies

Murine antibodies 28G3 (heavy chain variable region sequence: SEQ ID NO: 11; light chain variable region sequence: SEQ ID NO: 16) and 40F6 (heavy chain variable region sequence: SEQ ID NO: 29; light chain variable region sequence: SEQ ID NO: 34) were selected for humanization.

The murine antibodies 28G3 and 40F6 were humanized by CDR-grafting in combination with computer-aided design. Human germline antibody sequences with the highest sequence homology to the heavy chain variable region sequence and the light chain variable region sequence of the murine antibodies were selected from the protein database by alignment. CDRs of the murine antibodies 28G3 and 40F6 were grafted onto the framework regions of the selected human germline antibody sequences, and amino acid residues in the CDRs and/or in the framework regions were further mutated to obtain more candidate variable region sequences.

The above humanized VL region gene fragment was linked to a human κ chain constant region (with an amino acid sequence set forth in SEQ ID NO: 2) to construct a humanized light chain, and the above humanized VH region gene fragment was linked to a human IgG1 constant region (with an amino acid sequence set forth in SEQ ID NO: 1) to construct a humanized heavy chain. The humanized heavy chain expression plasmid and the humanized light chain expression plasmid were co-transfected into CHO cells for protein expression. The transfected cells were cultured at 37 °C with 8% $CO_2$. After 7-10 days of culturing, centrifugation was performed to obtain the cell culture supernatant, and the humanized antibodies in the cell culture supernatant were purified using a Protein A column (GE healthcare).

The amino acid sequence of the heavy chain of the humanized antibody hz28G3-1.1 is set forth in SEQ ID NO: 61, and the amino acid sequence of the light chain is set forth in SEQ ID NO: 62; the amino acid sequence of the heavy chain of the humanized antibody hz28G3-1.3 is set forth in SEQ ID NO: 63, and the amino acid sequence of the light chain is set forth in SEQ ID NO: 64. The amino acid sequences of the variable regions of the other humanized antibodies are set forth in Table S1.

## Example 2. Preparation of MC-GGFG-Deuterated DXd (MC-GGFG-DDDXd)

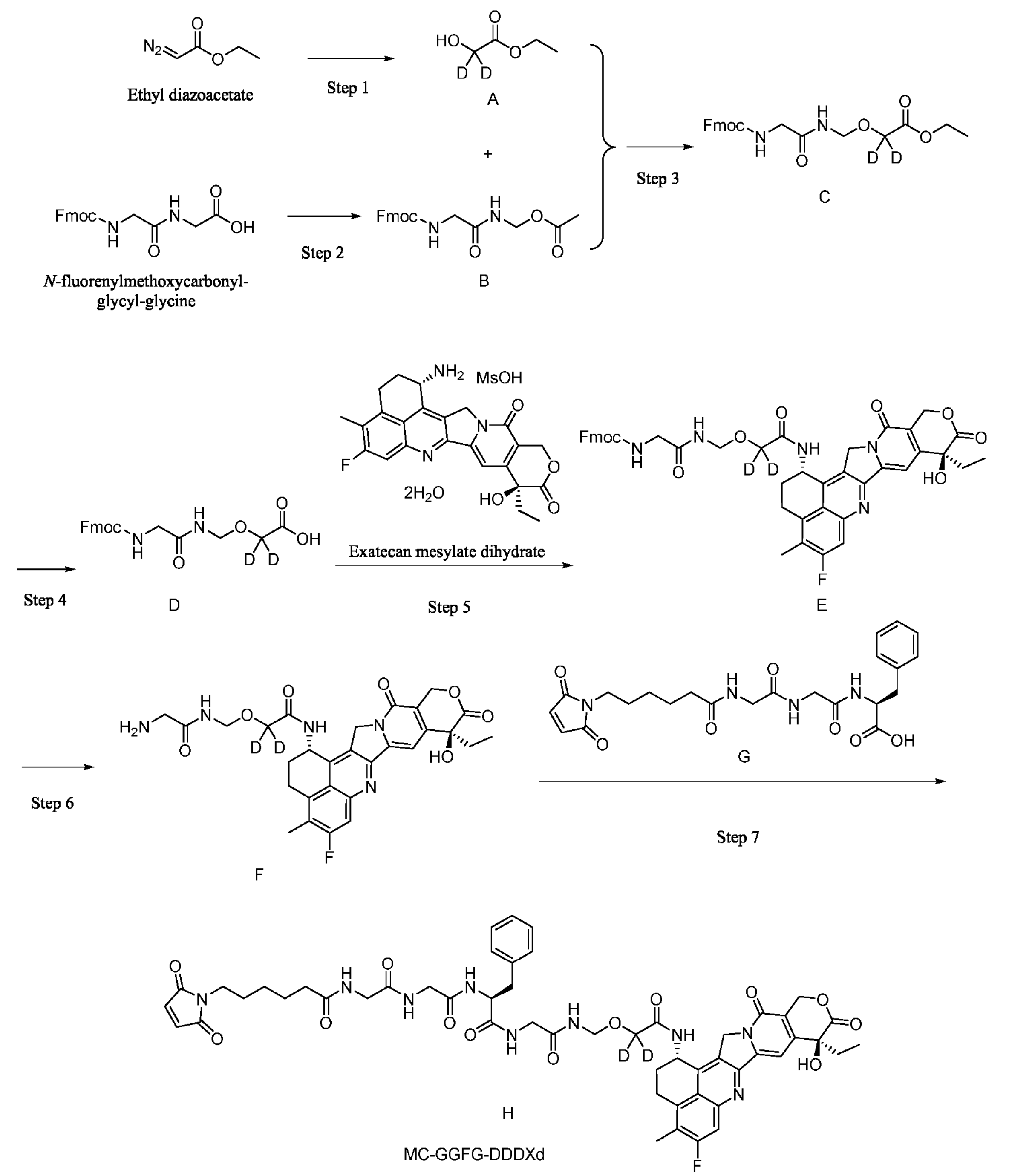


Step 1: synthesis of intermediate A

80 g of ethyl diazoacetate was added to a 3 L single-neck flask under nitrogen atmosphere, and 800 mL of dichloromethane and 800 mL of 1% deuterated acetic acid solution (8 g of deuterated acetic acid dissolved in 800 mL of deuterium oxide) were added. The single-neck flask was stirred at room temperature for 75 h under light-shielding conditions. Liquid separation was performed. The organic phase was collected, and the aqueous phase was extracted twice with dichloromethane (200 mL × 2). The organic phases were combined. The combined organic phase was washed with 200 mL of deuterium oxide. The resulting organic phase was dried over anhydrous sodium sulfate and filtered to remove sodium sulfate. The filtrate was concentrated to dryness under reduced pressure at 20 °C to obtain 49.25 g of

intermediate A in 66% yield. CAS of intermediate A: 1356471-71-0.

Step 2: synthesis of intermediate B

50 g of *N*-fluorenylmethoxycarbonyl-glycyl-glycine was weighed and added to a 2 L round-bottom flask, and 750 mL of tetrahydrofuran and 150 mL of glacial acetic acid were added. The mixture was stirred at 40 °C for 20 min, and 100 g of lead tetraacetate was added. The mixture was heated to 80 °C and then reacted for 3 h. The reaction solution was cooled to room temperature and filtered under vacuum. The filter cake was washed with 250 mL of ethyl acetate. The filtrate was concentrated to dryness and dissolved with 330 mL of dichloromethane and 670 mL of ethyl acetate to obtain the organic phase. The organic phase was washed 3 times with 30% aqueous potassium bicarbonate (500 mL × 3), then dried over anhydrous sodium sulfate, and filtered. The filtrate was concentrated to dryness under reduced pressure, and dissolved with 100 mL of dichloromethane. Then, 100 mL of n-hexane was added. The mixture was stirred at room temperature until a solid was precipitated, and then 300 mL of a mixed solution of n-hexane and dichloromethane (n-hexane:dichloromethane = 1:1) was added. The mixture was stirred overnight. The reaction solution was filtered. The filter cake was dried in a vacuum oven at 40 °C for 4 h to obtain 37.3 g of intermediate B in 72% yield. LC-MS (ESI) m/z: 391.09 $[M+Na]^+$; CAS of intermediate B: 1599440-06-8.

Step 3: synthesis of intermediate C

78 g of intermediate B was weighed and added to a 3000 mL single-neck flask, and 800 mL of dichloromethane and 45 g of intermediate A were added thereto. The 3000 mL single-neck flask was placed in an ice-water bath to be cooled to 0 °C. 16 g of lithium tert-butoxide was dissolved in 400 mL of dichloromethane to prepare a lithium tert-butoxide solution, which was then added to a 3000 mL single-neck flask and reacted at 0 °C for 3 h. The reaction solution was warmed to room temperature, and 800 mL of water was added. The mixture was stirred. Liquid separation was performed. The organic phase was collected, and the aqueous phase was extracted with 400 mL of dichloromethane. The organic phases were combined. The combined organic phase was washed once with 800 mL of saturated brine, dried over anhydrous sodium sulfate, and filtered. The filtrate was concentrated under reduced pressure and subjected to silica gel column chromatography (petroleum ether:ethyl acetate = 3:1) to give 61 g of intermediate C in 70% yield, LC-MS (ESI) m/z: 437.34 $[M+Na]^+$; CAS of intermediate C: 2760715-83-9.

Step 4: synthesis of compound D

31.2 g of compound C was added to 270 mL of deuterated methanol and 70 mL of deuterium oxide. The mixture was stirred in an ice bath, and then 5.5 g of NaOH was added. The mixture was warmed to room temperature and stirred overnight. 300 mL of ethyl acetate and 300 mL of water were added to the reaction solution for extraction. 20 mL of glacial acetic acid was added to the aqueous layer to adjust pH to 2-3, and a solid was precipitated. The mixture was filtered under vacuum to obtain 20.3 g of compound D in 69.8% yield. $^1$H-NMR (500 MHz, DMSO-$d_6$) δ 8.70 (1H, t, J = 6.6), 7.89 (2H, d, J = 7.5), 7.72 (2H, d, J = 7.5), 7.57 (1H, t, J = 6.0), 7.42 (2H, t, J = 7.5), 7.34 (2H, t, J = 7.5), 4.61 (2H, d, J = 6.6), 4.30 (2H, d, J = 7.1), 4.23 (1H, m), 3.64 (2H, d, J = 6.0); LC-MS (ESI) m/z: 409.08 $[M+Na]^+$.

Step 5: synthesis of compound E

2.0 g of exatecan mesylate dihydrate and 1.63 g of compound D were weighed and added to a 100 mL round-bottom flask. 40 mL of N,N-dimethylformamide was added. The mixture was stirred. The reaction solution was cooled to 0 °C. 2.0 g of 2-(7-azobenzotriazol)-N,N,N',N'-tetramethyluronium hexafluorophosphate and 1.82 g of N,N-diisopropylethylamine were added in sequence. The mixture was reacted at 0 °C for 3 h. The reaction solution was poured into 120 mL of ice water. The mixture was stirred for 1 h. The reaction solution was filtered. The filter cake was dissolved with dichloromethane. The reaction solution was subjected to silica gel column chromatography (100 g of 100-200 mesh silica gel, dichloromethane:methanol = 30:1, 2 L) to obtain 2.6 g of compound E in 92% yield. $^1$H-NMR (500 MHz, DMSO-$d_6$), δ 8.78 (1H, t, J = 6.6),8.47 (1H, d, J = 9.0), 7.86 (2H, d, J = 7.5), 7.73 (1H, d, J = 11.0), 7.68 (2H, d, J = 7.5), 7.53 (1H, t, J = 6.0), 7.39 (2H, t, J = 7.5), 7.30 (2H, m), 7.29 (1H, s), 6.50 (1H, brs), 5.56 (1H, m), 5.39 (2H, m), 5.14 (2H, m), 4.64 (2H, m), 4.25 (2H, d, J = 6.8), 4.19 (1H, m), 3.62 (2H, d, J = 6.0), 3.15 (2H, m), 2.35 (3H, s), 2.17 (2H, m),1.83 (2H, m), 0.85 (3H, t, J = 7.3); LC-MS (ESI) m/z: 804.84 $[M+H]^+$.

Step 6: preparation of compound F

0.38 g of 1,8-diazabicyclo[5.4.0]undec-7-ene was weighed and added to a 100 mL round-bottom flask, and then 20 mL of tetrahydrofuran was added to the round-bottom flask. The mixture was stirred. The reaction solution was cooled to

0 °C. 2.0 g of compound E was weighed and prepared into a solution with 20 mL of tetrahydrofuran. The prepared compound E solution was slowly added to the 100 mL round-bottom flask. The mixture naturally heated to room temperature and reacted for 3 h. The reaction solution was filtered under nitrogen atmosphere to give 1.45 g of compound F in 98% yield. $^1$H-NMR (500 MHz, DMSO-$d_6$) δ 8.76 (1H, m), 7.72 (1H, m), 7.28 (1H, s), 5.48 (3H, m), 5.16 (2H, m), 4.61 (2H, m), 3.40 (2H, m), 3.20 (2H, m), 2.35 (3H, s), 2.17 (2H, m), 1.83 (2H, m), 0.86 (3H, t, J = 7.1); LC-MS (ESI) m/z: 582.39 $[M+H]^+$.

Step 7: preparation of compound H

1.00 g of compound F and 0.97 g of compound G were weighed and added to a 100 mL round-bottom flask, and 10 mL of N,N-dimethylformamide was added. The mixture was cooled to -20 °C, and 0.34 g of 1-hydroxybenzotriazole and 0.49 g of 1-(3-dimethylaminopropyl)-3-ethylcarbodiimide hydrochloride were added. The mixture was reacted at -20 °C for 3 h. 20 mL of dichloromethane and 20 mL of water were added to the reaction solution. The mixture was stirred for 0.5 h and left to stand for liquid separation. The organic phase was collected. The organic phase was dried over anhydrous sodium sulfate and filtered. The filtrate was concentrated to dryness under reduced pressure. The residue was subjected to silica gel column chromatography (dichloromethane:methanol = 15:1) to obtain 400 mg of compound H in about 22% yield. MS m/z: 1037.08 $[M+H]^+$. $^1$H-NMR(500 MHz, DMSO-$d_6$) δ 8.62 (1H, t, J=6.5), 8.49 (1H, d, J=8.5), 8.29 (1H, t, J=5.5), 8.12 (1H, d, J=8.0), 8.06 (1H, t, J=5.5), 8.00 (1H, t, J=5.5), 7.74 (1H, d, J=10.5), 7.30 (1H, s), 7.27~7.12 (5H, m), 6.98 (2H, s), 6.51 (1H, brs), 5.61~5.58(1H, m), 5.45~5.37 (2H, m), 5.22~5.13 (2H, m), 4.64 (2H, d, J=6.5), 4.49~4.45 (1H, m), 3.76~3.57 (6H, m), 3.37~3.32 (2H, m), 3.24~3.09 (2H, m), 3.02 (1H, dd, J=4.5,14.0), 2.77 (1H, dd, J=9.5,13.5), 2.36 (3H,s), 2.23~2.14 (2H, m), 2.09 (2H, t, J=7.5), 1.91~1.79 (2H, m), 1.49~1.42 (4H, m), 1.20~1.14(2H, m), 0.87 (3H, t, J=7.5); HR-MS m/z: 1036.4194 $[M+H]^+$.

## Example 3. Preparation of Anti-CLDN18.2 Antibody-Drug Conjugates

**Reagents:**

Antibody: the anti-CLDN18.2 antibodies hz28G3-1.1 and hz28G3-1.3 prepared in Example 1, and 15F9 (prepared in-house, the amino acid sequences of the heavy and light chains are set forth in SEQ ID NOs: 67 and 68 of the present disclosure); and
linker-payload: compound H (MC-GGFG-DDDXd) prepared in Example 2.

**Experimental procedures:**

1. Reduction of the antibodies: The antibodies were each buffer-exchanged into a histidine buffer (1.43 mg/mL L-histidine and 2.27 mg/mL L-histidine hydrochloride monohydrate) at pH 6.0, and the antibody concentration was adjusted to 10 mg/mL with the histidine buffer (pH 6.0). The solution was added to a light-protected glass bottle, and a 10 mM TCEP (tris(2-carboxyethyl)phosphine hydrochloride) solution was added to enable the molar ratio of the antibody to TCEP to be 1:8. The mixture was incubated at 35 °C for 1 h in the dark with gentle stirring, so as to reduce the inter-chain disulfide bonds of the antibody to obtain reaction solution 1.
2. Conjugation of the antibodies with the linker-payload: After reaction solution 1 was incubated at 22 °C for 10 min, a DMSO solution was added (DMSO solution:reaction solution 1 (v/v) = 1:20), and the mixture was mixed uniformly. A linker-payload solution at a concentration of 10 mg/mL, which was dissolved with DMSO, was added to enable the molar ratio of the antibody to the linker-payload to be 1:10. The mixture was incubated at 22 °C for 2 h in the dark with gentle stirring, so as to conjugate the antibody with the linker-payload to obtain reaction solution 2.
3. Coupling termination and purification: Reaction solution 2 was ultrafiltered with a histidine buffer at pH 6.0 to obtain the anti-CLDN18.2 antibody-drug conjugates hz28G3-1.1-DDDXd, hz28G3-1.3-DDDXd, and 15F9-DDDXd.

## Example 4. Determination of DAR Values of Antibody-Drug Conjugates

DAR values were determined by the LC-MS method. An appropriate amount of the anti-CLDN18.2 antibody-drug conjugates prepared in Example 3 was taken, and an appropriate amount of glycosidase PNGase F (RHINO BIO, China) was added. The reaction was reacted at 37 °C overnight. Mass spectrometry was performed using a high-resolution mass spectrometer Xevo G2-XS (Waters, USA) with data collected in Native and positive ion modes. Data processing was performed using the software UNIFI 1.8.2.169 (Waters, USA).

The anti-CLDN18.2 antibody-drug conjugates prepared by the method of Example 3 include:

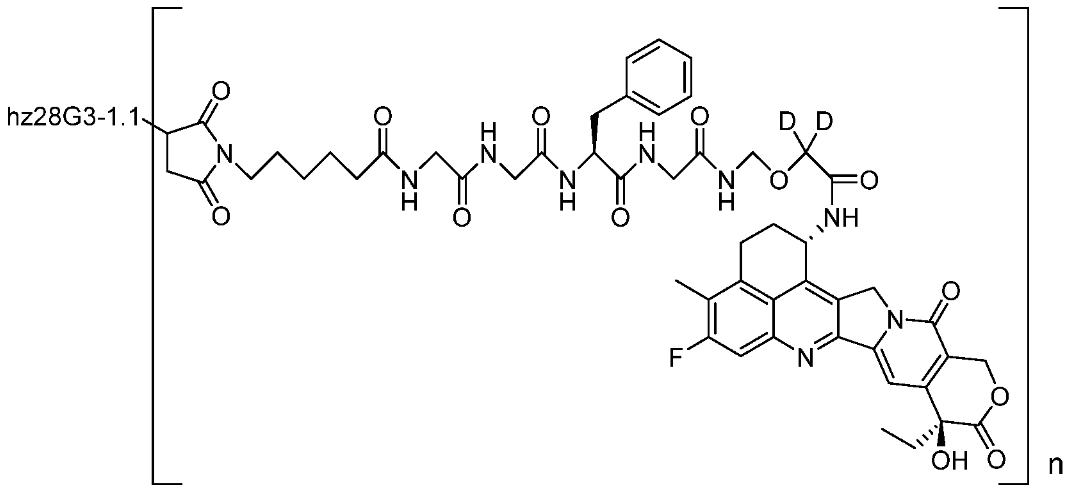

hz28G3-1.1-DDDXd, with the DAR value determined to be 7.2;

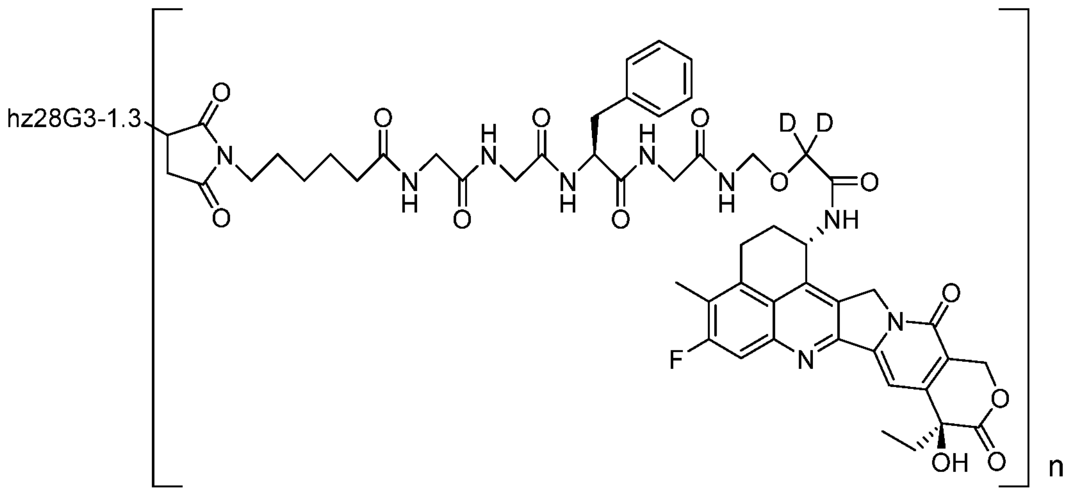

hz28G3-1.3-DDDXd with the DAR value determined to be 7.4; and

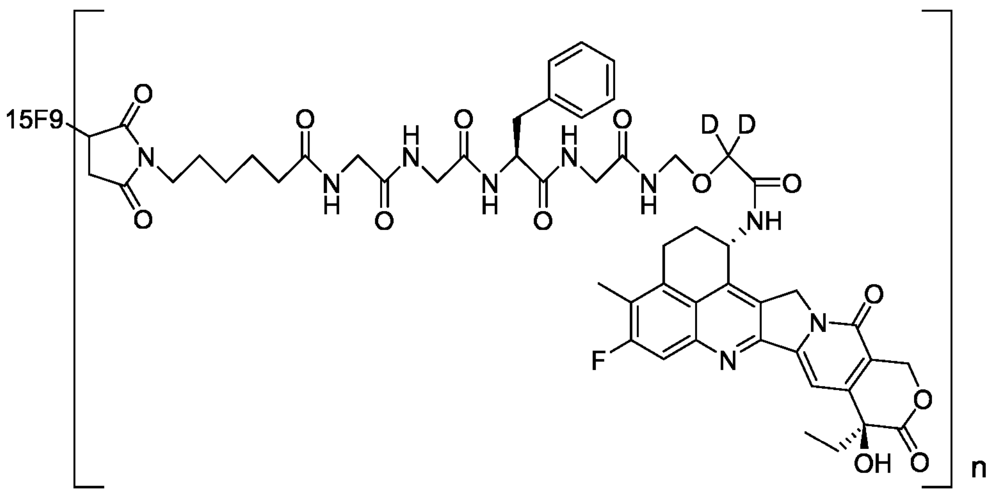

15F9-DDDXd with the DAR value determined to be 7.7.

**Example 5. Preparation of Anti-CLDN18.2 Antibody-Drug Conjugates and Determination of DAR Values**

The anti-CLDN18.2 antibody-drug conjugates were prepared by referring to the method of Example 3 and determined for DAR values by referring to the method of Example 4. The following anti-CLDN18.2 antibody-drug conjugates were obtained:

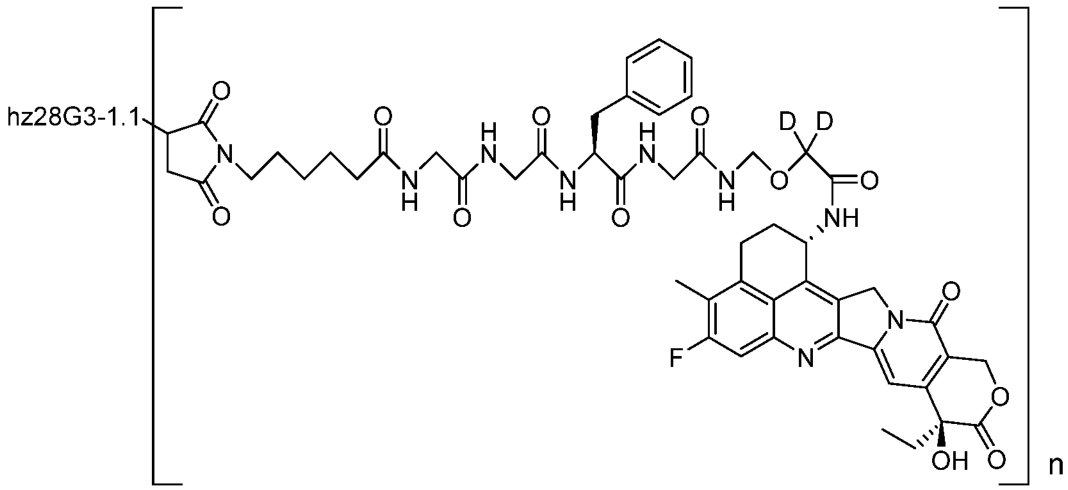

hz28G3-1.1-DDDXd with the DAR value determined to be 7.4; and

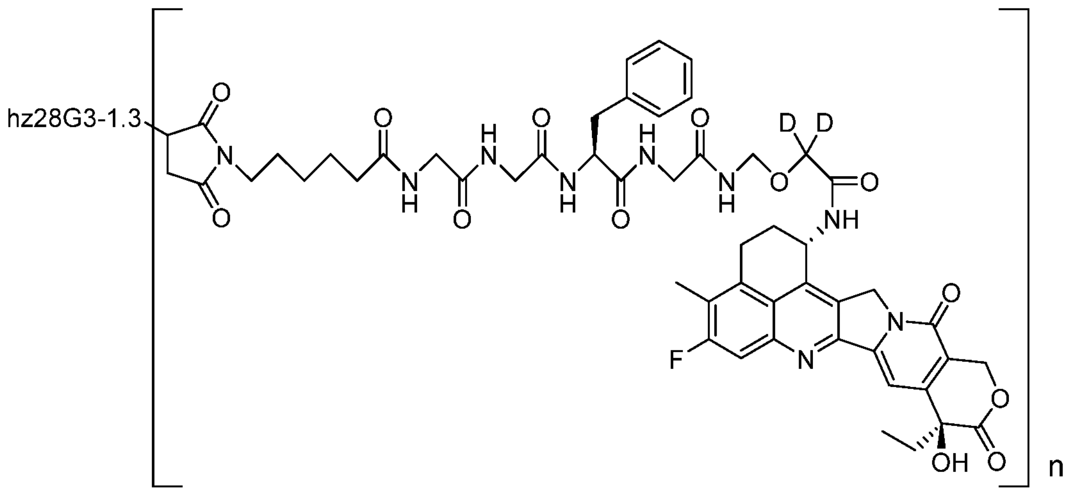

hz28G3-1.3-DDDXd, with the DAR value determined to be 7.6.

**Example 6. Aggregate Verification for Antibody-Drug Conjugates**

Components of the anti-CLDN18.2 antibody-drug conjugates prepared in Example 3 were separated using a gel chromatographic column. Elution was performed using a neutral buffer containing 10% isopropanol as the mobile phase. The components were eluted sequentially in descending order according to their molecular weights. The gel chromatographic column used was an ACQUITY UPLC Protein BEH SEC Column 200Å, 1.7 μm, 4.6 × 300 mm, and the column temperature was 25 °C. The mobile phase was 50 mM phosphate buffered saline-200 mM sodium chloride-10% isopropanol at pH 7.0 (12.53 g of disodium hydrogen phosphate dodecahydrate, 2.33 g of sodium dihydrogen phosphate dihydrate, and 11.69 g of sodium chloride were weighed, about 800 mL of ultrapure water was added, and the mixture was stirred until complete dissolution; 100 mL of isopropanol was added, ultrapure water was added to bring the volume to 1000 mL, and the mixture was mixed uniformly and then filtered through a 0.22 μm filter). 20 μg of the anti-CLDN18.2 antibody conjugates were precisely taken and injected into a liquid chromatograph, and the detection was performed at the wavelength of 280 nm. The flow rate was 0.3 mL/min, and isocratic elution was performed for 15 min.

Data were processed, and the results were quantitatively analyzed using the area normalization method. The peak area percentages of the aggregate, the monomer, and the low molecular weight impurities of the anti-CLDN18.2

antibody-drug conjugates were calculated. The aggregate peak appeared before the main peak, the monomer peak was the main peak, and the low molecular weight impurity peaks appeared after the main peak. The content proportions of the monomer, the aggregate, and the low molecular weight impurities of the anti-CLDN18.2 antibody-drug conjugates are shown in Table 1.

Table 1. The content proportions of the monomer, the aggregate, and the low molecular weight impurities of the anti-CLDN18.2 antibody-drug conjugates

| Name | hz28G3-1.1-DDDXd | hz28G3-1.3-DDDXd | 15F9-DDDXd |
|---|---|---|---|
| Aggregate | 0.80% | 0.54% | 1.62% |
| Monomer | 99.10% | 99.36% | 98.11% |
| Low molecular weight impurities | 0.10% | 0.10% | 0.27% |

**Example 7. Cell Binding Activity of Anti-CLDN18.2 Antibody-Drug Conjugates**

The cell-binding activity of the anti-CLDN18.2 antibody-drug conjugates prepared in Example 5 was determined by flow cytometry using hz28G3-1.1 and hz28G3-1.3 as controls. CLDN18.2-overexpressing NIH-3T3, BxPC-3, and NCI-N87 cell lines were diluted to $1 \times 10^6$ cells/mL and added to a 96-well plate at 100 $\mu$L/well. The anti-CLDN18.2 antibody-drug conjugate subjected to gradient dilution with an FACS buffer (Miltenyi Biotec, Cat. No. 130-091-221) (for BxPC-3 cells, an amount that resulted in a final concentration of 300 nM in the well was used as the initial concentration for the gradient dilution, with a 4-fold gradient dilution for a total of 10 concentrations; for NIH-3T3 and BxPC-3, an amount that resulted in a final concentration of 333.33 nM in the well was used as the initial concentration for the gradient dilution, with a 4-fold gradient dilution for a total of 10 concentrations) was added. After incubation at 4 °C for 60 min, the cell culture was centrifuged at 1000 rpm for 5 min, and the supernatant was discarded. The cells were washed 3 times with pre-cooled PBS (pH 7.4), and a goat-anti-human IgG Fc$\gamma$-PE secondary antibody (Jackson immunoresearch, Cat. No. 109-116-170) diluted at a ratio of 1:200 (v/v) was added at 100 $\mu$L/well. The cells were incubated at 4 °C for 30 min. The cells were washed 3 times with pre-cooled PBS (pH 7.4) and resuspended in 100 $\mu$L of PBS (pH 7.4). Then, the fluorescence signals were analyzed and detected using a flow cytometer (Sartorius, iQUE). The binding activity of the anti-CLDN18.2 antibody-drug conjugates against CLDN18.2 on the cell surface was expressed by mean fluorescence intensity (MFI) of staining. Data were analyzed using GraphPad Prism 5. The results are shown in FIGs. 1A-1C, and the calculated $EC_{50}$ values are shown in Table 2 below. The results showed that both hz28G3-1.1-DDDXd and hz28G3-1.3-DDDXd were able to effectively bind to CLDN18.2-overexpressing cells.

Table 2. Binding activity of the antibodies or antibody-drug conjugates against CLDN18.2-overexpressing cells

| | $EC_{50}$(nM) | | |
|---|---|---|---|
| | NIH-3T3 | BxPC-3 | NCI-N87 |
| hz28G3-1.1-DDDXd | 0.8959 | 1.129 | 1.166 |
| hz28G3-1.3-DDDXd | 0.5928 | 0.9462 | 1.020 |
| hz28G3-1.1 | 2.273 | 2.631 | 2.614 |
| hz28G3-1.3 | 1.756 | 3.425 | 2.731 |

**Example 8. Endocytosis Assay of Anti-CLDN18.2 Antibody-Drug Conjugates**

The endocytosis of the anti-CLDN18.2 antibody-drug conjugates prepared in Example 5 in CLDN18.2-overexpressing cells was assayed by flow cytometry using hz28G3-1.1 and hz28G3-1.3 as controls. CLDN18.2-overexpressing NIH-3T3, BxPC-3, and NCI-N87 cell lines and a CLDN18.2-positive natural cell line NUGC-4 were adjusted to a concentration of $2 \times 10^6$ cells/mL and added to a 96-well plate at 20 $\mu$L/well. After the labeled antibody (Sartorius, 90564) was co-incubated with the anti-CLDN18.2 antibody-drug conjugate subjected to gradient dilution with the FACS buffer (Miltenyi Biotec, Cat. No. 130-091-221) (the volume ratio of the labeled antibody to ADC was 1:1) at 37 °C for 15 min, the co-incubation system was added to the 96-well plate at 20 $\mu$L/well. An amount that resulted in a final concentration of 20 nM in the well was used as the initial concentration for the gradient dilution of the anti-CLDN18.2 antibody-drug conjugate, with a 3-fold gradient dilution for a total of 8 concentrations. After incubation for 2 h, the cells were placed in a flow cytometer (Sartorius, iQUE) and the fluorescence reading for the RL1 channel was determined. Data were analyzed using GraphPad Prism 5. The results are shown in FIGs. 2A-2D, and the calculated $EC_{50}$ values are shown in Table 3 below. The results

showed that both hz28G3-1.1-DDDXd and hz28G3-1.3-DDDXd were able to effectively exert endocytosis in CLDN18.2-overexpressing cells.

Table 3. Endocytosis of the antibodies or antibody-drug conjugates in CLDN18.2-overexpressing cells

| | $EC_{50}$(nM) | |
|---|---|---|
| | NIH-3T3 | NCI-N87 |
| hz28G3-1.1-DDDXd | 3.685 | 4.409 |
| hz28G3-1.3-DDDXd | 1.846 | 3.241 |
| hz28G3-1.1 | 2.570 | 7.842 |
| hz28G3-1.3 | 4.262 | 6.681 |

**Example 9. Killing of Tumor Cells by Anti-CLDN18.2 Antibody-Drug Conjugates**

To detect the killing of CLDN18.2-positive tumor cells by the anti-CLDN18.2 antibody-drug conjugates prepared in Example 5, the killing activity detection was performed using CLDN18.2-overexpressing NIH-3T3, BxPC-3, and NCI-N87 cells.

The cells in the logarithmic growth phase were taken. NIH-3T3, BxPC-3, and NCI-N87 cells were adjusted to cell densities of $1 \times 10^4$ cells/mL, $2 \times 10^4$ cells/mL, and $2 \times 10^4$ cells/mL respectively, and added to a 96-well plate at 100 μL/well. The cells were subjected to adherent culture at 37 °C with 5% $CO_2$ overnight. The anti-CLDN18.2 antibody-drug conjugate was prepared with a DMEM medium containing 10% FBS. For NIH-3T3 cells, 3 μg/mL was used as an initial concentration, with a 3-fold gradient dilution for a total of 9 concentrations; for BxPC-3 and NCI-N87 cells, 5 μg/mL was used as the initial concentration, with a 5-fold gradient dilution for a total of 9 concentrations. Cells subjected to overnight adherence culture were taken out. For the experimental group, the diluted anti-CLDN18.2 antibody-drug conjugate was added at 50 μL/well, and for the control group, a DMEM medium containing 10% FBS was added at 50 μL/well. After culturing for 96 h or 120 h, the cells were assayed using the CellTiter-Glo Luminescent Cell kit (Promega, Cat. No. G7572). Specifically, the 96-well plate was taken out, the CTG assay buffer (Promega, Cat. No. G7572) was added at 75 μL/well, and the mixture was mixed uniformly by shaking and incubated at room temperature for 10 min in the dark. Then, 180 μL of the solution was pipetted from each well and transferred to an opaque white plate, bubbles were removed, the chemiluminescence value was read, and the cell viability was calculated.

$$\text{Viability}(\%) = \frac{\text{Luminescence value of the experimental group}}{\text{Luminescence value of the control group}} \times 100\%$$

Data were analyzed using Graphpad Prism 5. The results are shown in FIGs. 3A-3C, and the calculated $IC_{50}$ values are shown in Table 4 below. The results showed that both hz28G3-1.1-DDDXd and hz28G3-1.3-DDDXd were able to effectively exert killing activity against CLDN18.2-overexpressing tumor cells.

Table 4. Killing activity of anti-CLDN18.2 antibody-drug conjugates against CLDN18.2-positive tumor cells

| | $IC_{50}$(nM) | | |
|---|---|---|---|
| | NIH-3T3 | BxPC-3 | NCI-N87 |
| hz28G3-1.1-DDDXd | 0.4395 | 0.1246 | 0.03811 |
| hz28G3-1.3-DDDXd | 0.2812 | 0.08861 | 0.03367 |

**Example 10. CDC Activity of Anti-CLDN18.2 Antibody-Drug Conjugates**

NIH-3T3 was selected as target cells, and the CDC activity of the anti-CLDN18.2 antibody-drug conjugates prepared in Example 5 was detected using the human complement (quidel corporation, Cat. No. A112).

Target cells NIH-3T3 were adjusted to a density of $8 \times 10^5$ cells/mL using a cell basal medium containing 0.1% BSA, and seeded into a 96-well plate at 50 μL/well. The anti-CLDN18.2 antibody-drug conjugate subjected to gradient dilution was seeded into the 96-well plate at 50 μL/well. An amount that resulted in a final concentration of 100 nM in the well was used as the initial concentration for the gradient dilution of the anti-CLDN18.2 antibody-drug conjugate, with a 4-fold gradient dilution for a total of 8 concentrations. Human complement was diluted with a cell basal medium containing

0.1% BSA and added to the 96-well plate at 50 μL/well to make the final human complement concentration of 10%. The cell culture was centrifuged at 200 g for 1 min. Then, the 96-well plate was incubated with shaking on a plate shaker at 37 °C for 30 min to allow the complement and the target cells to be thoroughly mixed uniformly and come into contact with each other. Finally, the cells were incubated in an incubator at 37 °C with 5% $CO_2$ for 2 h. After the incubation, the CellTiter-Glo Luminescent Cell Viability Assay (Promega, G7570) was added at 110 μL/well, and the cells were incubated at room temperature for 10 ± 5 min in the dark. The fluorescence value was determined using a microplate reader (Thermo) and the killing rate was calculated. Data were analyzed using GraphPad Prism 5. The results are shown in FIG. 4, and the calculated $IC_{50}$ values are shown in Table 5 below. The results showed that both hz28G3-1.1-DDDXd and hz28G3-1.3-DDDXd were able to induce a relatively strong CDC effect on CLDN18.2-positive tumor cells.

$$\text{Killing rate}(\%) = 1 - \frac{\text{Fluorescence value of the experimental group} - \text{Fluorescence value of the blank control group}}{\text{Fluorescence value of the target cell control group} - \text{Fluorescence value of the blank control group}} \times 100\%$$

Table 5. CDC Activity of anti-CLDN18.2 antibody-drug conjugates against NIH-3T3 cells

| | $IC_{50}$(nM) |
|---|---|
| hz28G3-1.1-DDDXd | ~0.3517 |
| hz28G3-1.3-DDDXd | ~0.3524 |

**Example 11. Non-Specific Killing By Anti-CLDN18.2 Antibody-Drug Conjugates**

To detect the non-specific killing effect of the anti-CLDN18.2 antibody-drug conjugates prepared in Example 5 on CLDN18.2-negative cells, the killing activity detection was performed using CLDN18.1-overexpressing NIH-3T3.

cDNA encoding human CLDN18.1 (SEQ ID NO: 66) was obtained by gene synthesis and then subcloned into an expression vector pcDNA3.1. The expression vector was transfected into NIH-3T3 cells according to the instructions of the Lipofectamine 2000 transfection reagent (Thermo, Cat. No. 11668019) to obtain CLDN18.1-overexpressing NIH-3T3 -CLDN18.1.

NIH-3T3-CLDN18.1 cells in the logarithmic growth phase were taken, adjusted to a cell density of $1 \times 10^4$ cells/mL, and added to a 96-well plate at 100 μL/well. The cells were placed in an incubator at 37 °C with 5% $CO_2$ for adherence culture overnight. The anti-CLDN18.2 antibody-drug conjugate was prepared with a DMEM medium containing 10% FBS. Cells subjected to overnight adherence culture were taken out. For the experimental group, the diluted anti-CLDN18.2 antibody-drug conjugate was added at 50 μL/well, and an amount that resulted in a final concentration of 20 nM in the well was used as the initial concentration for the gradient dilution, with a 3-fold gradient dilution for a total of 9 concentrations; for the control group, a DMEM medium containing 10% FBS was added at 50 μL/well. After culturing for 96 h, the cells were assayed using the CellTiter-Glo Luminescent Cell kit (Promega, Cat. No. G7572). Specifically, the 96-well plate was taken out, the CTG assay buffer (Promega, Cat. No. G7572) was added at 75 μL/well, and the mixture was mixed uniformly by shaking and incubated at room temperature for 10 min in the dark. Then, 180 μL of the solution was pipetted from each well and transferred to an opaque white plate, bubbles were removed, the chemiluminescence value was read, and the viability of the cells was calculated.

$$\text{Viability}(\%) = \frac{\text{Luminescence value of the experimental group}}{\text{Luminescence value of the control group}} \times 100\%$$

Data were analyzed using Graphpad Prism 5. The results are shown in FIG. 5. The results showed that hz28G3-1.1-DDDXd and hz28G3-1.3-DDDXd had no killing activity against NIH-3T3-CLDN18.1 cells.

**Example 12. Bystander Effect of Anti-CLDN18.2 Antibody-Drug Conjugates**

CLDN18.2-overexpressing BxPC-3 was selected as positive cells and U2OS stably expressing luciferase (U2OS-Luciferase) was selected as negative cells. These cells were mixed and cultured to detect the bystander effect of the anti-CLDN18.2 antibody-drug conjugates prepared in Example 5.

Positive cells and negative cells in the logarithmic growth phase were taken, both adjusted to a density of $4 \times 10^4$ cells/mL, and added to a 96-well plate at 50 μL/well. The anti-CLDN18.2 antibody-drug conjugate was subjected to gradient dilution with the McCoy's 5A complete medium (GIBCO, Cat. No. 16600-082) containing 10% FBS and added to the 96-well plate at 50 μL/well. The initial concentration of the anti-CLDN18.2 antibody-drug conjugate is 33.3 nM, with a 5-fold gradient dilution for a total of 8 concentrations. The 96-well plate was incubated in an incubator at 37 °C with 5% $CO_2$ for 120 h. After the incubation, the IL-31 factor (R&D Systems, Cat. No. 2824-IL-010/CF) was added at 20 μL/well for stimulation (final concentration: 10 ng/mL), and the cells were incubated in an incubator at 37 °C with 5% $CO_2$ for 4-6 h. After the incubation, the Luciferase Assay Substrate (Vazyme, Cat. No. DD1201-02) was added at 120 μL/well. The chemiluminescence value was determined using a microplate reader, and the viability of U2OS-Luciferase cells was calculated.

$$\text{Viability}(\%) = \frac{\text{Luminescence value of the experimental group}}{\text{Luminescence value of the control group}} \times 100\%$$

Data were analyzed using GraphPad Prism 5. The results are shown in FIG. 6. The results showed that both hz28G3-1.1-DDDXd and hz28G3-1.3-DDDXd had a significant bystander effect, and had comparable killing activity against negative cells in the existence of positive cells.

**Example 13. Pharmacokinetic Evaluation of Anti-CLDN18.2 Antibody-Drug Conjugates in Rats**

SD rats (160-180 g) (source: Laboratory Animal Operation Department, Shanghai Institute for Biomedical and Pharmaceutical Technologies) were randomly divided into 3 groups of 4 rats each, and were intravenously injected with 15F9-DDDXd, hz28G3-1.1-DDDXd, and hz28G3-1.1-DDDXd prepared in Example 3 at a dose of 10 mg/kg, respectively. Before and after the administration, SD rats were all not fasted and had free access to water.

About 0.2 mL of blood was collected from the jugular vein at 30 min, 2 h (± 1 min), 8 h (± 1 min), 24 h (± 5 min), 48 h (± 5 min), 4 d (± 10 min), 7 d (± 10 min), 10 d (± 10 min), 14 d (± 20 min), 21 d (± 20 min), or 28 d (± 20 min) after the administration, anticoagulated with EDTA-K2, and centrifuged at 4 °C for 10 min at 4000 rpm to separate the plasma.

An appropriate volume of the separated plasma was pipetted and diluted with a sample diluent (PBST containing 1% BSA) at a minimum dilution factor of (20-fold). For a sample that required a higher dilution, it was further diluted with a sample diluent containing 5% blank rat plasma. Then, the mixture was vortexed until it was mixed uniformly to obtain the test sample. The quantitative range of the detection method was 19.53125 ng/mL-2500 ng/mL. AffiniPure Goat Anti-Human IgG, F(ab')2 fragment specific (Jackson ImmunoResearch, 109-005-097) was diluted with the PBS buffer (pH 7.4) to a concentration of 1 μg/mL, added to a 96-well plate at 100 μL/well for coating, and incubated at 4 °C overnight. The 96-well plate was washed with PBST (PBS containing 0.5% of Tween-20). Then, the blocking buffer (PBST containing 1% of BSA) was added at 200 μL/well, and the plate was blocked at room temperature for 2-3 h. The blocking buffer was discarded. The diluted standard curve sample, the quality control sample, and the above test sample were added at 100 μL/well, and the mixture was incubated at room temperature for 2 h. After the 96-well plate was washed with PBST, Goat anti-Human Kappa Light Chain Cross-Adsorbed Secondary Antibody, HRP (Thermofisher, A18859) was added according to a volume ratio of 1:10,000, and the mixture was incubated at room temperature for 1 h. After the 96-well plate was washed with PBST, the TMB solution (Tiangen, Cat. No. PA107-01) was added at 100 μL/well, and the mixture was incubated at room temperature for 14 min in the dark. The reaction was stopped with 1 M $H_2SO_4$. The absorbance value was read using a Tecan Spark microplate reader at a wavelength of 450 nm/630 nm, and curve fitting was performed using a 4-parameter logistic fit.

Intravenous exposure of the anti-CLDN18.2 antibody-drug conjugates was evaluated by *in vivo* pharmacokinetics in rats and the mean pharmacokinetic parameters for total antibodies (including antibodies conjugated and unconjugated with cytotoxic drugs) in SD rats of each group were calculated, including drug peak concentration ($C_{max}$), time to peak ($T_{max}$), area under the concentration-time curve ($AUC_{(0-t)}$), elimination half-life ($t_{1/2}$), mean residence time ($MRT_{(0-t)}$), and clearance (CL), as shown in Table 6 below.

In terms of the exposure, mean retention time, and clearance in rats, hz28G3-1.1-DDDXd and hz28G3-1.3-DDDXd were comparable to the control 15F9-DDDXd. The half-life of hz28G3-1.3-DDDXd was better than that of hz28G3-1.1-DDDXd.

Table 6. Pharmacokinetic parameters for total antibodies (Mean ± SD)

| Pharmacokinetic parameter \ Drug | 15F9-DDDXd | hz28G3-1.3-DDDXd | hz28G3-1.1-DDDXd |
|---|---|---|---|
| $T_{max}$(d) | 0.0364±0 | 0.0208±0 | 0.0208±0 |
| $C_{max}$(μg/mL) | 184±18.9 | 190±8.13 | 193±6.67 |
| $AUC_{(0-21d)}$(d × μg/mL) | 621±50.7 | 578±14.0 | 573±9.4 |
| $t_{1/2}$(d) | 7.3±2.86 | 7.8±0.578 | 6.8±1.49 |
| $MRT_{(0-21d)}$(d) | 5.9±0.482 | 5.6±0.0529 | 5.7±0.258 |
| CL(mL/d/kg) | 14.4±2.35 | 15.4±0.378 | 15.8±0.879 |

**Example 14. Pharmacodynamic Evaluation of Anti-CLDN18.2 Antibody-Drug Conjugates in CB-17 SCID Mouse Xenograft Tumor Model of NUGC-4 Human Gastric Cancer**

NUGC-4 human gastric cancer cells were subcutaneously inoculated in the right axilla of SPF-grade female CB-17 SCID mice (source: Shanghai Lingchang Biotech Co., Ltd.) at 5 × $10^6$/mouse. When the mean tumor volume reached about 150 $mm^3$, the animals were divided into 3 groups of 6 animals each, with the specific groups and doses shown in Table 7. Both hz28G3-1.3-DDDXd and 15F9-DDDXd were prepared in Example 3. After the determination of the DAR value according to Example 4, the DAR value of hz28G3-1.3-DDDXd was 7.4, and the DAR value of 15F9-DDDXd was 7.7.

Table 7. Groups and doses

| Group | Drug | Dose (mg/kg) |
|---|---|---|
| 1 | Vehicle control | N/A |
| 2 | hz28G3-1.3-DDDXd | 10 |
| 3 | 15F9-DDDXd | 10 |

The day of grouping was day 0, and the tail vein administration was performed once on day 2 after grouping. The tumor volume was measured 2-3 times a week, and meanwhile, the mice were weighed and the data were recorded. The general behavior of the mice was observed and recorded every day. After the experiment was completed, the tumors were removed, weighed, and photographed.

The detection indexes and the calculation formulas are as follows:

Tumor volume TV ($mm^3$) = 1/2 × (a × $b^2$), where a represents the long diameter of the tumor, and b represents the short diameter of the tumor;
Relative tumor volume RTV = $TV_t/TV_0$, where $TV_0$ represents the tumor volume on day 0, and $TV_t$ represents the tumor volume at each measurement;
Relative tumor proliferation rate T/C (%) = ($T_{RTV}/C_{RTV}$) × 100%, where $T_{RTV}$ represents RTV of the treatment group, and $C_{RTV}$ represents RTV of the vehicle control group;
Tumor growth inhibition rate TGI (%) = (1 - $TW/TW_0$) × 100%; where TW represents the tumor weight of the treatment group, and $TW_0$ represents the tumor weight of the vehicle control group;
Weight change rate WCR (%) = (Wtt - $Wt_0$)/$Wt_0$ × 100%, where $Wt_0$ represents the body weight of the mouse on day 0, and Wtt represents the body weight of the mouse at each measurement.

The results of the detection indexes on day 33 are shown in Table 8 below. hz28G3-1.3-DDDXd had no significant toxicity, and had inhibitory activity against mouse xenograft tumor of NUGC-4 human gastric cancer, which was comparable to that of the control 15F9-DDDXd.

Table 8. Influence of anti-CLDN18.2 antibody-drug conjugates on the indexes in mouse subcutaneous xenograft tumor model of NUGC-4 human gastric cancer

| Group | TV($mm^3$) (mean ±SD) | RTV (mean ±SD) | T/C (%) | TGI(%) | Weight change rate (%) (mean ± SD) |
|---|---|---|---|---|---|
| Vehicle control | 1740±522 | 10.1±2.3 | N/A | N/A | 9.1±3.0 |
| hz28G3-1.3-DDDXd (10 mg/kg) | 260±131 | 1.5±0.6** | 14.7% | 86.7% | 11.1±2.7 |
| 15F9-DDDXd (10 mg/kg) | 293±84 | 1.7±0.4** | 16.8% | 82.1% | 10.6±4.8 |
| Note: comparison with the vehicle control group, where ** denotes p < 0.01. | | | | | |

**Example 15. Pharmacodynamic Evaluation of Anti-CLDN18.2 Antibody-Drug Conjugates in CD-17 SCID Mouse Xenograft Tumor Model of BxPC-3 Human Pancreatic Cancer**

CLDN18.2 highly-expressing BxPC-3 human pancreatic cancer tumor blocks were subcutaneously inoculated by block implantation into the right axilla of SPF-grade female CB-17 SCID mice (source: Shanghai Slac Laboratory Animal Co., Ltd.). When the mean tumor volume reached about 200 $mm^3$, the animals were divided into 3 groups of 4 animals each, with the specific groups and doses shown in Table 9. Both hz28G3-1.3-DDDXd and 15F9-DDDXd were prepared in Example 3. After the determination of the DAR value according to Example 4, the DAR value of hz28G3-1.3-DDDXd was 7.4, and the DAR value of 15F9-DDDXd was 7.7.

Table 9. Groups and doses

| Group | Dose (mg/kg) |
|---|---|
| Vehicle control | N/A |
| hz28G3-1.3-DDDXd | 5 |
| 15F9-DDDXd | 5 |

The day of grouping was day 0, and the tail vein administration was performed once on the day of grouping. The tumor volume was measured 2-3 times a week, and meanwhile, the mice were weighed and the data were recorded. The general behavior of the mice was observed and recorded every day. After the experiment was completed, the tumors were removed, weighed, and photographed.

The detection indexes and the calculation formulas are as follows:

Tumor volume TV ($mm^3$) = 1/2 × (a × $b^2$), where a represents the long diameter of the tumor, and b represents the short diameter of the tumor;
Relative tumor volume RTV = $TV_t/TV_0$, where $TV_0$ represents the tumor volume on day 0, and $TV_t$ represents the tumor volume at each measurement;
Relative tumor proliferation rate T/C (%) = ($T_{RTV}/C_{RTV}$) × 100%, where $T_{RTV}$ represents RTV of the treatment group, and $C_{RTV}$ represents RTV of the vehicle control group;
Tumor growth inhibition rate TGI (%) = (1 - $TW/TW_0$) × 100%; where TW represents the tumor weight of the treatment group, and TWo represents the tumor weight of the vehicle control group;
Weight change rate WCR (%) = (Wtt - $Wt_0$)/$Wt_0$ × 100%, wherein Wto is the body weight of the animal on day 0, and Wtt is the body weight of the animal at each measurement.

The detection results of indexes on day 21 are shown in Table 10 below. hz28G3-1.3-DDDXd had no significant toxicity, and had a significant inhibition effect on CLDN18.2-highly-expressing CB-17 SCID mouse xenograft tumor of BxPC-3 human pancreatic cancer, which was superior to that of the control 15F9-DDDXd.

Table 10. Influence of anti-CLDN18.2 antibody-drug conjugates on the indexes in mouse subcutaneous xenograft tumor model of BxPC-3 human pancreatic cancer

| Group | TV(mm3) (mean ±SD) | RTV (mean ±SD) | T/C(%) | TGI(%) | Weight change rate (%) (mean ± SD) |
|---|---|---|---|---|---|
| Vehicle control | 1779±457 | 7.9±1.8 | N/A | N/A | 3.6±3.5 |

(continued)

| Group | TV(mm3) (mean ±SD) | RTV (mean ±SD) | T/C(%) | TGI(%) | Weight change rate (%) (mean ± SD) |
|---|---|---|---|---|---|
| hz28G3-1.3-DDDXd | 694±572 | 2.7±1.1** | 33.8% | 69.7% | 4.8±5.2 |
| 15F9-DDDXd | 847±602 | 3.4±1.0** | 43.4% | 57.2% | 6.1±1.7 |
| Note: comparison with the vehicle control group, where ** denotes $p < 0.01$. | | | | | |

**Example 16. Preparation of Anti-CLDN18.2 Antibody-Drug Conjugates**

**Reagents:**

Antibody: the anti-CLDN18.2 antibody hz28G3-1.3 prepared in Example 1; and
linker-payload: compound H (MC-GGFG-DDDXd) prepared in Example 2.

**Experimental procedures:**

1. Reduction of the antibodies: The antibodies were buffer-exchanged into a histidine buffer (1.43 mg/mL L-histidine and 2.27 mg/mL L-histidine hydrochloride monohydrate) at pH 6.0, the antibody concentration was adjusted to 10 mg/mL with the histidine buffer (pH 6.0), and the pH was adjusted to 6.3. A stock solution of disodium ethylenediaminetetraacetate ($EDTA \cdot Na_2$) was added to make the final concentration of 5 mM. The solution was added to a light-protected glass bottle, and when the temperature of the solution was increased to 30 °C, a 10 mM TCEP solution was added to enable the molar ratio of the antibody to TCEP to be 1:8. The mixture was incubated at 30 °C for 100 min in the dark with stirring, so as to reduce the inter-chain disulfide bonds of the antibody to obtain reaction solution 1.
2. Conjugation of the antibodies with the linker-payload: After the temperature of reaction solution 1 was decreased to 22 °C, a DMSO solution was added (DMSO solution:reaction solution 1 (v/v) = 1:20), and the mixture was mixed uniformly. A linker-payload solution at a concentration of 10 mg/mL, which was dissolved with DMSO, was added to enable the molar ratio of the antibody to the linker-payload to be 1:9.5. The mixture was incubated at 22 °C for 2 h in the dark with gentle stirring, so as to conjugate the antibody with the linker-payload to obtain reaction solution 2.
3. Coupling termination and purification: Reaction solution 2 was ultrafiltered with a histidine buffer at pH 6.0 to obtain the anti-CLDN18.2 antibody-drug conjugate hz28G3-1.3-DDDXd.

The structure of the prepared hz28G3-1.3-DDDXd is as follows:

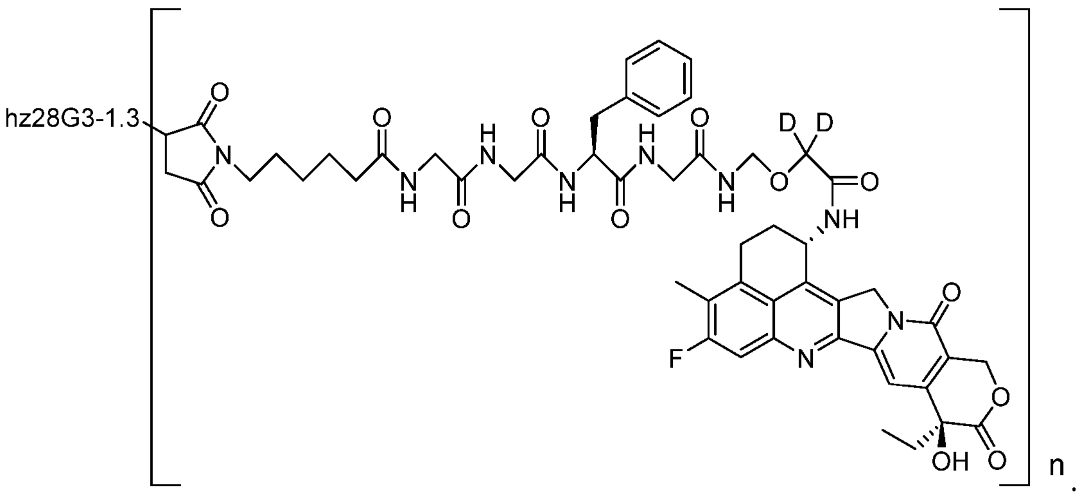

Its DAR value is determined to be 7.93. D8 accounts for 97.04%, and D0 to D7 account for 2.96%, which are based on the molar quantity.

The sequence information of the present disclosure is summarized in Table S2 below.

Table S2 Sequence information

| Description<br>Sequence/SEQ ID NO: |
|---|
| Heavy chain constant region of chimeric and humanized antibodies<br>ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK (SEQ ID NO: 1) |
| Light chain constant region of chimeric and humanized antibodies<br>RTVAAPSVFIFPPSDEQLKSGTASVVCLLNNFYPREAKVQWKVDNALQSGNSQESVTEQDSKDSTYSLSS TLTLSKADYEKHKVYACEVTHQGLSSPVTKSFNRGEC (SEQ ID NO: 2) |
| HCDR1 of mouse, chimeric, and humanized 28G3 |
| DYLML (SEQ ID NO: 3) |
| HCDR2 of mouse and chimeric 28G3 NINPYYGRTTYNLKFKG (SEQ ID NO: 4) |
| HCDR2 of hz28G3-1.1, hz28G3-1.3, hz28G3-2.1, and hz28G3-2.3 NINPYYGRTTYALKFKG (SEQ ID NO: 5) |
| HCDR2 of hz28G3-1.2, hz28G3-1.4, hz28G3-2.2, and hz28G3-2.4 NINPYYGRTYYALKFQG (SEQ ID NO: 6) |
| HCDR3 of mouse, chimeric, and humanized 28G3 DGYSLRNAMDY (SEQ ID NO: 7) |
| LCDR1 of mouse, chimeric, and humanized 28G3 KSSQSLFNSGNQKNYLA (SEQ ID NO: 8) |
| LCDR2 of mouse, chimeric, and humanized 28G3 GASTRES (SEQ ID NO: 9) |
| LCDR3 of mouse, chimeric, and humanized 28G3 QNAHSYPYT (SEQ ID NO: 10) |
| VH of mouse and chimeric 28G3:<br>EIQLQQTGPELVKPGASVTISCKTSGYSFTDYLMLWVKQSHGKSLEWIGNINPYYGRTTYNLKFKGKATL TVDRSSSTAYMQLNSLTSEDSAVYYCARDGYSLRNAMDYWGQGTSVTVSS (SEQ ID NO: 11) |
| VH of hz28G3-1.1 and hz28G3-2.1:<br>QVQLVQSGAEVKKPGASVKVSCKTSGYSFTDYLMLWVRQAPGQGLEWMGNINPYYGRTTYALKFKGR VTMTVDRSTSTVYMELSSLRSEDTAVYYCARDGYSLRNAMDYWGQGTLVTVSS (SEQ ID NO: 12) |
| VH of hz28G3-1.2 and hz28G3-2.2:<br>QVQLVQSGAEVKKPGASVKVSCKASGYTFTDYLMLWVRQAPGQGLEWMGNINPYYGRTYYALKFQGR VTMTVDRSTSTVYMELSSLRSEDTAVYYCARDGYSLRNAMDYWGQGTLVTVSS (SEQ ID NO: 13) |
| VH of hz28G3-1.3 and hz28G3-2.3:<br>QVQLVQSGAEVKKPGASVKVSCKTSGYSFTDYLMLWVRQAHGQSLEWMGNINPYYGRTTYALKFKGR VTMTVDRSTSTVYMELSSLRSEDTAVYYCARDGYSLRNAMDYWGQGTLVTVSS (SEQ ID NO: 14) |
| VH of hz28G3-1.4 and hz28G3-2.4:<br>QVQLVQSGAEVKKPGASVKVSCKASGYTFTDYLMLWVRQAHGQSLEWMGNINPYYGRTYYALKFQGR VTMTVDRSTSTVYMELSSLRSEDTAVYYCARDGYSLRNAMDYWGQGTLVTVSS (SEQ ID NO: 15) |
| VL of mouse and chimeric 28G3:<br>DIVMTQSPYSLSVSTGEKVTMSCKSSQSLFNSGNQKNYLAWYQQKPGQPPKLLIYGASTRESGVPDRFTG SGSGTDFTLTISSVQAEDLALYYCQNAHSYPYTFGGGTKLEIK (SEQ ID NO: 16) |
| VL of hz28G3-1.1, hz28G3-1.2, hz28G3-1.3, and hz28G3-1.4:<br>DIVMTQSPDSLAVSLGERATINCKSSQSLFNSGNQKNYLAWYQQKPGQPPKLLIYGASTRESGVPDRFSGS GSGTDFTLTISSLQAEDVAVYYCQNAHSYPYTFGQGTKVEIK (SEQ ID NO: 17) |

(continued)

| Description<br>Sequence/SEQ ID NO: |
|---|
| VL of hz28G3-2.1, hz28G3-2.2, hz28G3-2.3, and hz28G3-2.4:<br>DIVMTQSPYSLAVSLGERATINCKSSQSLFNSGNQKNYLAWYQQKPGQPPKLLIYGASTRESGVPDRFTGS GSGTDFTLTISSLQAEDVAVYYCQNAHSYPYTFGQGTKVEIK (SEQ ID NO: 18) |
| HCDR1 of mouse, chimeric, and humanized 40F6 SYGVY (SEQ ID NO: 19) |
| HCDR2 of mouse and chimeric 40F6, hz40F6-1.2, and hz40F6-2.2 VVWAGGNTNYNSALMS (SEQ ID NO: 20) |
| HCDR2 of hz40F6-1.1 and hz40F6-2.1 VVWAGGNTNYADSVKG (SEQ ID NO: 21) |
| HCDR2 of hz40F6-1.3 and hz40F6-2.3 VVWAGGNTNYADALKG (SEQ ID NO: 22 |
| HCDR2 of hz40F6-1.4 and hz40F6-2.4 VVWAGGNTNYNSALKG (SEQ ID NO: 23) |
| HCDR3 of mouse, chimeric, and humanized 40F6 DRGRLLAMDY (SEQ ID NO: 24) |
| LCDR1 of mouse, chimeric, and humanized 40F6 KSSQSLLNSGNQKNYLA (SEQ ID NO: 25) |
| LCDR2 of mouse, chimeric, and humanized 40F6 GASTRES (SEQ ID NO: 26) |
| LCDR3 of mouse and chimeric 40F6, hz40F6-1.1, hz40F6-1.2, hz40F6-1.3, and hz40F6-1.4 QNDHFFPFT (SEQ ID NO: 27) |
| LCDR3 of hz40F6-2.1, hz40F6-2.2, hz40F6-2.3, and hz40F6-2.4 QNDYFFPFT (SEQ ID NO: 28) |
| VH of mouse and chimeric 40F6:<br>QVQLKESGPGLVAPSQSLSITCTVSGFSLTSYGVYWVRQSPGKGLEWLGVVWAGGNTNYNSALMSRLSIS KDNSKSQVFLKMNSLQTDDTAMYYCARDRGRLLAMDYWGQGTSVTVSS (SEQ ID NO: 29) |
| VH of hz40F6-1.1 and hz40F6-2.1:<br>QVQLVESGGGVVQPGRSLRLSCAASGFTLTSYGVYWVRQAPGKGLEWVAVVWAGGNTNYADSVKGRF TISKDNSKSTLYLQMNSLRAEDTAVYYCARDRGRLLAMDYWGQGTLVTVSS (SEQ ID NO: 30) |
| VH of hz40F6-1.2 and hz40F6-2.2:<br>QVQLVESGGGVVQPGRSLRLSCAASGFTLTSYGVYWVRQAPGKGLEWVAVVWAGGNTNYNSALMSRF TISKDNSKSTLYLQMNSLRAEDTAVYYCARDRGRLLAMDYWGQGTLVTVSS (SEQ ID NO: 31) |
| VH of hz40F6-1.3 and hz40F6-2.3:<br>QVQLVESGGGVVQPGRSLRLSCAASGFTLTSYGVYWVRQAPGKGLEWVAVVWAGGNTNYADALKGRF TISKDNSKSTLYLQMNSLRAEDTAVYYCARDRGRLLAMDYWGQGTLVTVSS (SEQ ID NO: 32) |
| VH of hz40F6-1.4 and hz40F6-2.4:<br>QVQLVESGGGVVQPGRSLRLSCTVSGFTLTSYGVYWVRQAPGKGLEWVAVVWAGGNTNYNSALKGRFT ISKDNSKSTLYLQMNSLRAEDTAVYYCARDRGRLLAMDYWGQGTLVTVSS (SEQ ID NO: 33) |
| VL of mouse and chimeric 40F6:<br>DVVMTQSPSSLSVSAGEKVTMSCKSSQSLLNSGNQKNYLAWYQQKPGQPPKLLIYGASTRESGVPDRFT GSGSGTDFTLTISSVQAEDLAVYYCQNDHFFPFTFGSGTKLEIK (SEQ ID NO: 34) |
| VL of hz40F6-1.1, hz40F6-1.2, hz40F6-1.3, and hz40F6-1.4:<br>DIVMTQSPDSLAVSLGERATINCKSSQSLLNSGNQKNYLAWYQQKPGQPPKLLIYGASTRESGVPDRFTGS GSGTDFTLTISSLQAEDVAVYYCQNDHFFPFTFGQGTKVEIK (SEQ ID NO: 35) |
| VL of hz40F6-2.1, hz40F6-2.2, hz40F6-2.3, and hz40F6-2.4:<br>DIVMTQSPDSLAVSLGERATINCKSSQSLLNSGNQKNYLAWYQQKPGQPPKLLIYGASTRESGVPDRFSGS GSGTDFTLTISSLQAEDVAVYYCQNDYFFPFTFGQGTKVEIK (SEQ ID NO: 36) |
| HCDR1 of mouse and chimeric 34G6 SYGVH (SEQ ID NO: 37) |
| HCDR2 of mouse and chimeric 34G6 VIWSDGSTTYNSALKS (SEQ ID NO: 38) |
| HCDR3 of mouse and chimeric 34G6 HAYYGNSFDY (SEQ ID NO: 39) |

(continued)

| Description<br>Sequence/SEQ ID NO: |
|---|
| LCDR1 of mouse and chimeric 34G6 KSSQSLLNSGNQKSYLT (SEQ ID NO: 40) |
| LCDR2 of mouse and chimeric 34G6 WASTRES (SEQ ID NO: 41) |
| LCDR3 of mouse and chimeric 34G6 QNVYIFPLT (SEQ ID NO: 42) |
| VH of mouse and chimeric 34G6:<br>QVQLKESGPDLVAPSQSLSITCTVSGFSLTSYGVHWVRQPPGKGLEWLVVIWSDGSTTYNSALKSRLSISK DNSKSQVFLKMNSLQTDDTAIYYCARHAYYGNSFDYWGQGTTLTVSS (SEQ ID NO: 43) |
| VL of mouse and chimeric 34G6:<br>DIVMTQSPSSLTVTAGEKVTMSCKSSQSLLNSGNQKSYLTWYQQKPGQPPKLLIYWASTRESGVPDRFTG SGSGTDFTLTISSVQAEDLAVYYCQNVYIFPLTFGAGTKLELK (SEQ ID NO: 44) |
| HCDR1 of mouse and chimeric 10D8 NYGMN (SEQ ID NO: 45) |
| HCDR2 of mouse and chimeric 10D8 WININTGEPRYAEEFKG (SEQ ID NO: 46) |
| HCDR3 of mouse and chimeric 10D8 |
| YGYGNSFPY (SEQ ID NO: 47) |
| LCDR1 of mouse and chimeric 10D8 KSSOSLLNSGNORNYLT (SEQ ID NO: 48) |
| LCDR2 of mouse and chimeric 10D8 WASTRES (SEQ ID NO: 49) |
| LCDR3 of mouse and chimeric 10D8 QNDYSYPLT (SEQ ID NO: 50) |
| VH of mouse and chimeric 10D8:<br>QIQLAQSGPELKKPGETVKVSCKASGYTFKNYGMNWVKQAPGKGLKWMGWININTGEPRYAEEFKGRI AFSLETSASTAYLLINNLKNEDTATYFCARYGYGNSFPYWGQGTLVTVSA (SEQ ID NO: 51) |
| VL of mouse and chimeric 10D8:<br>DIVMTQSPSSLTVTVGEKVTMSCKSSQSLLNSGNQRNYLTWYQQKPGQPPKLLIYWASTRESGVPDRFTG SGSGTAFTLTVSSVQAEDLAVYYCQNDYSYPLTFGAGTKLEMK (SEQ ID NO: 52) |
| HCDR1 of mouse and chimeric 22F12 SYGVY (SEQ ID NO: 53) |
| HCDR2 of mouse and chimeric 22F12 VIWAGGSTNYNSALMS (SEQ ID NO: 54) |
| HCDR3 of mouse and chimeric 22F12 DRGRLLSMDY (SEQ ID NO: 55) |
| LCDR1 of mouse and chimeric 22F12 RSSQSLLNSGNQKNYLA (SEQ 1D NO: 56) |
| LCDR2 of mouse and chimeric 22F12 GASTRES (SEQ ID NO: 57) |
| LCDR3 of mouse and chimeric 22F12 QNVHFFPFT (SEQ ID NO: 58) |
| VH of mouse and chimeric 22F12:<br>QVQLKESGPGLVAPSQSLSITCTVSGFSLTSYGVYWVRQPPGKGLEWLGVIWAGGSTNYNSALMSRLSIN KDNSKSQVFLKMNSLQTDDTAMYYCARDRGRLLSMDYWGQGTSVTVSS (SEQ ID NO: 59) |
| VL of mouse and chimeric 22F12:<br>DIVMTQSPSSLSVSAGEKVTMSCRSSQSLLNSGNQKNYLAWYQQKPGQPPKLLIYGASTRESGVPDRFTG SGSGTDFTLTIGSVQAEDLAVYYCQNVHFFPFTFGSGTKLEIK (SEQ ID NO: 60) |
| Heavy chain of hz28G3-1.1:<br>QVQLVQSGAEVKKPGASVKVSCKTSGYSFTDYLMLWVRQAPGQGLEWMGNINPYYGRTTYALKFKGR VTMTVDRSTSTVYMELSSLRSEDTAVYYCARDGYSLRNAMDYWGQGTLVTVSSASTKGPSVFPLAPSSK STSGGTAALGCLVKDYFPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 61) |

(continued)

| Description<br>Sequence/SEQ ID NO: |
|---|
| chain of hz28G3-1.1:<br>DIVMTQSPDSLAVSLGERATINCKSSQSLFNSGNQKNYLAWYQQKPGQPPKLLIYGASTRESGVPDRFSGS<br>GSGTDFTLTISSLQAEDVAVYYCQNAHSYPYTFGQGTKVEIKRTVAAPSVFIFPPSDEQLKSGTASVVCLLN<br>NFYPREAKVQWKVDNALQSGNSQESVTEQDSKDSTYSLSSTLTLSKADYEKHKVYACEVTHQGLSSPVT<br>KSFNRGEC (SEQ ID NO: 62)<br>Light |
| Heavy chain of hz28G3-1.3:<br>QVQLVQSGAEVKKPGASVKVSCKTSGYSFTDYLMLWVRQAHGQSLEWMGNINPYYGRTTYALKFKGR<br>VTMTVDRSTSTVYMELSSLRSEDTAVYYCARDGYSLRNAMDYWGQGTLVTVSSASTKGPSVFPLAPSSK<br>STSGGTAALGCLVKDYFPEPVTVSWNSGALTSGVHTFPAVLQSSGLYSLSSVVTVPSSSLGTQTYICNVNH<br>KPSNTKVDKKVEPKSCDKTHTCPPCPAPELLGGPSVFLFPPKPKDTLMISRTPEVTCVVVDVSHEDPEVKF<br>NWYVDGVEVHNAKTKPREEQYNSTYRVVSVLTVLHQDWLNGKEYKCKVSNKALPAPIEKTISKAKGQP<br>REPQVYTLPPSRDELTKNQVSLTCLVKGFYPSDIAVEWESNGQPENNYKTTPPVLDSDGSFFLYSKLTVDK<br>SRWQQGNVFSCSVMHEALHNHYTQKSLSLSPGK (SEQ ID NO: 63) |
| Light chain of hz28G3-1.3:<br>DIVMTQSPDSLAVSLGERATINCKSSQSLFNSGNQKNYLAWYQQKPGQPPKLLIYGASTRESGVPDRFSGS<br>GSGTDFTLTISSLQAEDVAVYYCQNAHSYPYTFGQGTKVEIKRTVAAPSVFIFPPSDEQLKSGTASVVCLLN |
| NFYPREAKVQWKVDNALQSGNSQESVTEQDSKDSTYSLSSTLTLSKADYEKHKVYACEVTHQGLSSPVT<br>KSFNRGEC (SEQ ID NO: 64) |
| Human CLDN18.2:<br>MAVTACQGLGFVVSLIGIAGIIAATCMDQWSTQDLYNNPVTAVFNYQGLWRSCVRESSGFTECRGYFTLL<br>GLPAMLQAVRALMIVGIVLGAIGLLVSIFALKCIRIGSMEDSAKANMTLTSGIMFIVSGLCAIAGVSVFANM<br>LVTNFWMSTANMYTGMGGMVQTVQTRYTFGAALFVGWVAGGLTLIGGVMMCIACRGLAPEETNYKAV<br>SYHASGHSVAYKPGGFKASTGFGSNTKNKKIYDGGARTEDEVQSYPSKHDYV (SEQ ID NO: 65) |
| Human CLDN18.1:<br>MSTTTCQVVAFLLSILGLAGCIAATGMDMWSTQDLYDNPVTSVFQYEGLWRSCVRQSSGFTECRPYFTIL<br>GLPAMLQAVRALMIVGIVLGAIGLLVSIFALKCIRIGSMEDSAKANMTLTSGIMFIVSGLCAIAGVSVFANM<br>LVTNFWMSTANMYTGMGGMVQTVQTRYTFGAALFVGWVAGGLTLIGGVMMCIACRGLAPEETNYKAV<br>SYHASGHSVAYKPGGFKASTGFGSNTKNKKIYDGGARTEDEVQSYPSKHDYV (SEQ ID NO: 66) |
| Heavy chain of 15F9:<br>QVQLVQSGSELKKPGASVKVSCKASGYTFTNYGINWVRQAPGQGLEWMGWINPNTGETTYAEEFKGRF<br>VFSLDTSVSTAYLQISSLKAEDTAVYFCARLYYGNRFDSWGQGTLVTVSSASTKGPSVFPLAPSSKSTSGGT<br>AALGCLVKDYFPEPVTVSWNSGALTSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTPEVTCVVVDVSHEDPEVKFNWYVD<br>GVEVHNAKTKPREEQYNSTYRVVSVLTVLHQDWLNGKEYKCKVSNKALPAPIEKTISKAKGQPREPQVY<br>TLPPSRDELTKNQVSLTCLVKGFYPSDIAVEWESNGQPENNYKTTPPVLDSDGSFFLYSKLTVDKSRWQQG<br>NVFSCSVMHEALHNHYTQKSLSLSPGK (SEQ ID NO: 67) |
| Light chain of 15F9:<br>DIVMTQSPDSLAVSLGERATINCKSSQSLLNSGNQRNYLTWYQQKPGQPPKLLIYWASTRESGVPDRFSGS<br>GSGTDFTLTISSLQAEDVAVYYCQNAYYYPLTFGGGTKVEIKRTVAAPSVFIFPPSDEQLKSGTASVVCLLN<br>NFYPREAKVQWKVDNALQSGNSQESVTEQDSKDSTYSLSSTLTLSKADYEKHKVYACEVTHQGLSSPVT<br>KSFNRGEC (SEQ ID NO: 68)<br>HCDR2 of mouse, chimeric, and humanized 28G3<br>NINPYYGRT$X_1$Y$X_2$LKF$X_3$G (SEQ ID NO: 69), wherein $X_1$ = T or Y; $X_2$ = N or A; $X_3$ = K or Q |
| HCDR2 of mouse, chimeric, and humanized 40F6<br>VVWAGGNTNY$X_4X_5X_6X_7X_8X_9$ (SEQ ID NO: 70), wherein $X_4$ = N or A; $X_5$ = S or D; $X_6$ = A or S; $X_7$ = L or V; $X_8$ = M or K; $X_9$ = S or G |

(continued)

| Description<br>Sequence/SEQ ID NO: |
| --- |
| LCDR3 of mouse, chimeric, and humanized 40F6<br>QNDX$_{10}$FFPFT (SEQ ID NO: 71), wherein X$_{10}$ = H or Y |

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

## Claims

1. An antibody-drug conjugate of general formula Ab-(L-U)$_n$ or a pharmaceutically acceptable salt or a solvate thereof, wherein Ab is an antibody or antigen-binding fragment thereof, L is a linker, U is a cytotoxic drug, and n is an integer selected from 1-10, wherein Ab is an anti-CLDN18.2 antibody or an antigen-binding fragment thereof comprising HCDR1, HCDR2, HCDR3, LCDR1, LCDR2, and LCDR3, wherein

    (1) the HCDR1, the HCDR2, the HCDR3, the LCDR1, the LCDR2, and the LCDR3 comprise amino acid sequences set forth in SEQ ID NOs: 3, 5, 7, 8, 9, and 10, respectively;
    (2) the HCDR1, the HCDR2, the HCDR3, the LCDR1, the LCDR2, and the LCDR3 comprise amino acid sequences set forth in SEQ ID NOs: 3, 4, 7, 8, 9, and 10, respectively;
    (3) the HCDR1, the HCDR2, the HCDR3, the LCDR1, the LCDR2, and the LCDR3 comprise amino acid sequences set forth in SEQ ID NOs: 3, 6, 7, 8, 9, and 10, respectively;
    (4) the HCDR1, the HCDR2, the HCDR3, the LCDR1, the LCDR2, and the LCDR3 comprise amino acid sequences set forth in SEQ ID NOs: 19, 20, 24, 25, 26, and 27, respectively;
    (5) the HCDR1, the HCDR2, the HCDR3, the LCDR1, the LCDR2, and the LCDR3 comprise amino acid sequences set forth in SEQ ID NOs: 19, 21, 24, 25, 26, and 27, respectively;
    (6) the HCDR1, the HCDR2, the HCDR3, the LCDR1, the LCDR2, and the LCDR3 comprise amino acid sequences set forth in SEQ ID NOs: 19, 22, 24, 25, 26, and 27, respectively;
    (7) the HCDR1, the HCDR2, the HCDR3, the LCDR1, the LCDR2, and the LCDR3 comprise amino acid sequences set forth in SEQ ID NOs: 19, 23, 24, 25, 26, and 27, respectively;
    (8) the HCDR1, the HCDR2, the HCDR3, the LCDR1, the LCDR2, and the LCDR3 comprise amino acid sequences set forth in SEQ ID NOs: 19, 20, 24, 25, 26, and 28, respectively;
    (9) the HCDR1, the HCDR2, the HCDR3, the LCDR1, the LCDR2, and the LCDR3 comprise amino acid sequences set forth in SEQ ID NOs: 19, 21, 24, 25, 26, and 28, respectively;
    (10) the HCDR1, the HCDR2, the HCDR3, the LCDR1, the LCDR2, and the LCDR3 comprise amino acid sequences set forth in SEQ ID NOs: 19, 22, 24, 25, 26, and 28, respectively;
    (11) the HCDR1, the HCDR2, the HCDR3, the LCDR1, the LCDR2, and the LCDR3 comprise amino acid sequences set forth in SEQ ID NOs: 19, 23, 24, 25, 26, and 28, respectively;
    (12) the HCDR1, the HCDR2, the HCDR3, the LCDR1, the LCDR2, and the LCDR3 comprise amino acid sequences set forth in SEQ ID NOs: 37, 38, 39, 40, 41, and 42, respectively;
    (13) the HCDR1, the HCDR2, the HCDR3, the LCDR1, the LCDR2, and the LCDR3 comprise amino acid sequences set forth in SEQ ID NOs: 45, 46, 47, 48, 49, and 50, respectively; or
    (14) the HCDR1, the HCDR2, the HCDR3, the LCDR1, the LCDR2, and the LCDR3 comprise amino acid sequences set forth in SEQ ID NOs: 53, 54, 55, 56, 57, and 58, respectively.

2. The antibody-drug conjugate or the pharmaceutically acceptable salt or the solvate thereof according to claim 1,

wherein the anti-CLDN18.2 antibody or the antigen-binding fragment thereof comprises a heavy chain variable region and a light chain variable region, wherein

(1) the heavy chain variable region and the light chain variable region comprise amino acid sequences having at least 80% identity to amino acid sequences set forth in SEQ ID NOs: 14 and 17, respectively;
(2) the heavy chain variable region and the light chain variable region comprise amino acid sequences having at least 80% identity to amino acid sequences set forth in SEQ ID NOs: 12 and 17, respectively;
(3) the heavy chain variable region and the light chain variable region comprise amino acid sequences having at least 80% identity to amino acid sequences set forth in SEQ ID NOs: 13 and 17, respectively;
(4) the heavy chain variable region and the light chain variable region comprise amino acid sequences having at least 80% identity to amino acid sequences set forth in SEQ ID NOs: 11 and 16, respectively;
(5) the heavy chain variable region and the light chain variable region comprise amino acid sequences having at least 80% identity to amino acid sequences set forth in SEQ ID NOs: 15 and 17, respectively;
(6) the heavy chain variable region and the light chain variable region comprise amino acid sequences having at least 80% identity to amino acid sequences set forth in SEQ ID NOs: 12 and 18, respectively;
(7) the heavy chain variable region and the light chain variable region comprise amino acid sequences having at least 80% identity to amino acid sequences set forth in SEQ ID NOs: 13 and 18, respectively;
(8) the heavy chain variable region and the light chain variable region comprise amino acid sequences having at least 80% identity to amino acid sequences set forth in SEQ ID NOs: 14 and 18, respectively;
(9) the heavy chain variable region and the light chain variable region comprise amino acid sequences having at least 80% identity to amino acid sequences set forth in SEQ ID NOs: 15 and 18, respectively;
(10) the heavy chain variable region and the light chain variable region comprise amino acid sequences having at least 80% identity to amino acid sequences set forth in SEQ ID NOs: 29 and 34, respectively;
(11) the heavy chain variable region and the light chain variable region comprise amino acid sequences having at least 80% identity to amino acid sequences set forth in SEQ ID NOs: 30 and 35, respectively;
(12) the heavy chain variable region and the light chain variable region comprise amino acid sequences having at least 80% identity to amino acid sequences set forth in SEQ ID NOs: 31 and 35, respectively;
(13) the heavy chain variable region and the light chain variable region comprise amino acid sequences having at least 80% identity to amino acid sequences set forth in SEQ ID NOs: 32 and 35, respectively;
(14) the heavy chain variable region and the light chain variable region comprise amino acid sequences having at least 80% identity to amino acid sequences set forth in SEQ ID NOs: 33 and 35, respectively;
(15) the heavy chain variable region and the light chain variable region comprise amino acid sequences having at least 80% identity to amino acid sequences set forth in SEQ ID NOs: 30 and 36, respectively;
(16) the heavy chain variable region and the light chain variable region comprise amino acid sequences having at least 80% identity to amino acid sequences set forth in SEQ ID NOs: 31 and 36, respectively;
(17) the heavy chain variable region and the light chain variable region comprise amino acid sequences having at least 80% identity to amino acid sequences set forth in SEQ ID NOs: 32 and 36, respectively;
(18) the heavy chain variable region and the light chain variable region comprise amino acid sequences having at least 80% identity to amino acid sequences set forth in SEQ ID NOs: 33 and 36, respectively;
(19) the heavy chain variable region and the light chain variable region comprise amino acid sequences having at least 80% identity to amino acid sequences set forth in SEQ ID NOs: 43 and 44, respectively;
(20) the heavy chain variable region and the light chain variable region comprise amino acid sequences having at least 80% identity to amino acid sequences set forth in SEQ ID NOs: 51 and 52, respectively; or
(21) the heavy chain variable region and the light chain variable region comprise amino acid sequences having at least 80% identity to amino acid sequences set forth in SEQ ID NOs: 59 and 60, respectively.

**3.** The antibody-drug conjugate or the pharmaceutically acceptable salt or the solvate thereof according to claim 1 or 2, wherein the anti-CLDN18.2 antibody or the antigen-binding fragment thereof is of an IgG1, IgG2, or IgG4 isotype.

**4.** The antibody-drug conjugate or the pharmaceutically acceptable salt or the solvate thereof according to any one of claims 1-3, wherein the anti-CLDN18.2 antibody further comprises a heavy chain constant region and a light chain constant region, wherein the heavy chain constant region comprises an amino acid sequence set forth in SEQ ID NO: 1 or an amino acid sequence having 1, 2, 3, 4, or 5 amino acid substitutions, deletions, and additions compared to the amino acid sequence set forth in SEQ ID NO: 1, and the light chain constant region comprises an amino acid sequence set forth in SEQ ID NO: 2 or an amino acid sequence having 1, 2, 3, 4, or 5 amino acid substitutions, deletions, and additions compared to the amino acid sequence set forth in SEQ ID NO: 2.

**5.** The antibody-drug conjugate or the pharmaceutically acceptable salt or the solvate thereof according to any one of

claims 1-3, wherein the anti-CLDN18.2 antibody comprises a heavy chain and a light chain, wherein

(1) the heavy chain comprises an amino acid sequence having at least 80% identity to an amino acid sequence set forth in SEQ ID NO: 63 and the light chain comprises an amino acid sequence having at least 80% identity to an amino acid sequence set forth in SEQ ID NO: 64; or
(2) the heavy chain comprises an amino acid sequence having at least 80% identity to an amino acid sequence set forth in SEQ ID NO: 61 and the light chain comprises an amino acid sequence having at least 80% identity to an amino acid sequence set forth in SEQ ID NO: 62.

**6.** The antibody-drug conjugate, or the pharmaceutically acceptable salt or the solvate thereof according to any one of claims 1-5, wherein U is a camptothecin topoisomerase I inhibitor and L and/or U has a deuterated modification; preferably, U is SN-38, an SN-38 derivative, exatecan, or an exatecan derivative; more preferably, U is an exatecan derivative.

**7.** The antibody-drug conjugate or the pharmaceutically acceptable salt or the solvate thereof according to claim 6, wherein -U is a structure represented by formula Ia below:

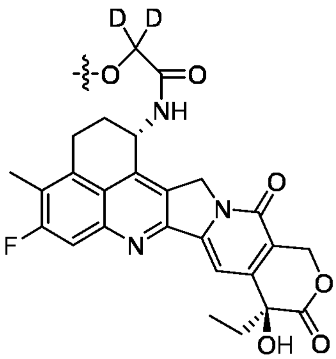

Ia.

**8.** The antibody-drug conjugate or the pharmaceutically acceptable salt or the solvate thereof according to claim 6 or 7, wherein -L- is a structure represented by formula IIa below:

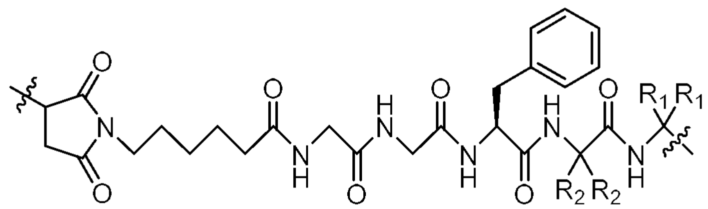

IIa,

wherein $R_1$ and $R_2$ are each independently selected from hydrogen and deuterium;
and the 3-position of -(succinimidyl-3-yl-N)- in the structure shown is linked to Ab, and the methylene at the other end is linked to U.

**9.** The antibody-drug conjugate or the pharmaceutically acceptable salt or the solvate thereof according to claim 1 or 6, wherein -L-U is a structure represented by formula IIIa below:

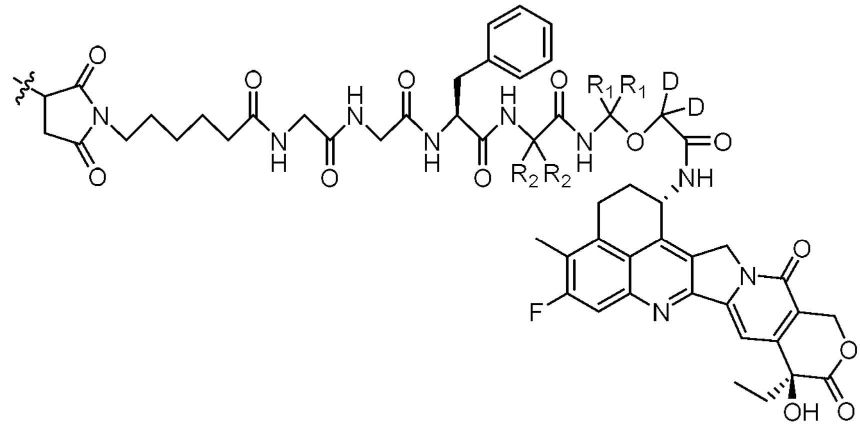
IIIa,

wherein $R_1$ and $R_2$ are each independently selected from hydrogen and deuterium.

**10.** The antibody-drug conjugate or the pharmaceutically acceptable salt or the solvate thereof according to claim 9, wherein -L-U is a structure represented by formula IIIa-1, IIIa-2, IIIa-3, or IIIa-4 below:

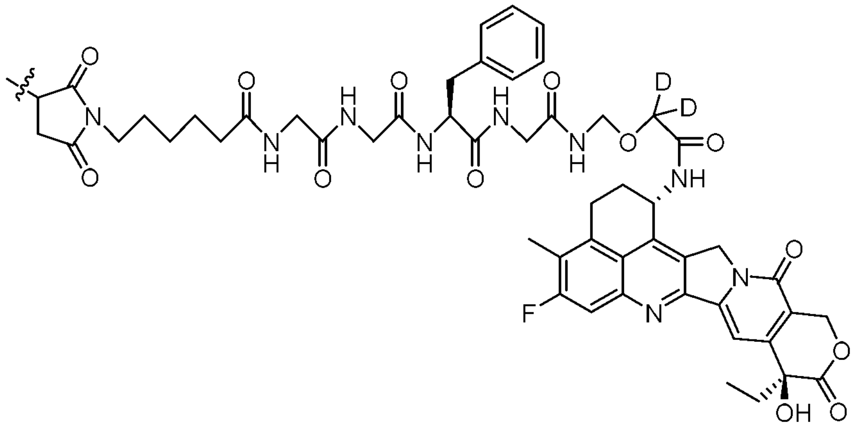
IIIa-1,

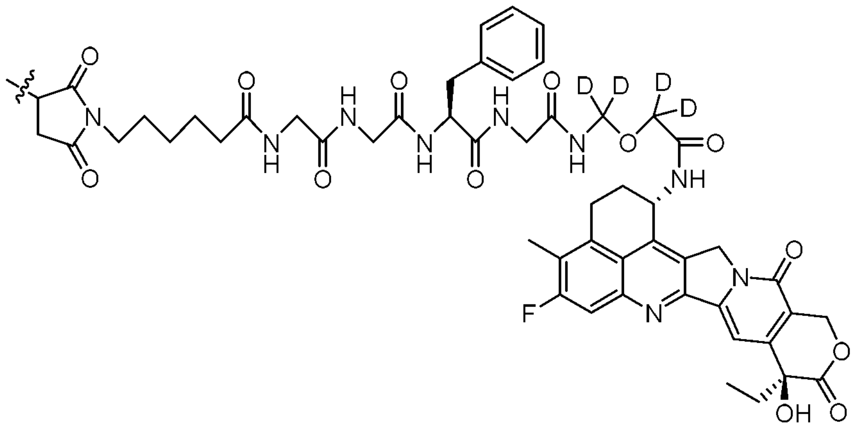
IIIa-2,

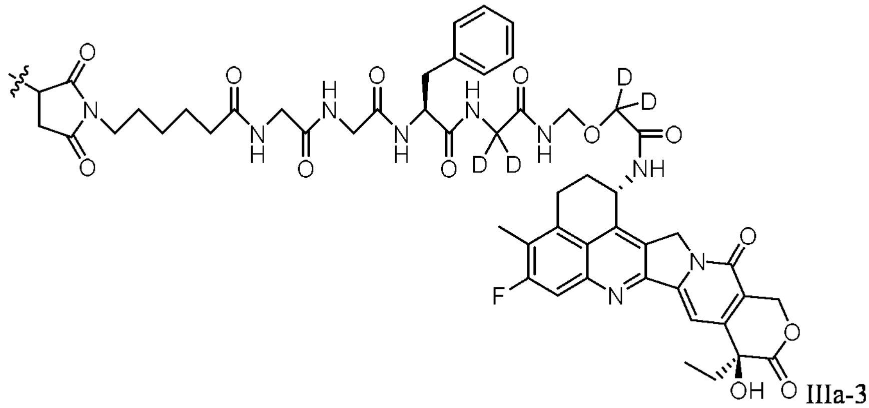

IIIa-3,

or

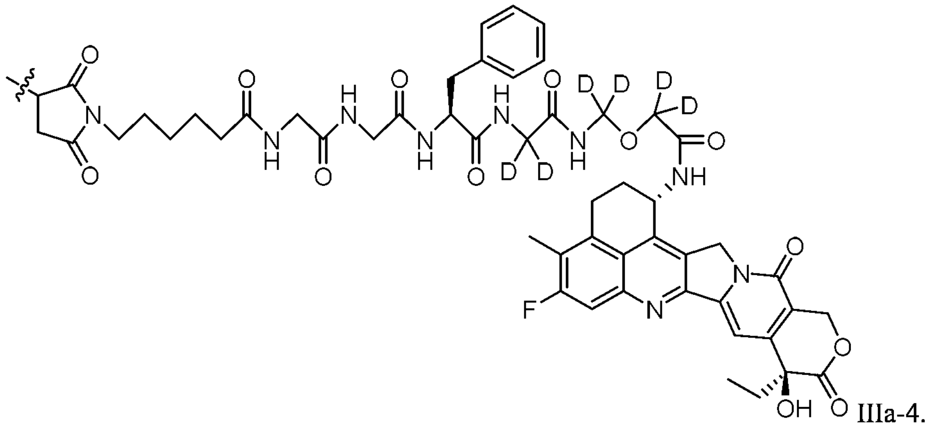

IIIa-4.

**11.** The antibody-drug conjugate or the pharmaceutically acceptable salt or the solvate thereof according to claim 1 or 6, wherein the antibody-drug conjugate has a structure represented by formula IV below:

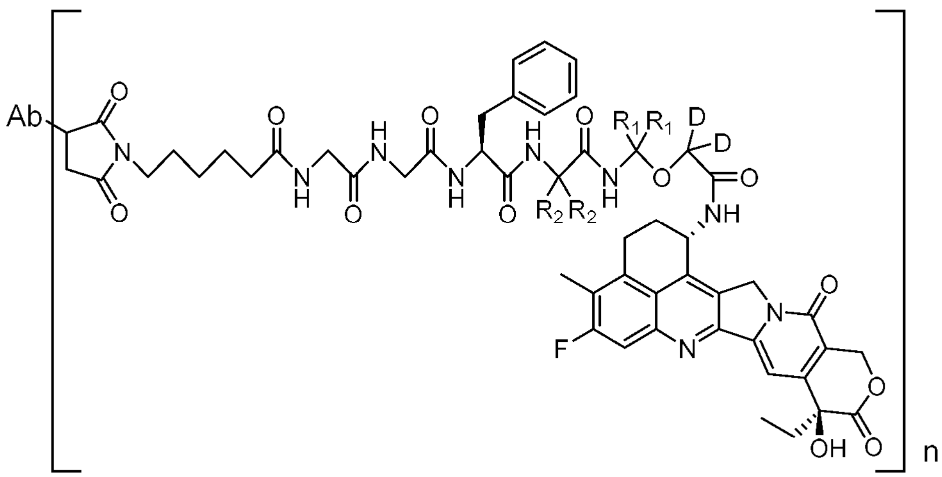

IV,

wherein $R_1$ and $R_2$ are each independently selected from hydrogen and deuterium.

**12.** The antibody-drug conjugate or the pharmaceutically acceptable salt or the solvate thereof according to claim 11, wherein the antibody-drug conjugate has a structure represented by formula IV-1, IV-2, IV-3, or IV-4 below:

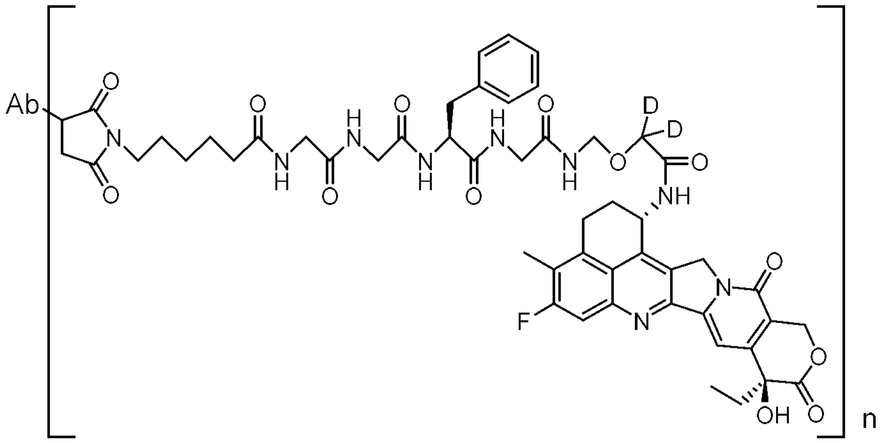 IV-1,

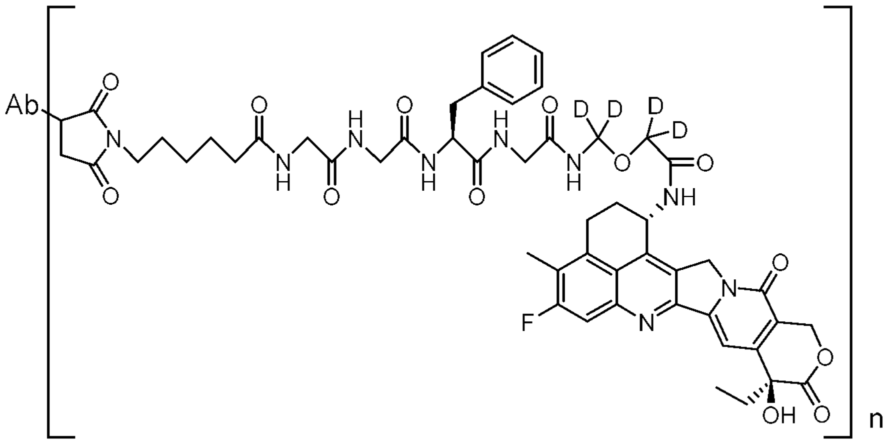 IV-2,

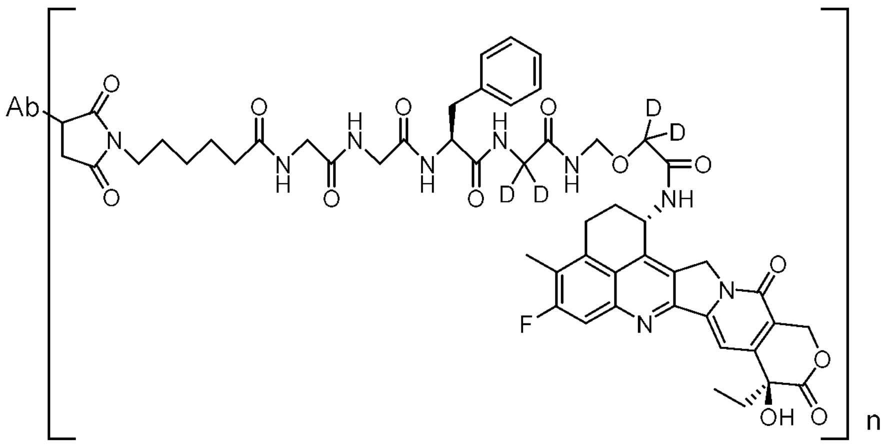 IV-3,

or

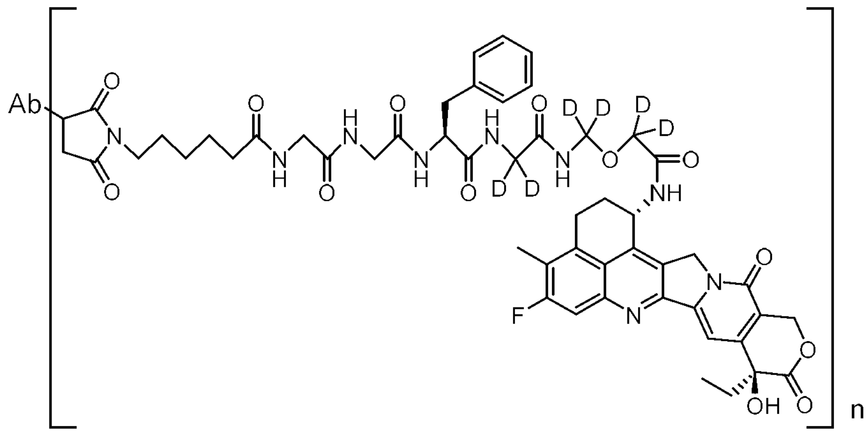

IV-4.

**13.** The antibody-drug conjugate or the pharmaceutically acceptable salt or the solvate thereof according to any one of claims 1-12, wherein n is an integer selected from 1 to 10, 1 to 9, 1 to 8, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8, preferably an integer selected from 2 to 8, 3 to 8, 4 to 8, 5 to 8, 6 to 8, and 7 to 8, and more preferably an integer selected from 6 to 8.

**14.** The antibody-drug conjugate or the pharmaceutically acceptable salt or the solvate thereof according to any one of claims 1-13, wherein the antibody-drug conjugate or the pharmaceutically acceptable salt or the solvate thereof exhibits one of or a combination of several of the following properties:

(a) binding to CLDN18.2;
(b) blocking binding of CLDN18.2 to a ligand;
(c) showing endocytosis in cells expressing CLDN18.2;
(d) having killing activity against tumor cells expressing CLDN18.2;
(e) having a bystander effect;
(f) having ADCC activity;
(g) having CDC activity;
(h) not binding to CLDN18.1;
(i) not binding to cells not expressing CLDN18.2; and
(j) having no ADCC and/or CDC activity against cells expressing CLDN18.1.

**15.** A pharmaceutical composition comprising the antibody-drug conjugate or the pharmaceutically acceptable salt or the solvate thereof according to any one of claims 1-14, wherein preferably, the antibody-drug conjugate or the pharmaceutically acceptable salt or the solvate thereof in the pharmaceutical composition is heterogeneous or homogeneous; optionally, the pharmaceutical composition further comprises a pharmaceutically acceptable carrier.

**16.** The pharmaceutical composition according to claim 15, wherein the antibody-drug conjugate or the pharmaceutically acceptable salt or the solvate thereof in the pharmaceutical composition is heterogeneous, and the pharmaceutical composition has a DAR of 0 to 10, 0 to 9, 0 to 8, 1 to 10, 1 to 9, 1 to 8, 2 to 10, 2 to 9, 2 to 8, 3 to 8, 4 to 8, 5 to 8, 6 to 8, 7 to 8, 7.2 to 8, 7.4 to 8, 7.5 to 8, 7.6 to 8, 7.7 to 8, 7.8 to 8, 7.8 to 7.9, or 7.9 to 8; preferably 7 to 8, 7.2 to 8, 7.4 to 8, 7.6 to 8, or 7.7 to 8; more preferably about 7.1, about 7.2, about 7.4, about 7.6, about 7.7, or about 8.

**17.** The antibody-drug conjugate or the pharmaceutically acceptable salt or the solvate thereof according to any one of claims 1-14, or the pharmaceutical composition according to claim 15 or 16, for use in treating a tumor, wherein optionally, the antibody-drug conjugate or the pharmaceutically acceptable salt or the solvate thereof, or the pharmaceutical composition is used in combination with a second or additional therapeutic agent.

**18.** The antibody-drug conjugate or the pharmaceutically acceptable salt or the solvate thereof, or the pharmaceutical composition for use according to claim 17, wherein the tumor is a CLDN18.2-positive tumor; preferably, the tumor is gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, endometrial cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer,

neuroendocrine tumor, leukemia, lymphoma, and myeloma.

**19.** A method for preparing the antibody-drug conjugate or the pharmaceutically acceptable salt or the solvate thereof according to any one of claims 11-14, comprising treating an anti-CLDN18.2 antibody or an antigen-binding fragment thereof under reducing conditions, and then reacting the anti-CLDN18.2 antibody or the antigen-binding fragment thereof with a linker-payload selected from a structure represented by formula III, wherein the structure represented by formula III is as follows:

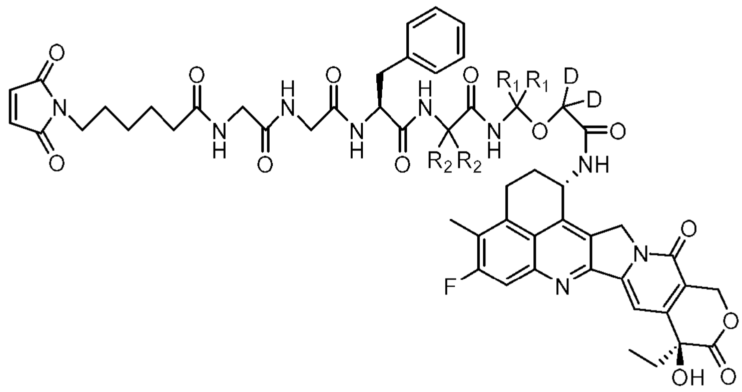

III,

wherein $R_1$ and $R_2$ are each independently selected from hydrogen and deuterium.

**20.** The method according to claim 19, wherein $R_1$ and $R_2$ are hydrogen.

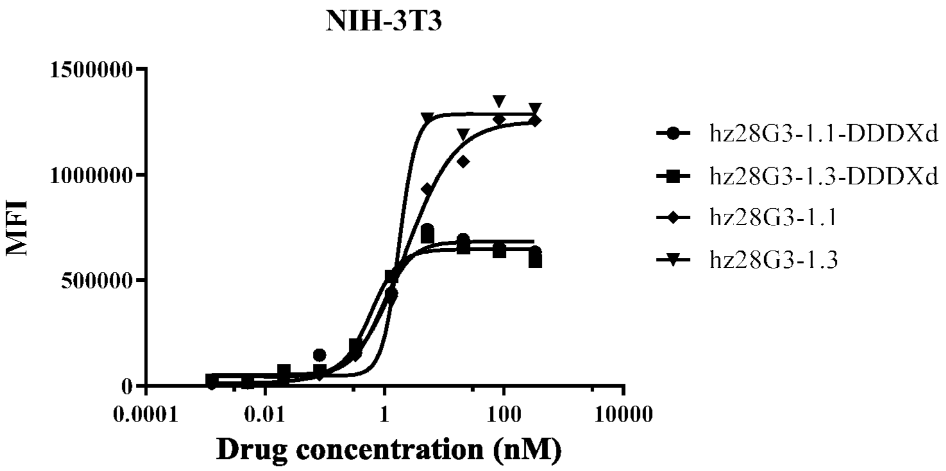


FIG. 1A

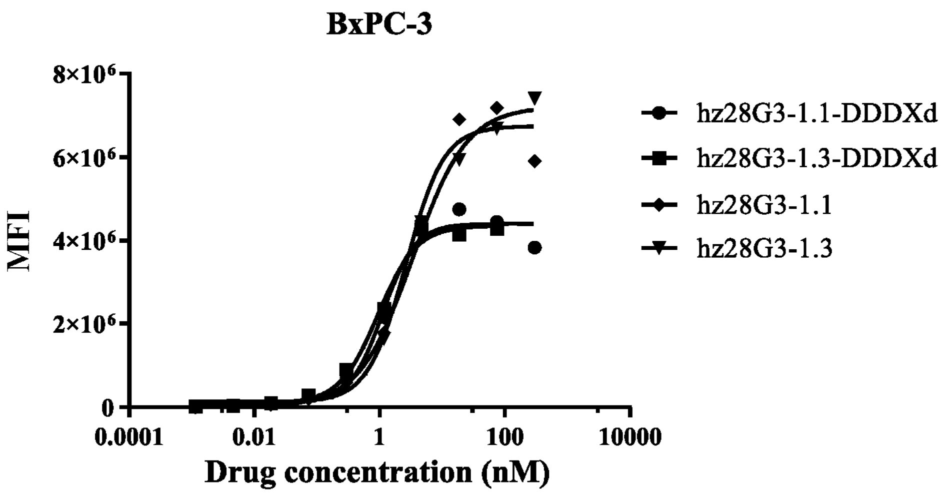


FIG. 1B

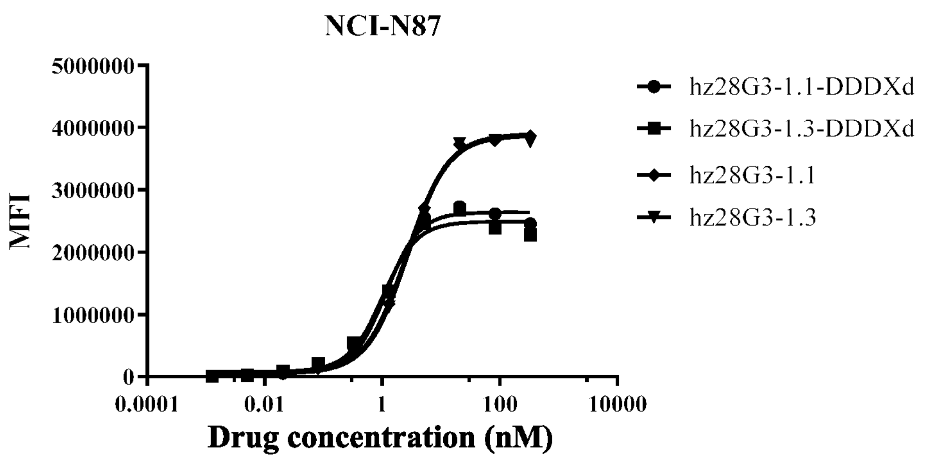


FIG. 1C

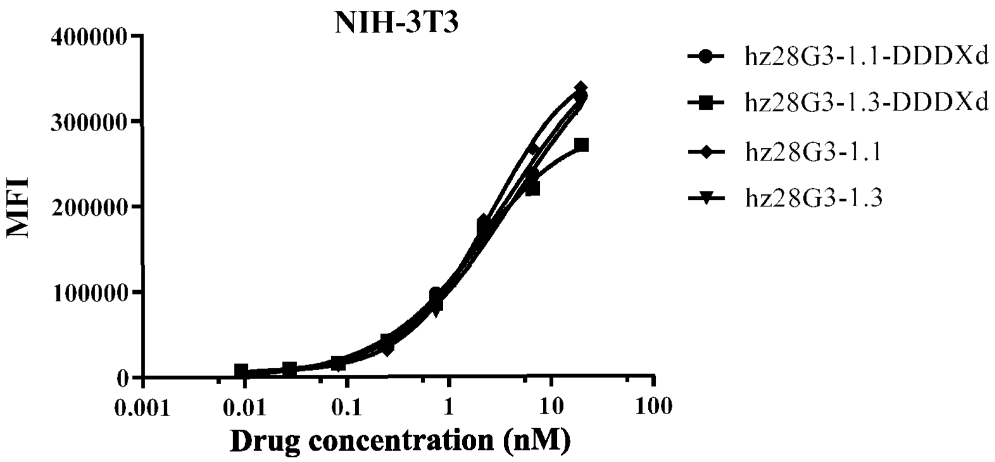


FIG. 2A

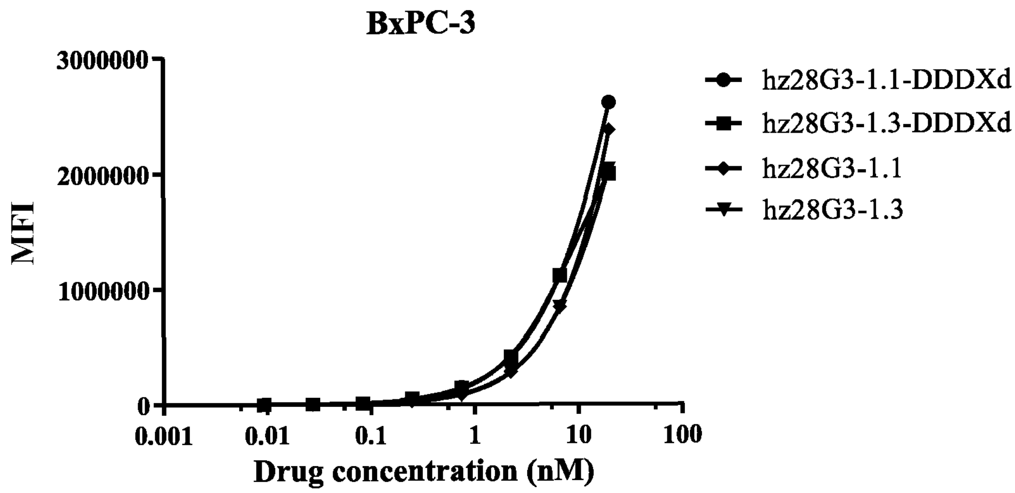


FIG. 2B

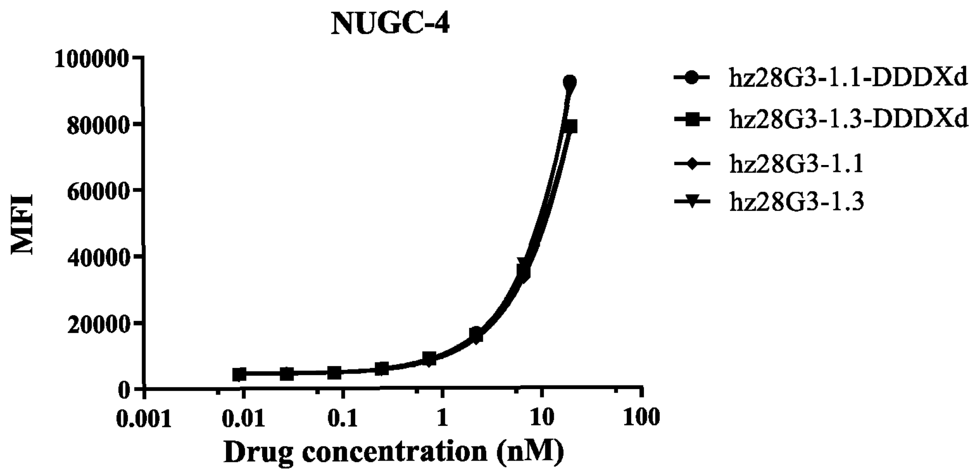


FIG. 2C

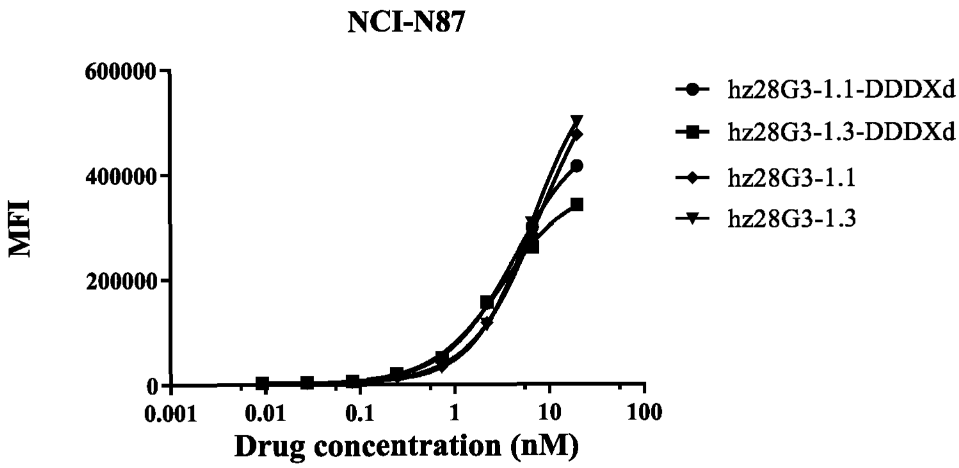


FIG. 2D

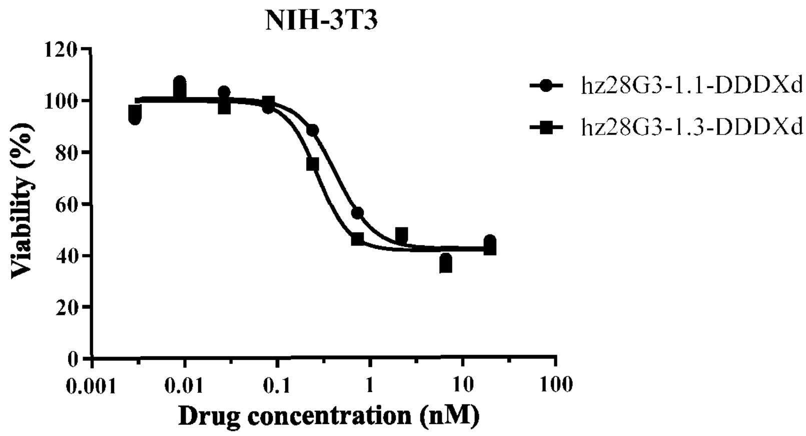


FIG. 3A

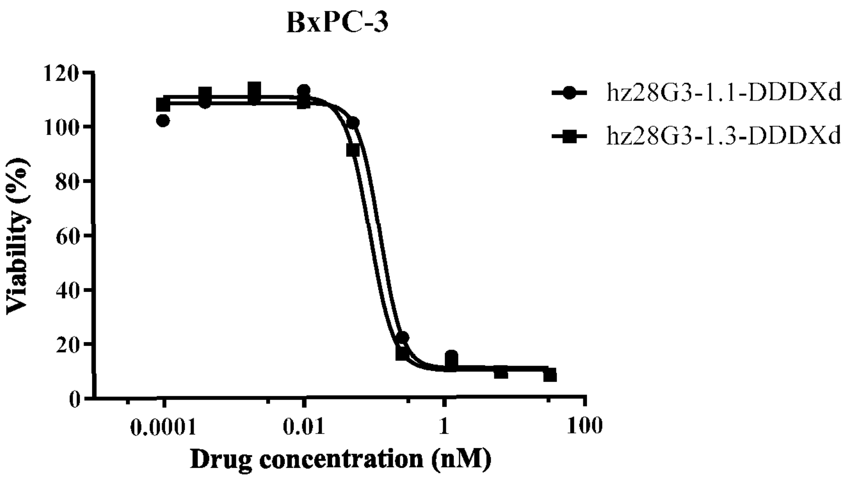


FIG. 3B

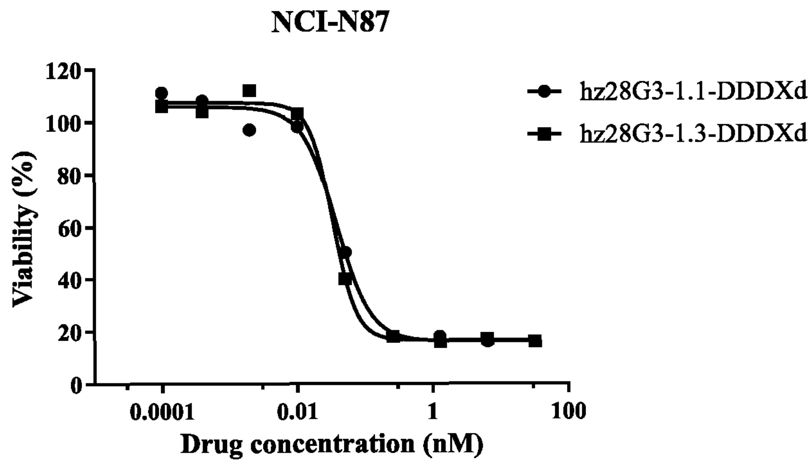


FIG. 3C

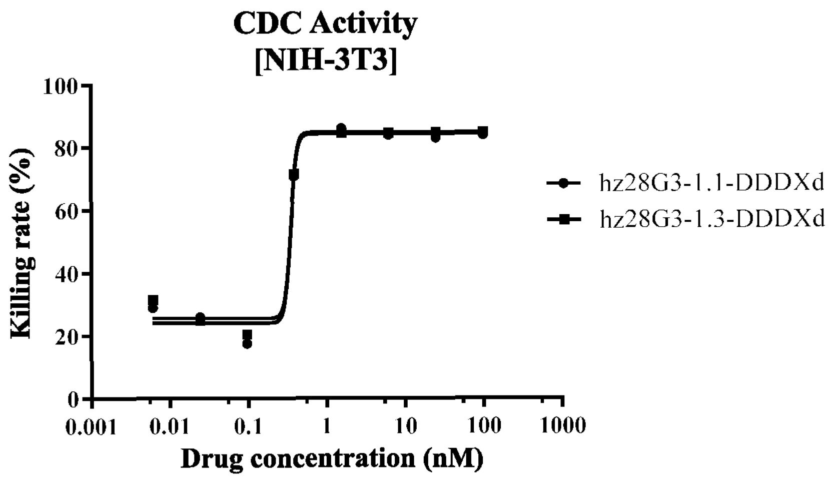


FIG. 4

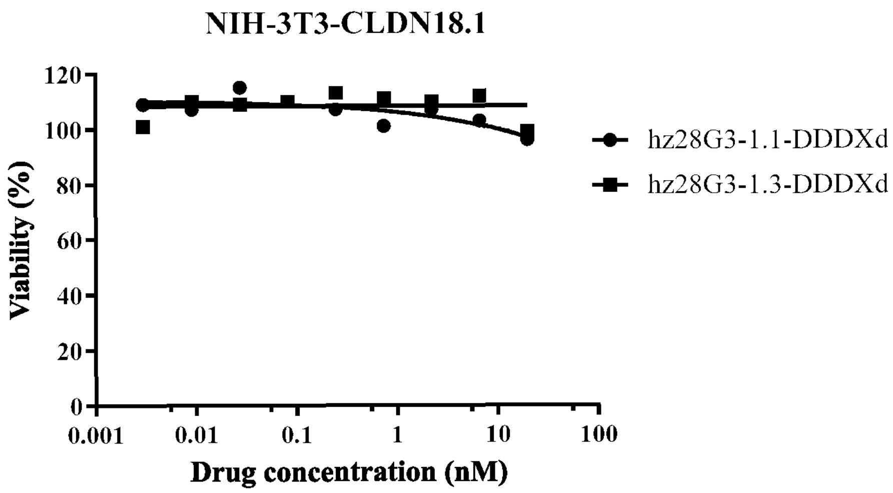


FIG. 5

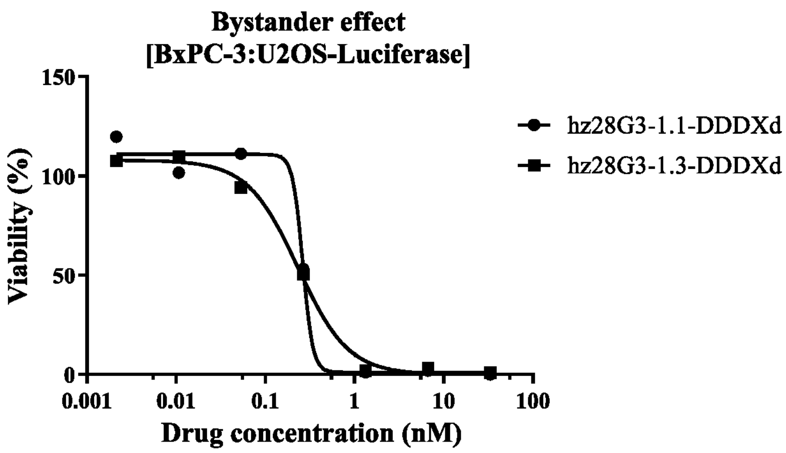
Bystander effect
[BxPC-3:U2OS-Luciferase]
150
100
50
0
Viability (%)
0.001
0.01
0.1
1
10
100
Drug concentration (nM)
hz28G3-1.1-DDDXd
hz28G3-1.3-DDDXd

FIG. 6

**INTERNATIONAL SEARCH REPORT**

International application No.
**PCT/CN2023/134928**

**A. CLASSIFICATION OF SUBJECT MATTER**

A61K47/68(2017.01)i; C07K16/28(2006.01)i; A61K47/65(2017.01)i; A61K39/395(2006.01)i; A61K31/4745(2006.01)i; A61P35/00(2006.01)i

According to International Patent Classification (IPC) or to both national classification and IPC

**B. FIELDS SEARCHED**

Minimum documentation searched (classification system followed by classification symbols)

A61K C07K A61P

Documentation searched other than minimum documentation to the extent that such documents are included in the fields searched

Electronic data base consulted during the international search (name of data base and, where practicable, search terms used)

CNABS, DWPI, WPABS, CNTXT, ENTXT, WOTXT, USTXT, JPTXT, EPTXT, 中国专利生物序列检索系统, China Patents Biological Sequence Search System, CNKI, STNext, GenBank, EBI-EMBL, PubMed, ISI Web of Knowledge: 正大天晴, 陈天玺, 紧密连接蛋白18.2, 密蛋白18.2, 紧密连接蛋白18A2, 密蛋白18A2, 抗体, 抗原结合片段, 喜树碱, SN-38, 依沙替康, 依喜替康, 偶联物, 缀合物, SEQ ID NOs: 1-62, CHIA TAI TIANQING, CLDN18.2, Claudin18.2, CLDN18A2, Claudin18A2, CLDN18, antibody, camptothecin, exatecan, conjugate, GGFG, ADC

**C. DOCUMENTS CONSIDERED TO BE RELEVANT**

| Category* | Citation of document, with indication, where appropriate, of the relevant passages | Relevant to claim No. |
|---|---|---|
| Y | CN 114904015 A (JIANGSU MABWELL HEALTH PHARMACEUTICAL R&D CO., LTD.) 16 August 2022 (2022-08-16)<br>descriptions 0014-0028, 0032-0035, 0041, 0052-0055, 0076-0078, 0081, and 0237-0238, and embodiments 4-5 | 19-20 |
| PY | WO 2023078386 A1 (CHIA TAI TIANQING PHARMACEUTICAL GROUP CO., LTD.) 11 May 2023 (2023-05-11)<br>claims 1-8 and 16 | 19-20 |
| A | CN 114904015 A (JIANGSU MABWELL HEALTH PHARMACEUTICAL R&D CO., LTD.) 16 August 2022 (2022-08-16)<br>descriptions, 0014-0028, 0032-0035, 0041, 0052-0055, 0076-0078, 0081, and 0237-0238, and embodiments 4-5 | 1-18 |
| A | CN 111110862 A (L&L BIOPHARMA CO., LTD.) 08 May 2020 (2020-05-08)<br>claims 1-19 | 1-20 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **2024年3月7日** | **14 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/134928**

**C. DOCUMENTS CONSIDERED TO BE RELEVANT**

| Category* | Citation of document, with indication, where appropriate, of the relevant passages | Relevant to claim No. |
|---|---|---|
| A | CN 112237634 A (SHANGHAI FUDAN-ZHANGJIANG BIO-PHARMACEUTICAL CO., LTD.) 19 January 2021 (2021-01-19)<br>claims 1-7 and 9-10 | 1-20 |
| A | CN 113766933 A (SHANGHAI FUDAN-ZHANGJIANG BIO-PHARMACEUTICAL CO., LTD.) 07 December 2021 (2021-12-07)<br>claims 1-9 and 13-15, and description, embodiment 12 | 1-20 |
| A | US 2022372133 A1 (THE REGENTS OF THE UNIVERSITY OF CALIFORNIA) 24 November 2022 (2022-11-24)<br>claims 51 and 53, description, paragraphs 0175, 0184, 0188-0193, and 0210, and embodiments 9-10 | 1-20 |

Form PCT/ISA/210 (second sheet) (July 2022)

**INTERNATIONAL SEARCH REPORT**

International application No.
**PCT/CN2023/134928**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/134928**

| Patent document cited in search report | | | Publication date (day/month/year) | Patent family member(s) | | | Publication date (day/month/year) |
|---|---|---|---|---|---|---|---|
| CN | 114904015 | A | 16 August 2022 | WO | 2022171134 | A1 | 18 August 2022 |
| WO | 2023078386 | A1 | 11 May 2023 | WO | 2023078386 | A8 | 01 February 2024 |
| | | | | TW | 202323285 | A | 16 June 2023 |
| CN | 111110862 | A | 08 May 2020 | | None | | |
| CN | 112237634 | A | 19 January 2021 | | None | | |
| CN | 113766933 | A | 07 December 2021 | EP | 3991754 | A1 | 04 May 2022 |
| | | | | EP | 3991754 | A4 | 17 May 2023 |
| | | | | KR | 20220025861 | A | 03 March 2022 |
| | | | | AU | 2020301289 | A1 | 24 February 2022 |
| | | | | WO | 2020259258 | A1 | 30 December 2020 |
| | | | | BR | 112021026580 | A2 | 03 May 2022 |
| | | | | US | 2022233708 | A1 | 28 July 2022 |
| | | | | JP | 2022542222 | A | 30 September 2022 |
| | | | | JP | 7407845 | B2 | 04 January 2024 |
| | | | | CA | 3144790 | A1 | 30 December 2020 |
| US | 2022372133 | A1 | 24 November 2022 | US | 11834499 | B1 | 05 December 2023 |
| | | | | EP | 3999548 | A1 | 25 May 2022 |
| | | | | EP | 3999548 | A4 | 16 August 2023 |
| | | | | MX | 2022000652 | A | 02 June 2022 |
| | | | | KR | 20220035414 | A | 22 March 2022 |
| | | | | BR | 112022000371 | A2 | 10 May 2022 |
| | | | | AU | 2020313978 | A1 | 27 January 2022 |
| | | | | JP | 2022541435 | A | 26 September 2022 |
| | | | | WO | 2021011885 | A1 | 21 January 2021 |
| | | | | WO | 2021011885 | A9 | 15 April 2021 |
| | | | | CA | 3146665 | A1 | 21 January 2021 |
| | | | | IL | 289663 | A | 01 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211521035


### Non-patent literature cited in the description


- **NIIMI et al.** *Mol Cell Biol*, 2001, vol. 21 (21), 7380-7390
- **SAHIN, U. et al.** *Clin Cancer Res.*, 2008, vol. 14, 7624-34
- **TANAKA et al.** *J Histochem Cytochem*, 2011, vol. 59 (10), 942-952
- **MICKE et al.** *Int J Cancer*, 2014, vol. 135 (9), 2206-2214
- **SHIMOBABA et al.** *Biochim Biophys Acta*, 2016, vol. 1863 (6), 1170-1178
- **SING H et al.** *J Hema tol Oncol*, 2017, vol. 10 (1), 105
- **TOKUMITSU et al.** *Cytopathology*, 2017, vol. 28 (2), 116-121
- **WARD et al.** *Nature*, 1989, vol. 341, 544-546
- **BIRD et al.** *Science*, 1988, vol. 242, 423-426
- **HUSTON et al.** *Proc. Natl. Acad. Sci.*, 1988, vol. 85, 5879-5883
- *CHEMICAL ABSTRACTS*, 1599440-06-8
- *CHEMICAL ABSTRACTS*, 2760715-83-9